# EUROPEAN PATENT SPECIFICATION

(11) **EP 2 563 240 B1**
(45) Date of publication and mention of the grant of the patent: **03.10.2018**
(21) Application number: 11778070.0
(22) Date of filing: 29.04.2011
(51) Int. Cl.: A61B 17/064, D02J 3/00, A61L 17/00, D01F 6/06

(54) **HIGH-DENSITY SELF-RETAINING SUTURES**
HOCHDICHTE SELBSTHALTENDE NÄHTE
SUTURES D'AUTO-RETENUE À HAUTE DENSITÉ

(30) Priority: 29.04.2010 US 329436 P
(43) Date of publication of application: 06.03.2013
(73) Proprietor: Ethicon LLC, Guaynabo 00969 (PR)
(72) Inventor: GROSS, Jeffrey, M., Encinitas, CA 92024 (US); DRUBETSKY, Lev, Coquitlam, British Columbia V3E 2S9 (CA); NAIMAGON, Alexander, Richmond, British Columbia V6Y 1M3 (CA); D'AGOSTINO, William, L., Hamden, CT 06518 (US)
(74) Representative: Small, Gary James
(86) International application number: PCT/US2011/034660
(87) International publication number: WO 2011/139916

(56) References cited:
- EP-A1- 1 858 243
- WO-A2-2004/100801
- WO-A2-2009/097556
- DE-A1-102008 004 574
- US-A1- 2004 060 409
- US-A1- 2008 255 611
- US-A1- 2009 012 560
- US-B2- 6 848 152

## Description

### FIELD OF INVENTION

The present invention relates generally to self-retaining sutures and sutures having a high density of retainers and apparatus and methods for manufacturing such self-retaining sutures and sutures.

### BACKGROUND OF INVENTION

Wound closure devices such as sutures, staples and tacks have been widely used in superficial and deep surgical procedures in humans and animals for closing wounds, repairing traumatic injuries or defects, joining tissues together (bringing severed tissues into approximation, closing an anatomical space, affixing single or multiple tissue layers together, creating an anastomosis between two hollow/luminal structures, adjoining tissues, attaching or reattaching tissues to their proper anatomical location), attaching foreign elements to tissues (affixing medical implants, devices, prostheses and other functional or supportive devices), and for repositioning tissues to new anatomical locations (repairs, tissue elevations, tissue grafting and related procedures) to name but a few examples.

Sutures are often used as wound closure devices. Sutures typically consist of a filamentous suture thread attached to a needle with a sharp point. Suture threads can be made from a wide variety of materials including bioabsorbable (i.e., that break down completely in the body over time), or non-absorbable (permanent; non-degradable) materials. Absorbable sutures have been found to be particularly useful in situations where suture removal might jeopardize the repair or where the natural healing process renders the support provided by the suture material unnecessary after wound healing has been completed; as in, for example, completing an uncomplicated skin closure. Non-degradable (non-absorbable) sutures are used in wounds where healing may be expected to be protracted or where the suture material is needed to provide physical support to the wound for long periods of time; as in, for example, deep tissue repairs, high tension wounds, many orthopedic repairs and some types of surgical anastomosis. Also, a wide variety of surgical needles are available; the shape and size of the needle body and the configuration of the needle tip is typically selected based upon the needs of the particular application.

To use an ordinary suture, a suture needle is advanced through the desired tissue on one side of the wound and then through the adjacent side of the wound. The suture is then formed into a "loop" which is completed by tying a knot in the suture to hold the wound closed. Knot tying takes time and causes a range of complications, including, but not limited to (i) spitting, a condition where the suture, usually a knot, pushes through the skin after a subcutaneous closure), (ii) infection (bacteria are often able to attach and grow in the spaces created by a knot), (iii) bulk/mass (a significant amount of suture material left in a wound is the portion that comprises the knot), (iv) slippage (knots can slip or come untied), and (v) irritation (knots serve as a bulk "foreign body" in a wound). Suture loops associated with knot tying may lead to ischemia (knots can create tension points that can strangulate tissue and limit blood flow to the region) and increased risk of dehiscence or rupture at the surgical wound. Knot tying is also labor intensive and can comprise a significant percentage of the time spent closing a surgical wound. Additional operative procedure time is not only bad for the patient (complication rates rise with time spent under anesthesia), but it also adds to the overall cost of the operation (many surgical procedures are estimated to cost between $15 and $30 per minute of operating time).

Self-retaining sutures (including barbed sutures) differ from conventional sutures in that self-retaining sutures possess numerous tissue retainers (such as barbs) which anchor the self-retaining suture into the tissue following deployment and resist movement of the suture in a direction opposite to that in which the retainers face, thereby eliminating the need to tie knots to affix adjacent tissues together (a "knotless" closure). Knotless tissue-approximating devices having barbs have been previously described in, for example, U.S. Pat. No. 5,374,268, disclosing armed anchors having barb-like projections, while suture assemblies having barbed lateral members have been described in U.S. Pat. Nos. 5,584,859 and 6,264,675. Sutures having a plurality of barbs positioned along a greater portion of the suture are described in U.S. Pat No. 5,931,855, which discloses a unidirectional barbed suture, and U.S. Pat. No. 6,241,747, which discloses a bidirectional barbed suture. Methods and apparatus for forming barbs on sutures have been described in, for example, U.S. Pat. Nos. 6,848,152. Self-retaining systems for wound closure also result in better approximation of the wound edges, evenly distribute the tension along the length of the wound (reducing areas of tension that can break or lead to ischemia), decrease the bulk of suture material remaining in the wound (by eliminating knots) and reduce spitting (the extrusion of suture material - typically knots - through the surface of the skin). All of these features are thought to reduce scarring, improve cosmesis, and increase wound strength relative to wound closures using plain sutures or staples. Thus, self-retaining sutures, because such sutures avoid knot tying, allow patients to experience an improved clinical outcome, and also save time and costs associated with extended surgeries and follow-up treatments.

The ability of self-retaining sutures to anchor and hold tissues in place even in the absence of tension applied to the suture by a knot is a feature that also provides superiority over plain sutures. When closing a wound that is under tension, this advantage manifests itself in several ways: (i) self-retaining sutures have a multiplicity of retainers which can dissipate tension along the entire length of the suture (providing hundreds of "anchor" points that produce a superior cosmetic result and lessens the chance that the suture will "slip" or pull through) as opposed to knotted interrupted sutures which concentrate the tension at discrete points; (ii) complicated wound geometries can be closed (circles, arcs, jagged edges) in a uniform manner with more precision and accuracy than can be achieved with interrupted sutures; (iii) self-retaining sutures eliminate the need for a "third hand" which is often required for maintaining tension across the wound during traditional suturing and knot tying (to prevent "slippage" when tension is momentarily released during tying); (iv) self-retaining sutures are superior in procedures where knot tying is technically difficult, such as in deep wounds or laparoscopic/endoscopic procedures; and (v) self-retaining sutures can be used to approximate and hold the wound prior to definitive closure. As a result, self-retaining sutures provide easier handling in anatomically tight or deep places (such as the pelvis, abdomen and thorax) and make it easier to approximate tissues in laparoscopic/endoscopic and minimally invasive procedures; all without having to secure the closure via a knot. Greater accuracy allows self-retaining sutures to be used for more complex closures (such as those with diameter mismatches, larger defects or purse string suturing) than can be accomplished with plain sutures.

A self-retaining suture may be unidirectional, having one or more retainers oriented in one direction along the length of the suture thread; or bidirectional, typically having one or more retainers oriented in one direction along a portion of the thread, followed by one or more retainers oriented in another (often opposite) direction over a different portion of the thread (as described with barbed retainers in U.S. Pat. Nos. 5,931,855 and. 6,241,747). Although any number of sequential or intermittent configurations of retainers are possible, a common form of bidirectional self-retaining suture involves a needle at one end of a suture thread which has barbs having tips projecting "away" from the needle until the transition point (often the midpoint) of the suture is reached; at the transition point the configuration of barbs reverses itself about 180° (such that the barbs are now facing in the opposite direction) along the remaining length of the suture thread before attaching to a second needle at the opposite end (with the result that the barbs on this portion of the suture also have tips projecting "away" from the nearest needle). Projecting "away" from the needle means that the tip of the barb is further away from the needle and the portion of suture comprising the barb may be pulled more easily through tissue in the direction of the needle than in the opposite direction. Put another way, the barbs on both "halves" of a typical bidirectional self-retaining suture have tips that point towards the middle, with a transition segment (lacking barbs) interspersed between them, and with a needle attached to either end.

German patent application publication DE 10 2008 004 574 A1 discloses anchoring elements which can be placed on a suture in particular in one or more rows and/or as helices. In another embodiment, anchoring elements are located on a suture surface at a density of 6-10 anchoring elements per 5 mm length of the suture.

US patent application publication US 2008/255611 A1 discloses self-retaining sutures with primary retainers and secondary retainers on the primary retainers.

European patent application publication EP1858243A1 discloses a barbed suture for connecting human or animal tissue in combination with a surgical needle, said combination comprising a barbed suture attached to a surgical needle, wherein the suture comprises a plurality of barbs projecting from an elongated body having a first end and a diameter, each barb facing in a direction and being adapted for resisting movement of the suture when in tissue, in an opposite direction from the direction in which the barb faces, characterized in that the elongated has a non-circular diameter and the surgical needle has a diameter with a ratio of the surgical needle diameter to the barbed suture diameter of about 3:1 or less.

### SUMMARY OF INVENTION

A self-retaining suture in accordance with the present invention is specified in claim 1.

It is desirable in some applications to provide self-retaining sutures having profiles, materials and diameters upon which it is difficult to provide retainers. Thus, it is desirable to provide improved self-retaining sutures which have enhanced ability to anchor into the surrounding tissue, enhanced tissue holding capabilities, enhanced maximum load, and enhanced clinical performance.

It is particularly desirable to provide improved self-retaining sutures of small diameter which have enhanced ability to anchor into the surrounding tissue, enhanced tissue holding capabilities, enhanced maximum load, and enhanced clinical performance.

The present disclosure provides improved self-retaining sutures which have enhanced ability to anchor into the surrounding tissue, enhanced tissue holding capabilities, enhanced maximum load, and enhanced clinical performance.

The present disclosure further provides improved self-retaining sutures of small diameter which have enhanced ability to anchor into the surrounding tissue, enhanced tissue holding capabilities, enhanced maximum load, and enhanced clinical performance.

The present disclosure still further provides apparatus and methods for manufacturing improved self-retaining sutures of small diameter.

The present disclosure yet further provides clinical methods and procedures enabled by such improved self-retaining sutures of small diameter.

For example, there is herein disclosed a self-retaining suture comprising: a suture thread with a plurality of retainers distributed along the suture thread; wherein the plurality of retainers is distributed at a density of about at least 39 retainers per cm (100 retainers per inch) along a length of the suture thread; and wherein the plurality of retainers is distributed in a pattern selected from: a quadra-helix pattern; a double-helix pattern; and a single helix pattern. In this and other examples provided herein, the suture may optionally be further characterized by one, or any two or more not-inconsistent combinations of the following features, which are exemplary of features disclosed herein and therefore are non-limiting: the pattern has a pitch (P) and the retainers have a length (L) and wherein P < 2L; the density of retainers is about at least 79 retainers per cm (200 retainers per inch) along a length of the suture thread; the density of retainers is about at least 160 retainers per cm (400 retainers per inch) along a length of the suture thread; the density of retainers is about at least 315 retainers per cm (800 retainers per inch) along a length of the suture thread; the density of retainers is about at least 470 retainers per cm (1200 retainers per inch) along a length of the suture thread; the plurality of retainers is distributed in a double-helix pattern having one retainer per repeating unit of the pattern; the plurality of retainers is distributed in a double-helix pattern having at least two retainers per repeating unit of the pattern; the plurality of retainers is distributed in a quadra-helix pattern having at least four retainers per repeating unit of the pattern; the suture thread is of a size in the range of 4-0 to 12-0; the suture thread is no larger than size 4-0; the suture thread is no larger than size 6-0; the suture thread is no larger than 8-0; the retainers comprise a portion of the suture thread deformed by a mechanical process into a shape adapted to engage tissue; the retainers comprise a portion of the suture thread having a cut which partially separates a portion of the suture thread into a shape adapted to engage tissue; the retainers comprise a portion of the suture thread from which a portion of material has been removed to partially separate a portion of the suture thread into a shape adapted to engage tissue; the retainers are formed using a sapphire blade; each retainer has a length (L) and the suture thread has a diameter (SD) and wherein L > 0.6SD; the retainers are characterized by an aspect ratio and the aspect ratio of the retainers is greater than 2.5; the aspect ratio of the retainers is greater than 3; the aspect ratio of the retainers is greater than 3.5; the aspect ratio of the retainers is greater than 3.5 but no greater than 6; the aspect ratio of the retainers is greater than 4; the aspect ratio of the retainers is greater than 5; the aspect ratio of the retainers is greater than 4 but no greater than 6; the aspect ratio of the retainers is greater than 5 but no greater than 7; the suture is characterized by the number of retainers per suture diameter in axial length of suture, for instance the number is greater than 1 and less than 5; the number of retainers per suture diameter in axial length of suture is greater than 3; the number of retainers per suture diameter in axial length of suture is greater than 3 and less than 5; the number of retainers per suture diameter in axial length of suture is greater than 4; the sum of the lengths of the retainers in 25 mm (an inch) of suture is greater than 25mm (1 inch); the sum of the lengths of the retainers in 25 mm (an inch) of suture is greater than 25mm (1 inch) and less than 100 mm (4 inches); the sum of the lengths of the retainers in 25 mm (an inch) of suture is greater than 50 mm (2 inches) and less than 100 mm (4 inches); the sum of the lengths of the retainers in 25 mm (an inch) of suture is greater than 75 mm (3 inches) and less than 100 mm (4 inches); the sum of the lengths of the retainers in 25 mm (an inch) of suture is greater than 75 mm (3 inches); the suture thread is a monofilament suture into which the retainers are cut; the suture thread is a polypropylene suture into which the retainers are cut; the suture thread is a drawn polymeric fiber suture into which the retainers are cut; the suture has a needle at each end; the suture has a needle at one end and an anchor at one end; and/or the suture has a needle at one end and an anchor at one end, wherein the anchor is an anchor selected from: a loop, a tack, a staple, a clip, a pledget, and a short barbed segment of suture without a needle.

In another example, a self-retaining suture comprises: a suture thread; a plurality of retainers distributed along the suture thread; wherein the suture thread has a suture diameter (SD) no greater than about 300µm; wherein the retainers have a cut depth (C) between 5% and 35% of the suture diameter (SD); wherein the retainers have a retainer length (L) greater than 50% of the suture diameter (SD); and wherein the retainers are distributed at a density greater than 2 retainers per suture diameters (SD) in length of suture thread. In this and other examples provided herein, the suture may optionally be further characterized by one, or any two or more not-inconsistent combinations of the following features, which are exemplary of features disclosed herein and therefore are non-limiting: the plurality of retainers is distributed at a density greater than 2 retainers per retainer length (L) of suture thread; the retainers are distributed at a density greater than 2.5 retainers per retainer length (L) of suture thread; the retainers are distributed at a density greater than 3 retainers per retainer length (L) of suture thread; the retainer length (L) is between 500% and 800% of the cut depth (C); the suture thread has a suture diameter (SD) no greater than about 100µm; and the suture thread has a suture diameter (SD) no greater than about 50µm; the suture thread has a suture diameter (SD) less than about 100µm and the retainers have a length greater than 50µm and the retainers are distributed at a density of at least 4 retainers per 100µm of suture thread; the suture thread has a suture diameter SD less than about 60µm and the retainers have a length greater than 25µm and the retainers are distributed at a density of at least 4 retainers per 60µm of suture thread; the retainers are characterized by an aspect ratio and the aspect ratio of the retainers is greater than 2.5; the aspect ratio of the retainers is greater than 3; the aspect ratio of the retainers is greater than 3.5; the aspect ratio of the retainers is greater than 3.5 but no greater than 6; the aspect ratio of the retainers is greater than 4; the aspect ratio of the retainers is greater than 5; the aspect ratio of the retainers is greater than 4 but no greater than 6; the aspect ratio of the retainers is greater than 5 but no greater than 7; the suture is characterized by the number of retainers per suture diameter in axial length of suture, for instance the number is greater than 1 and less than 5; the number of retainers per suture diameter in axial length of suture is greater than 3; the number of retainers per suture diameter in axial length of suture is greater than 3 and less than 5; the number of retainers per suture diameter in axial length of suture is greater than 4; the sum of the lengths of the retainers in 25 mm (an inch) of suture is greater than 25 mm (1 inch); the sum of the lengths of the retainers in 25 mm (an inch) of suture is greater than 25 mm (1 inch) and less than 100 mm (4 inches); the sum of the lengths of the retainers in 25 mm (an inch) of suture is greater than 50 mm (2 inches) and less than 100 mm (4 inches); the sum of the lengths of the retainers in 25 mm (an inch) of suture is greater than 75 mm (3 inches) and less than 100 mm (4 inches); the sum of the lengths of the retainers in 25 mm (an inch) of suture is greater than 75 mm (3 inches); the suture thread is a monofilament suture into which the retainers are cut; the suture thread is a polypropylene suture into which the retainers are cut; the suture thread is a drawn polymeric fiber suture into which the retainers are cut; the suture has a needle at each end; the suture has a needle at one end and an anchor at one end; and/or the suture has a needle at one end and an anchor at one end, wherein the anchor is an anchor selected from: a loop, a tack, a staple, a clip, a pledget, and a short barbed segment of suture without a needle.

In a further example, there is provided a self-retaining suture comprising a suture thread and a plurality of retainers distributed along the suture thread wherein: the suture thread has a suture diameter (SD) no greater than 300µm; the retainers have a retainer length L greater than 20% of the suture diameter (SD); and the retainers are distributed at a density greater than 79 retainers per cm (200 retainers per inch). In this and other examples provided herein, the suture may optionally be further characterized by one, or any two or more not-inconsistent combinations of the following features, which are exemplary of features disclosed herein and therefore are non-limiting: the retainers are distributed at a density of between 79 retainers per cm (200 retainers per inch) and 630 retainers per cm (1,600 retainers per inch); the retainers are distributed at a density greater than 160 retainers per cm (400 retainers per inch); the retainers are distributed at a density greater than 315 retainers per cm (800 retainers per inch); the retainers are distributed at a density greater than 470 retainers per cm (1200 retainers per inch); the suture thread has a suture diameter (SD) smaller than 100µm; the suture thread has a suture diameter (SD) smaller than 100µm and the self-retaining suture has at least 500 retainers within a 25 mm (one inch) length of the suture thread; the suture thread has a suture diameter (SD) no greater than 50µm and the self-retaining suture has at least 800 retainers within a 25 mm (one inch) length of the suture thread; the retainers are characterized by an aspect ratio and the aspect ratio of the retainers is greater than 2.5; the aspect ratio of the retainers is greater than 3; the aspect ratio of the retainers is greater than 3.5; the aspect ratio of the retainers is greater than 3.5 but no greater than 6; the aspect ratio of the retainers is greater than 4; the aspect ratio of the retainers is greater than 5; the aspect ratio of the retainers is greater than 4 but no greater than 6; the aspect ratio of the retainers is greater than 5 but no greater than 7; the suture is characterized by the number of retainers per suture diameter in axial length of suture, for instance the number is greater than 1 and less than 5; the number of retainers per suture diameter in axial length of suture is greater than 3; the number of retainers per suture diameter in axial length of suture is greater than 3 and less than 5; the number of retainers per suture diameter in axial length of suture is greater than 4; the sum of the lengths of the retainers in 25 mm (an inch) of suture is greater than 25 mm (1 inch); the sum of the lengths of the retainers in 25 mm (an inch) of suture is greater than 25 mm (1 inch) and less than 100 mm (4 inches); the sum of the lengths of the retainers in 25 mm (an inch) of suture is greater than 50 mm (2 inches) and less than 100 mm (4 inches); the sum of the lengths of the retainers in 25 mm (an inch) of suture is greater than 75 mm (3 inches) and less than 100 mm (4 inches); the sum of the lengths of the retainers in 25 mm (an inch) of suture is greater than 75 mm (3 inches); the suture thread is a monofilament suture into which the retainers are cut; the suture thread is a polypropylene suture into which the retainers are cut; the suture thread is a drawn polymeric fiber suture into which the retainers are cut; the suture has a needle at each end; the suture has a needle at one end and an anchor at one end; and/or the suture has a needle at one end and an anchor at one end, wherein the anchor is an anchor selected from: a loop, a tack, a staple, a clip, a pledget, and a short barbed segment of suture without a needle.

Another example provides a self-retaining medical device comprising: a suture having a diameter less than about 350µm and greater than about 250µm; the suture having a longitudinal axis; a plurality of retainers, each retainer being formed by an angled cut into a section of the suture; each retainer having a retainer length measured along said axis wherein the retainer length is greater than about 300µm and less than about 500µm; the retainers being distributed in pairs, each pair comprising a first retainer and a second retainer; wherein the second retainer of each pair is positioned at substantially the same position along the axis and substantially 180 degrees around said axis from the first retainer of each pair; wherein for each pair of retainers there is at least one adjacent pair of retainers; wherein each pair of retainers is displaced by a pitch length along the axis and substantially 90 degrees around said axis relative to the adjacent pair of retainers; and wherein the pitch length is no less than about 300µm and no greater than about 550µm. In this and other examples provided herein, the suture may optionally be further characterized by one, or any two or more not-inconsistent combinations of the following features, which are exemplary of features disclosed herein and therefore are non-limiting: the retainer length is at least about 400µm; the retainer length is at least about 400µm and the pitch length is no greater than about 500µm; the retainer length is at least about 400µm and the pitch length is no less than about 400µm; the pitch length is no more than 100µm greater than the retainer length; wherein the retainer length is about 420µm; the pitch length is about 500µm; the retainer length is about 420µm and the pitch length is about 500µm; the retainers are characterized by an aspect ratio and the aspect ratio of the retainers is greater than 2.5; the aspect ratio of the retainers is greater than 3; the aspect ratio of the retainers is greater than 3.5; the aspect ratio of the retainers is greater than 3.5 but no greater than 6; the aspect ratio of the retainers is greater than 4; the aspect ratio of the retainers is greater than 5; the aspect ratio of the retainers is greater than 4 but no greater than 6; the aspect ratio of the retainers is greater than 5 but no greater than 7; the suture is characterized by the number of retainers per suture diameter in axial length of suture, for instance the number is greater than 1 and less than 5; the number of retainers per suture diameter in axial length of suture is greater than 3; the number of retainers per suture diameter in axial length of suture is greater than 3 and less than 5; the number of retainers per suture diameter in axial length of suture is greater than 4; the sum of the lengths of the retainers in 25 mm (an inch) of suture is greater than 25 mm (1 inch); the sum of the lengths of the retainers in 25 mm (an inch) of suture is greater than 25 mm (1 inch) and less than 75 mm (4 inches); the sum of the lengths of the retainers in 25 mm (an inch) of suture is greater than 50 mm (2 inches) and less than 100 mm (4 inches); the sum of the lengths of the retainers in 25 mm (an inch) of suture is greater than 75 mm (3 inches) and less than 100 mm (4 inches); the sum of the lengths of the retainers in 25 mm (an inch) of suture is greater than 75 mm (3 inches); the suture thread is a monofilament suture into which the retainers are cut; the suture thread is a polypropylene suture into which the retainers are cut; the suture thread is a drawn polymeric fiber suture into which the retainers are cut; the suture has a needle at each end; the suture has a needle at one end and an anchor at one end; the suture has a needle at one end and an anchor at one end, wherein the anchor is an anchor selected from: a loop, a tack, a staple, a clip, a pledget, and a short barbed segment of suture without a needle; and/or the suture is a polypropylene monofilament of USP 2-0.

In yet another example, a self-retaining medical device comprises: a suture having a suture diameter and a longitudinal axis; a plurality of retainers, each retainer being formed by an angled cut into a section of the suture; each retainer having a retainer length greater than one suture diameter measured along said axis; the retainers being distributed in pairs, each pair comprising a first retainer and a second retainer at substantially the same position along the axis but on the opposite side of the suture; wherein for each pair of retainers there is at least one adjacent pair of retainers; and wherein the retainers of each pair of retainers are displaced by a pitch length less than two suture diameters along the axis and substantially 90 degrees around said axis relative to the retainers of the adjacent pair of retainers. In this and other examples provided herein, the suture may optionally be further characterized by one, or any two or more not-inconsistent combinations of the following features, which are exemplary of features disclosed herein and therefore are non-limiting: the retainers are distributed at a retainer density no less than about 39 retainers per cm (100 retainers per inch); the combined lengths of retainers within 25 mm (an inch) of suture is greater than 25 mm (an inch); the combined lengths of retainers within 25 mm (an inch) of suture is greater than 38 mm (1.5 inches); the pitch length is no more than 1.5 suture diameters; the pitch length is no more than 100µm greater than the retainer length; the pitch length is no more than 120% of the retainer length; the suture is size USP 2-0; the suture diameter is less than 350µm; the retainer length is at least about 400µm; the suture diameter is less than about 350µm, the retainer length is at least about 400µm and the pitch length is no greater than about 500µm; the suture diameter is less than about 350µm and the retainer length is about 420µm; the suture diameter is less than about 350µm and the pitch length is about 500µm; the suture diameter is less than about 350µm, the retainer length is about 420µm and the pitch length is about 500µm; the retainers are characterized by an aspect ratio and the aspect ratio of the retainers is greater than 2.5; the aspect ratio of the retainers is greater than 3; the aspect ratio of the retainers is greater than 3.5; the aspect ratio of the retainers is greater than 3.5 but no greater than 6; the aspect ratio of the retainers is greater than 4; the aspect ratio of the retainers is greater than 5; the aspect ratio of the retainers is greater than 4 but no greater than 6; the aspect ratio of the retainers is greater than 5 but no greater than 7; the suture is characterized by the number of retainers per suture diameter in axial length of suture, for instance the number is greater than 1 and less than 5; the number of retainers per suture diameter in axial length of suture is greater than 3; the number of retainers per suture diameter in axial length of suture is greater than 3 and less than 5; the number of retainers per suture diameter in axial length of suture is greater than 4; the sum of the lengths of the retainers in 25 mm (an inch) of suture is greater than 25 mm (1 inch); the sum of the lengths of the retainers in 25 mm (an inch) of suture is greater than 25 mm (1 inch) and less than 100 mm (4 inches); the sum of the lengths of the retainers in 25 mm (an inch) of suture is greater than 50 mm (2 inches) and less than 100 mm (4 inches); the sum of the lengths of the retainers in 25 mm (an inch) of suture is greater than 75 mm (3 inches) and less than 100 mm (4 inches); the sum of the lengths of the retainers in 25mm (an inch) of suture is greater than 75 mm (3 inches); the suture thread is a monofilament suture into which the retainers are cut; the suture thread is a polypropylene suture into which the retainers are cut; the suture thread is a drawn polymeric fiber suture into which the retainers are cut; the suture has a needle at each end; the suture has a needle at one end and an anchor at one end; the suture has a needle at one end and an anchor at one end, wherein the anchor is an anchor selected from: a loop, a tack, a staple, a clip, a pledget, and a short barbed segment of suture without a needle; and/or the suture is a polypropylene monofilament of USP 2-0.

As a further example, a self-retaining medical device comprises: a USP 2-0 polypropylene monofilament suture having a longitudinal axis; a plurality of retainers, each retainer being formed by an angled cut into a section of the suture; each retainer having a retainer length greater than 400µm measured along said axis; the retainers being distributed in pairs, each pair comprising a first retainer and a second retainer at substantially the same position along the axis but on the opposite side of the suture; wherein for each pair of retainers there is at least one adjacent pair of retainers; and wherein the retainers of each pair of retainers are displaced by a pitch length no greater than about 550µm along the axis and substantially 90 degrees around said axis relative to the retainers of the adjacent pair of retainers. In this and other examples provided herein, the suture may optionally be further characterized by one, or any two or more not-inconsistent combinations of the following features, which are exemplary of features disclosed herein and therefore are non-limiting: the suture diameter is less than about 350µm, the retainer length is about 420µm and the pitch length is about 500µm; the retainers are characterized by an aspect ratio and the aspect ratio of the retainers is greater than 2.5; the aspect ratio of the retainers is greater than 3; the aspect ratio of the retainers is greater than 3.5; the aspect ratio of the retainers is greater than 3.5 but no greater than 6; the aspect ratio of the retainers is greater than 4; the aspect ratio of the retainers is greater than 5; the aspect ratio of the retainers is greater than 4 but no greater than 6; the aspect ratio of the retainers is greater than 5 but no greater than 7; the suture is characterized by the number of retainers per suture diameter in axial length of suture, for instance the number is greater than 1 and less than 5; the number of retainers per suture diameter in axial length of suture is greater than 3; the number of retainers per suture diameter in axial length of suture is greater than 3 and less than 5; the number of retainers per suture diameter in axial length of suture is greater than 4; the sum of the lengths of the retainers in 25 mm (an inch) of suture is greater than 25 mm (1 inch); the sum of the lengths of the retainers in 25 mm (an inch) of suture is greater than 25 mm (1 inch) and less than 100 mm (4 inches); the sum of the lengths of the retainers in 25 mm (an inch) of suture is greater than 50 mm (2 inches) and less than 100 mm (4 inches); the sum of the lengths of the retainers in 25 mm (an inch) of suture is greater than 75 mm (3 inches) and less than 100 mm (4 inches); the sum of the lengths of the retainers in 25 mm (an inch) of suture is greater than 75 mm (3 inches); the suture thread is a monofilament suture into which the retainers are cut; the suture thread is a polypropylene suture into which the retainers are cut; the suture thread is a drawn polymeric fiber suture into which the retainers are cut; the suture has a needle at each end; the suture has a needle at one end and an anchor at one end; and/or the suture has a needle at one end and an anchor at one end, wherein the anchor is an anchor selected from: a loop, a tack, a staple, a clip, a pledget, and a short barbed segment of suture without a needle.

The present disclosure also provides devices for forming a retainer on a suture or equivalent. For example, in one examples, a device is adapted to form a retainer on a suture having a suture diameter (SD) wherein the device comprises: an anvil adapted to support a suture; said anvil including a gap aligned with the suture and adapted for receiving at least a part of the suture; a spring adapted to hold the suture against said anvil and within said gap; and a cutting blade adapted to cut a retainer in a suture when the spring holds the suture against said anvil and within said gap. In this and other examples provided herein, the device may optionally be further characterized by one, or any two or more not-inconsistent combinations of the following features, which are exemplary of features disclosed herein and therefore are non-limiting: said gap is adjustable; said anvil includes a step which precludes contact between the suture and the anvil beyond the step; said step is located at an adjustable distance (D) from the spring; said step is located within two sutures diameters (SD) of the spring; said gap is adapted to receive a retainer that has been previously formed in said suture by said cutting blade; the gap has a width smaller than the suture diameter (SD) and greater than half the suture diameter (SD); the cutting blade passes within one suture diameter (SD) of the retainer when cutting a retainer; the cutting blade passes within one suture diameter (SD) of the step when cutting a retainer; the anvil has a surface for supporting the suture adjacent the gap and wherein the surface is curved; said anvil includes a first circular anvil segment and a second circular anvil segment and said gap is located between said first circular anvil segment and said second circular anvil segment; said cutting blade is adapted to be urged about across a longitudinal axis of a suture; the anvil includes a relief that communicates with said gap and is positioned after said cutting blade and adapted to receive retainers so that said retainers are not compressed by said anvil; said spring has a trailing edge and said relief defines a step, and wherein a proximity of the step to the trailing edge is adapted to allow for the creation of retainers at high density by providing support of the suture adjacent a cutting region associated with the blade while preventing interference between already formed retainers and said anvil; said anvil has a relief that defines a step that is located on the opposite side of the cutting blade from the spring and wherein, a distance between the cutting blade and said step is adapted for defining the density of retainer that can be formed; said anvil has a relief that defines a step and wherein the distance between the spring and the step is adjustable in order to adjust a density of retainers that can be created by said device on a suture; said anvil is rotatable and said anvil can be rotated to adjust the distance between the spring and the step; said anvil has a relief that defines a step and wherein the distance between the spring and the step is less than two suture diameters (SD); said anvil has a relief that defines a step; and wherein said cutting blade is adapted to cut a retainer on the suture thread between the spring and the step; said anvil has a relief that defines a step and wherein said cutting blade is adapted to cut a retainer on the suture thread between the spring and the step and wherein said cutting blade is adapted to cut a retainer on the suture thread at a distance from the step less than a pitch of the self-retaining suture; said gap is adapted to prevent already formed retainers from being crushed; said cutting blade is mounted in order to have two degrees of linear freedom of adjustment and two degrees of rotational freedom of adjustment relative to a suture; and the device further comprises a chuck adapted for holding suture, and/or wherein said chuck includes at least one degree of rotational freedom and one degree of linear freedom.

In another example, a device is adapted to form a plurality of retainers in a surgical filament comprising: an anvil adapted to support the surgical filament; said anvil including a gap aligned with the surgical filament adapted for receiving a portion of the surgical filament; a compressor adapted to push the suture against said anvil such that a portion of the surgical filament is received in the gap; and a retainer formation device adapted to form a retainer in the surgical filament adjacent the compressor. In this and other examples provided herein, the device may optionally be further characterized by one, or any two or more not-inconsistent combinations of the following features, which are exemplary of features disclosed herein and therefore are non-limiting: said gap is adjustable; said anvil includes a step which precludes contact between the surgical filament and the anvil beyond the step; ; said step is located at an adjustable distance (D) from the compressor; said step is located within two surgical filaments diameters (SD) of the compressor; said gap is adapted to receive a retainer that has been previously formed in said surgical filament by said retainer formation device; said gap has a width smaller than the surgical filament diameter (SD) and greater than half the surgical filament diameter (SD); the retainer formation device passes within one surgical filament diameter (SD) of the retainer when forming a retainer; the retainer formation device passes within one surgical filament diameter (SD) of the step when forming a retainer; the anvil has a surface for supporting the surgical filament adjacent the gap and wherein the surface is curved; said anvil includes a first circular anvil segment and a second circular anvil segment and said gap is located between said first circular anvil segment and said second circular anvil segment; said retainer formation device is adapted to be urged about across a longitudinal axis of a surgical filament; the anvil includes a relief that communicates with said gap and is positioned after said retainer formation device and adapted to receive retainers so that said retainers are not compressed by said anvil; said compressor is a spring which has a trailing edge and said relief defines a step, and wherein a proximity of the step to the trailing edge is adapted to allow for the creation of retainers at high density by providing support of the surgical filament adjacent a cutting region associated with the blade while preventing interference between already formed retainers and said anvil; said anvil has a relief that defines a step that is located on the opposite side of the retainer formation device from the compressor and wherein, a distance between the retainer formation device and said step is adapted for defining the density of retainer that can be formed; said anvil has a relief that defines a step and wherein the distance between the compressor and the step is adjustable in order to adjust a density of retainers that can be created by said device on a surgical filament; said anvil is rotatable and said anvil can be rotated to adjust the distance between the compressor and the step; said anvil has a relief that defines a step and wherein the distance between the compressor and the step is less than two surgical filament diameters (SD); said anvil has a relief that defines a step; and wherein said cutting blade is adapted to cut a retainer on the surgical filament thread between the compressor and the step; said anvil has a relief that defines a step; and wherein said cutting blade is adapted to cut a retainer on the surgical filament thread between the compressor and the step and wherein said retainer formation device is adapted to form a retainer on the surgical filament thread at a distance from the step less than a pitch of the self-retaining surgical filament; said gap is adapted to prevent already formed retainers from being crushed; said retainer formation device is mounted in order to have two degrees of linear freedom of adjustment and two degrees of rotational freedom of adjustment relative to a surgical filament; said device further comprising a chuck adapted for holding surgical filament, wherein said chuck includes at least one degree of rotational freedom and one degree of linear freedom; and/or the retainer formation device is a sapphire blade.

In another example directed to a device, a device is adapted to form a plurality of retainers along a suture, the retainers being spaced at a pitch (P) along the suture, and the suture having a suture diameter (SD), the device comprising: an anvil adapted to support the suture; a compressor adapted to push the suture against said anvil; a step in the anvil adapted to distance the anvil from the suture; a retainer formation device adapted to form a retainer in a suture at a position between the compressor and the step; and wherein said retainer formation device is positioned so as to form a retainer at a distance from the step which is less than the pitch (P). In this and other examples provided herein, the device may optionally be further characterized by one, or any two or more not-inconsistent combinations of the following features, which are exemplary of features disclosed herein and therefore are non-limiting: the device is adapted to form a plurality of retainers along a suture having a suture diameter (SD) less than about 300µm, the retainers being spaced at a pitch (P) less than about 500µm along the suture, wherein said retainer formation device is positioned so as to from a retainer at a distance from the step which is less than about 500µm; said compressor contacts the suture to push the suture against the anvil within 1000µm from the step along the suture; the device is adapted to form a plurality of retainers along a suture having a suture diameter (SD) less than about 100µm, the retainers being spaced at a pitch (P) less than about 100µm along the suture, wherein said retainer formation device is positioned so as to form a retainer at a distance from the step which is less than about 100µm; said compressor contacts the suture to push the suture against the anvil within 200µm from the step along the suture; the device is adapted to form a plurality of retainers along a suture having a suture diameter (SD) less than about 50µm, the retainers being spaced at a pitch (P) less than about 70µm along the suture, wherein said retainer formation device is positioned so as to form a retainer at a distance from the step which is less than about 70µm; said retainer formation device is positioned so as to form a retainer at an adjustable distance from the step; said compressor contacts the suture to push the suture against the anvil within 140µm from the step along the suture; the anvil comprises a channel aligned with the suture and extending from the step towards the compressor, wherein the channel has a width less than the diameter of the suture and a depth sufficient to prevent interference with a previously-formed retainer located on an opposite side of the suture from a retainer currently being formed; the channel is at least as long as the pitch (P) of the suture and the width of the channel is optionally adjustable; an/or the anvil comprises a first anvil component and a second anvil component and wherein the channel comprises a gap between the first anvil component and the second anvil component adjacent the step, where the gap is optionally adjustable.

In one more example, there is provided a device adapted to form a plurality of retainers along a surgical filament having a diameter SD, wherein the device comprises: a compressor, a blade, and an anvil; the anvil comprising a support surface adapted for supporting the suture; the support surface comprising a channel aligned with a longitudinal axis of the suture; the channel having a width W greater than 0.5SD and less than 0.9SD; the support surface having a step which intersects the channel; the compressor being positioned to push the suture against the support surface of the anvil within a distance L of the step, where L<4SD; and the blade being configured to cut a retainer in the surgical filament at a position between the compressor and the step. In this and other examples provided herein, the device may optionally be further characterized by one, or any two or more not-inconsistent combinations of the following features, which are exemplary of features disclosed herein and therefore are non-limiting: the device is adapted to form a plurality of retainers along a surgical filament having a diameter no greater than about 500µm; the device is adapted to form a plurality of retainers along a surgical filament having a diameter SD no greater than about 300µm; the device is adapted to form a plurality of retainers along a surgical filament having a diameter SD no greater than about 100µm; the device is adapted to form a plurality of retainers along a surgical filament having a diameter SD no greater than about 50µm; the density of retainers is about at least 630 retainers per cm (1600 retainers per inch) along a length of the suture thread; the retainers are distributed at a density of between 79 retainers per cm (200 retainers per inch) and 630 retainers per cm (1,600 retainers per inch); and/or the retainers are characterized by an aspect ratio and the aspect ratio of the retainers is greater than 2.5; the aspect ratio of the retainers is greater than 3; the aspect ratio of the retainers is greater than 3.5; the aspect ratio of the retainers is greater than 3.5 but no greater than 6; the aspect ratio of the retainers is greater than 4; the aspect ratio of the retainers is greater than 5; the aspect ratio of the retainers is greater than 4 but no greater than 6; the aspect ratio of the retainers is greater than 5 but no greater than 7.

In addition to sutures and the like, and devices for preparing sutures and the like, there is herein provided methods for creating such sutures and the like. For example, there is herein disclosed a method for creating a plurality of retainers on a suture thread having a longitudinal axis and a suture diameter (SD) wherein the method comprises: (a) securing the suture thread to a support; (b) operating a cutting blade to move along a cutting axis substantially perpendicular to the longitudinal axis of the suture thread to cut a retainer of length L in the suture thread; (c) releasing the suture thread from the support; (d) rotating the suture thread around the longitudinal axis of the suture thread; (e) repeating steps (a), (b) and (c) at least once; (f) subsequent to step (e), releasing the suture thread from the support and advancing the suture thread a distance P along its longitudinal axis relative to the cutting blade; and (g) repeating steps (a) through (f) at least ten times. In this and other examples provided herein, the method may optionally be further characterized by one, or any two or more not-inconsistent combinations of the following features, which are exemplary of features disclosed herein and therefore are non-limiting: L is greater than 20% of SD and less than 200% of SD; P is less than 500µm; P is less than about 100µm; P is less than about 70µm; P is no greater than 2L; P is no greater than 1.5L; P is no greater than 1.2L; the retainers are characterized by an aspect ratio and the aspect ratio of the retainers is greater than 2.5; the aspect ratio of the retainers is greater than 3; the aspect ratio of the retainers is greater than 3.5; the aspect ratio of the retainers is greater than 3.5 but no greater than 6; the aspect ratio of the retainers is greater than 4; the aspect ratio of the retainers is greater than 5; the aspect ratio of the retainers is greater than 4 but no greater than 6; the aspect ratio of the retainers is greater than 5 but no greater than 7; step (e) comprises repeating steps (a), (b) and (c) at least three times prior to step (f); the support comprises an anvil having a channel aligned with the suture and smaller in width than the suture diameter (SD) and wherein step (a) comprises approximating the anvil to a compressor to trap the suture between the compressor and the anvil with a segment of the suture thread received within the channel and thereby secure the suture thread to the support; and/or the support comprises an anvil having a channel aligned with the suture and smaller in width than the suture diameter (SD) and wherein step (a) comprises approximating the anvil to a spring to trap the suture between the compressor and the spring with a segment of the suture thread received within the channel and thereby secure the suture thread to the support.

The details of one or more embodiments are set forth in the description below. Other features, objects and advantages will be apparent from the description, the drawings, and the claims.

### BRIEF DESCRIPTION OF THE DRAWINGS

Features of the invention, and the nature and various advantages thereof will be apparent from the accompanying drawings and the following detailed description of various embodiments of the invention.
FIGS. 1A and 1B are views of a self-retaining suture.
FIGS. 1C and 1D are enlarged sectional views of the suture thread of the self-retaining suture of FIGS. 1A and 1B illustrating parameters of the retainers and retainer distribution.
FIGS. 1E, 1F, 1G, 1H and 1I are views of alternative configurations of self-retaining suture.
FIG. 2A is a plain view of an apparatus for forming retainers on a suture thread.
FIG. 2B is a method of operating the apparatus of FIG. 2A.
FIG. 3A is a top view of a cutting assembly used in the apparatus of FIG. 2A.
FIG. 3B is a side view of the cutting assembly of FIG. 3A.
FIG. 3C is a side view of the cutting assembly of FIG. 3A illustrating relative movement of the main components.
FIG 3D is a partial perspective view of the cutting assembly of FIG 3A.
FIG. 3E is an enlarged perspective view of the cutting assembly of FIG. 3A.
FIG. 3F is a partial sectional view of the cutting assembly of FIG. 3A.
FIG. 3G is an enlarged top view of the cutting assembly of FIG. 3A.
FIG. 3H is a schematic representation illustrating movement of the cutting blade in response to adjustment of one cutting assembly parameter.
FIG. 4A is a perspective view of the cutting head of the cutting assembly of FIG. 3A.
FIG. 4B is an enlarged view of the blade holder and blade of FIG. 4A.
FIG. 4C is an enlarged view of the blade of FIG. 4A.
FIG. 4D is an enlarged view of the blade of FIG. 4A.
FIG. 4E is an alternative blade.
FIG. 4F is an alternative blade.
FIGS. 5A is an enlarged view of the suture thread retaining spring of the cutting assembly of FIG. 3A.
FIG. 5B is a sectional view of the spring of FIG. 5A.
FIG. 5C is a perspective view of the spring of FIG. 5A.
FIG. 6A is a perspective view of the chuck assembly of the apparatus of FIG. 2A.
FIG. 6B is a partial perspective view of the chuck assembly of FIG. 6A.
FIG. 6C is a partial perspective alternate view of the chuck assembly of FIG. 6A.
FIG. 7A is a perspective view of a self-retaining suture having retainers distributed in a single helix pattern.
FIG. 7B is a perspective view of a self-retaining suture having retainers distributed in a double helix pattern.
FIG. 7C is a perspective view of a self-retaining suture having retainers distributed in a quadra-helix pattern.
FIG. 7D illustrates a shape of a single retainer.
FIG. 7E illustrates an alternate shape of a single retainer.
FIG. 7F illustrates another shape of a single retainer.
FIG. 7G is a perspective view of a self-retaining suture having retainers distributed in in-phase double helix pattern.
FIG. 7H shows a sectional view of the self-retaining suture of FIG. 7G.
FIGS. 8A-8G show images of a 2-0 quadra-helix self-retaining sutures .
FIG. 8H shows an image of a 2-0 double-helix self-retaining suture.
FIG. 8l shows an image of a 6-0 double-helix self-retaining suture.

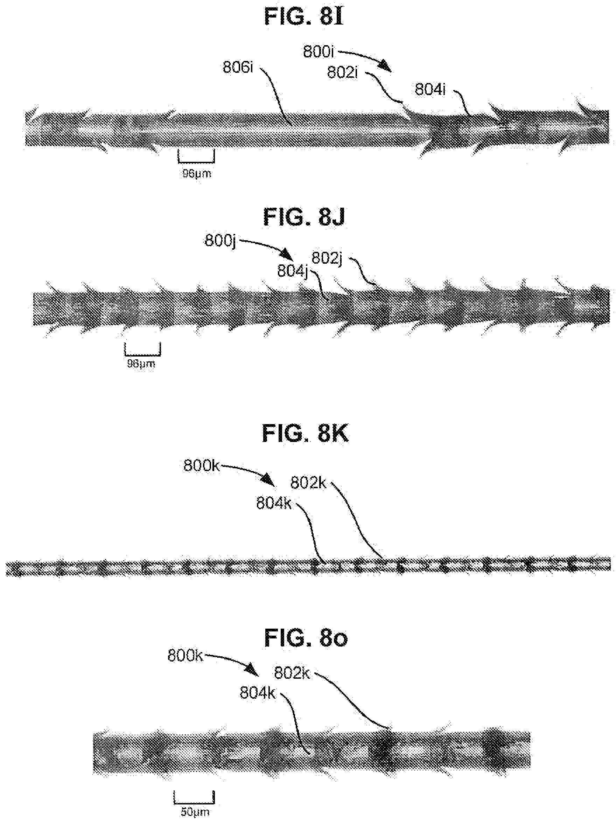

FIG. 8J shows an image of a 6-0 quadra-helix self-retaining suture.
FIG. 8K shows an image of an 8-0 quadra-helix self-retaining suture.

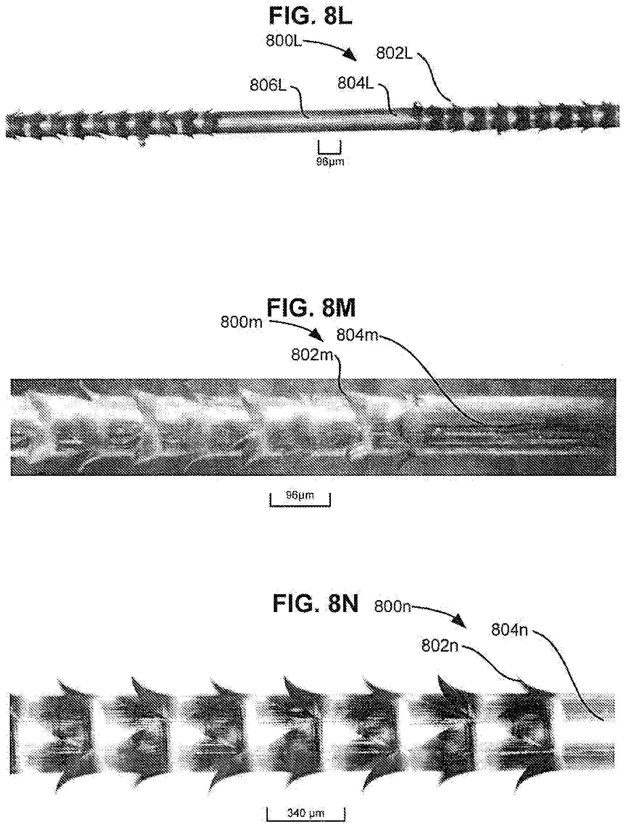

FIG. 8L shows an image of a 6-0 quadra-helix self-retaining suture.
FIG. 8M shows an image of a 6-0 quadra-helix self-retaining suture.
FIGS. 8N shows an image of a 2-0 quadra-helix self-retaining suture.
FIG. 8o shows an enlarged view of suture of FIG. 8K.

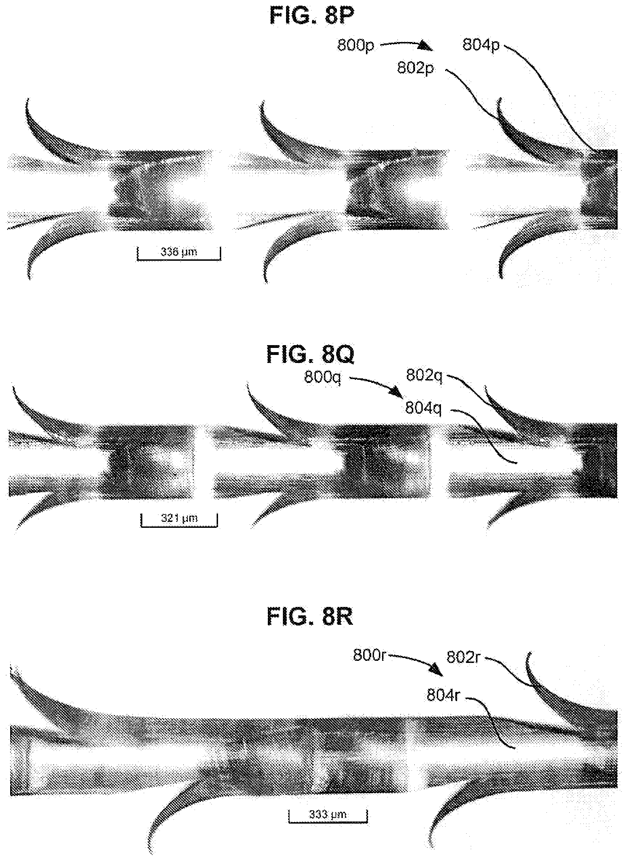

FIG. 8P shows an image of a 2-0 double-helix self-retaining suture.
FIG. 8Q shows an image of a 2-0 double-helix self-retaining suture.
FIG. 8R shows an image of a 2-0 double-helix self-retaining suture.

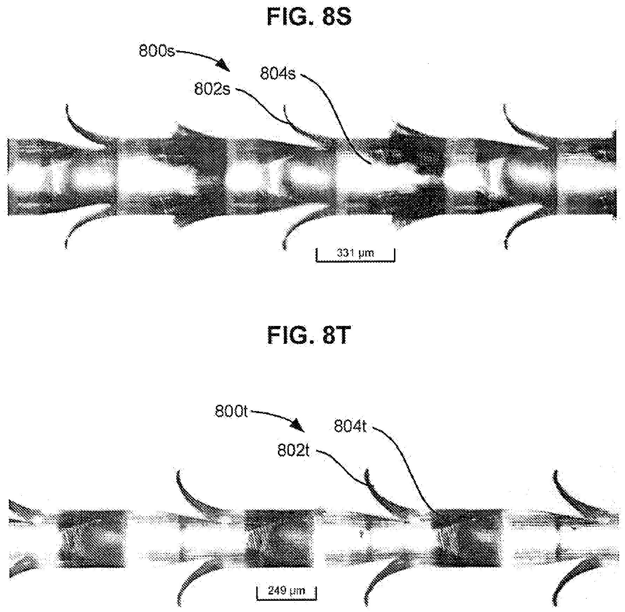

FIG. 8S shows an image of a 2-0 quadra-helix self-retaining suture.
FIG. 8T shows an image of a 3-0 double-helix self-retaining suture.

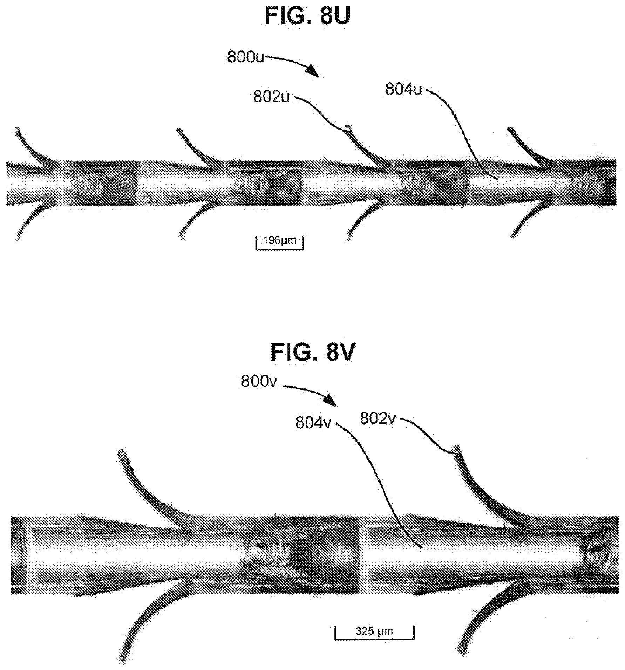

FIG. 8U shows an image of a 4-0 double-helix self-retaining suture.
FIG. 8V shows an image of a 2-0 double-helix self-retaining suture.

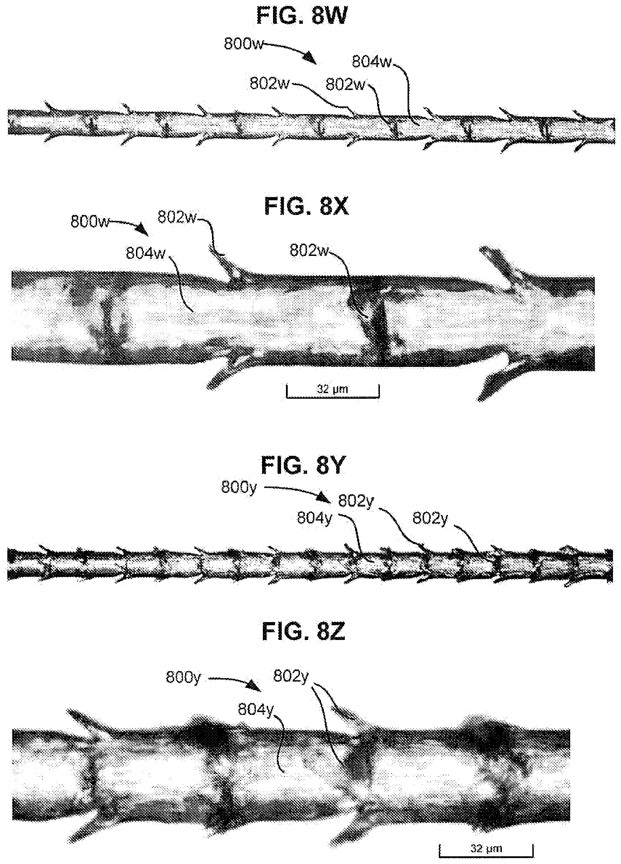

FIG. 8W shows an image of a 10-0 double-helix self-retaining suture.
FIG. 8X shows an enlarged view of the suture of FIG. 8W.
FIG. 8Y shows an image of a 10-0 quadra-helix self-retaining suture.
FIG. 8Z shows an enlarged view of the suture of FIG. 8Y.

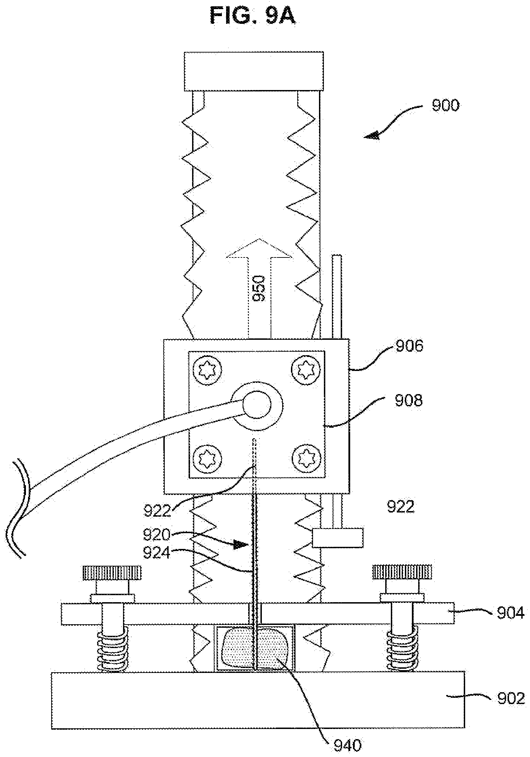

FIG. 9A shows a schematic representation of apparatus for testing tissue holding strength of self-retaining sutures.

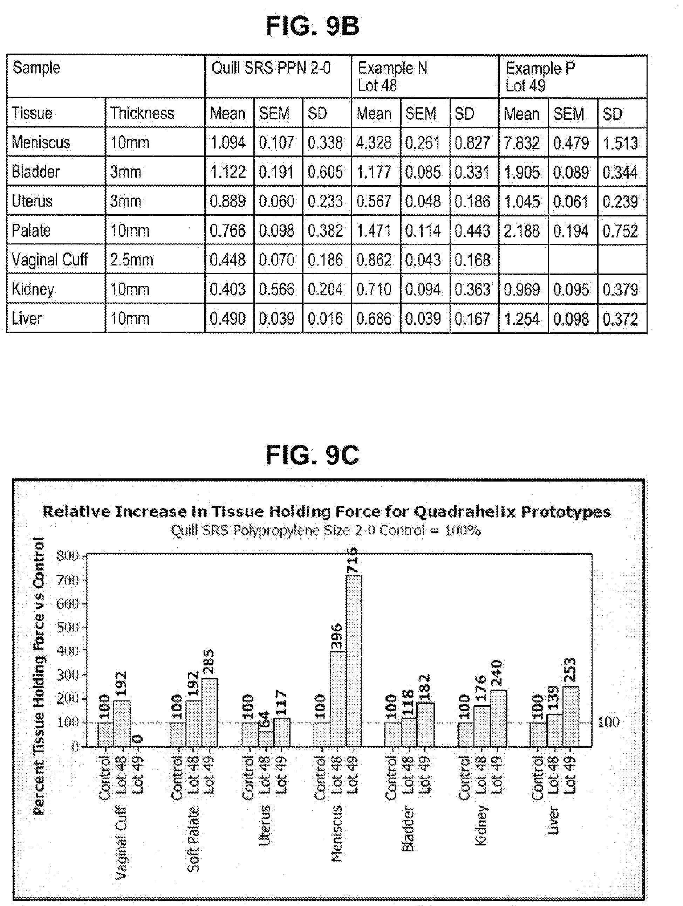

FIG. 9B is a table of results of analysis of tissue holding strength of self-retaining suture.
FIG. 9C is a chart of results of analysis of tissue holding strength of self-retaining suture.

### DETAILED DESCRIPTION

### Definitions

Definitions of certain terms that may be used hereinafter include the following.

"Self-retaining suture" refers to a surgical suture that includes features on the suture thread for engaging tissue without the need for a knot or suture anchor. A "self-retaining suture" may also include devices for deploying the suture into tissue. Such deployment devices include, without limitation, suture needles and other deployment devices as well as sufficiently rigid and sharp ends on the suture itself to penetrate tissue.

"Tissue retainer" (or simply "retainer") or "barb" refers to a physical feature of a suture thread which is adapted to mechanically engage tissue and resist movement of the suture in at least one axial directions. By way of example only, tissue retainer or retainers can include hooks, projections, barbs, darts, extensions, bulges, anchors, protuberances, spurs, bumps, points, cogs, tissue engagers, traction devices, surface roughness, surface irregularities, surface defects, edges, facets and the like. In certain configurations, tissue retainers are adapted to engage tissue to resist movement of the suture in a direction other than the direction in which the suture is deployed into the tissue by the surgeon, by being oriented to substantially face the deployment direction. In some embodiments the retainers lie flat when pulled in the deployment direction and open or "fan out" when pulled in a direction contrary to the deployment direction. As the tissue-penetrating end of each retainer faces away from the deployment direction when moving through tissue during deployment, the tissue retainers should not catch or grab tissue during this phase. Once the self-retaining suture has been deployed, a force exerted in another direction (often substantially opposite to the deployment direction) causes the retainers to be displaced from the deployment position (i.e. resting substantially along the suture body), forces the retainer ends to open (or "fan out") from the suture body in a manner that catches and penetrates into the surrounding tissue, and results in tissue being caught between the retainer and the suture body; thereby "anchoring" or affixing the self-retaining suture in place. In certain other embodiments, the tissue retainers may be configured to permit motion of the suture in one direction and resist movement of the suture in another direction without fanning out or deploying. In each of the sutures and retainers of the present invention, in one optional embodiment, the retainers may be characterized as a plurality of barbs extending from the periphery of the body and tapering from a broad base to a narrow tip. In addition, or also optionally, the retainers may be characterized as a plurality of barbs that yield toward the suture body during movement of the suture through the tissue in the desired direction of movement of the suture through the tissue, and the barbs resist movement of the suture through the tissue in a direction opposite the desired direction of movement of the suture. Typically, a needle will be located at an end of the suture, and the barbs will yield toward the suture body as the suture is pulled through tissue in the direction that the needle is moving. In certain other configurations, the tissue retainer may be configured or combined with other tissue retainers to resist motion of the suture in either direction. Typically, a suture having such retainers is deployed through a device such as a cannula which prevents contact between the retainers and the tissue until the suture is in the desired location.

"Retainer configurations" refers to configurations of tissue retainers and can include features such as size, shape, flexibility, surface characteristics, and so forth. These are sometimes also referred to as "barb configurations".

"Retainer distribution" and "retainer pattern" refers to the arrangement of retainers along and around a suture thread and can include features such as density and orientation.

"Bidirectional suture" refers to a self-retaining suture having retainers oriented in one direction at one end and retainers oriented in the other direction at the other end. A bidirectional suture is typically armed with a needle at each end of the suture thread. A bidirectional suture may have a transition segment.

"Transition segment" refers to a retainer-free (barb-free) portion of a bidirectional suture located between a first set of retainers (barbs) oriented in one direction and a second set of retainers (barbs) oriented in another direction. The transition segment can be at about the midpoint of the self-retaining suture, or closer to one end of the self-retaining suture to form an asymmetrical self-retaining suture.

"Suture thread" refers to the filamentary body component of a suture or suture. The suture thread may be a monofilament, or contain multiple filaments as in a braided suture. The suture thread may be made of any suitable biocompatible material, and may be further treated with any suitable biocompatible material, whether to enhance the sutures' strength, resilience, longevity, or other qualities, or to equip the sutures to fulfill additional functions besides joining tissues together, repositioning tissues, or attaching foreign elements to tissues.

"Monofilament suture" refers to a suture comprising a monofilamentary suture thread.

"Braided suture" refers to a suture comprising a multifilamentary suture thread. The filaments in such suture threads are typically braided, twisted, or woven together.

"Degradable suture" (also referred to as "biodegradable suture" or "absorbable suture") refers to a suture which, after introduction into a tissue is broken down and absorbed by the body. Typically, the degradation process is at least partially mediated by, or performed in, a biological system. "Degradation" refers to a chain scission process by which a polymer chain is cleaved into oligomers and monomers. Chain scission may occur through various mechanisms, including, for example, by chemical reaction (e.g., hydrolysis, oxidation/reduction, enzymatic mechanisms or a combination of these) or by a thermal or photolytic process. Degradable suture material may include polymers such as polyglycolic acid, copolymers of glycolide and lactide, copolymers of trimethylene carbonate and glycolide with diethylene glycol (e.g., MAXONTM, Tyco Healthcare Group), terpolymer composed of glycolide, trimethylene carbonate, and dioxanone (e.g., BIOSYNTM [glycolide (60%), trimethylene carbonate (26%), and dioxanone (14%)], Tyco Healthcare Group), copolymers of glycolide, caprolactone, trimethylene carbonate, and lactide (e.g., CAPROSYNTM, Tyco Healthcare Group). A dissolvable suture can also include partially deacetylated polyvinyl alcohol. Polymers suitable for use in degradable sutures can be linear polymers, branched polymers or multi-axial polymers. Examples of multi-axial polymers used in sutures are described in U.S. Patent Application Publication Nos. 2002/0161168, 2004/0024169, and 2004/0116620. Sutures made from degradable suture material lose tensile strength as the material degrades. Degradable sutures can be in either a braided multifilament form or a monofilament form.

"Non-degradable suture" (also referred to as "non-absorbable suture") refers to a suture comprising material that is not degraded by chain scission such as chemical reaction processes (e.g., hydrolysis, oxidation/reduction, enzymatic mechanisms or a combination or these) or by a thermal or photolytic process. Non-degradable suture material includes polyamide (also known as nylon, such as nylon 6 and nylon 6.6), polyester (e.g., polyethylene terephthlate), polytetrafluoroethylene (e.g., expanded polytetrafluoroethylene), polyether-ester such as polybutester (block copolymer of butylene terephthalate and polytetra methylene ether glycol), polyurethane, metal alloys, metal (e.g., stainless steel wire), polypropylene, polyethelene, silk, and cotton. Sutures made of non-degradable suture material are suitable for applications in which the suture is meant to remain permanently or is meant to be physically removed from the body.

"Suture diameter" refers to the diameter of the body of the suture. It is to be understood that a variety of suture lengths may be used with the sutures described herein and that while the term "diameter" is often associated with a circular periphery, it is to be understood herein to indicate a cross-sectional dimension associated with a periphery of any shape. Suture sizing is based upon diameter. United States Pharmacopeia ("USP") designation of suture size runs from 0 to 7 in the larger range and 1-0 to 11-0 in the smaller range; in the smaller range, the higher the value preceding the hyphenated zero, the smaller the suture diameter. The actual diameter of a suture will depend on the suture material, so that, by way of example, a suture of size 5-0 and made of collagen will have a diameter of 0.15 mm, while sutures having the same USP size designation but made of a synthetic absorbable material or a non-absorbable material will each have a diameter of 0.1 mm. The selection of suture size for a particular purpose depends upon factors such as the nature of the tissue to be sutured and the importance of cosmetic concerns; while smaller sutures may be more easily manipulated through tight surgical sites and are associated with less scarring, the tensile strength of a suture manufactured from a given material tends to decrease with decreasing size. It is to be understood that the sutures and methods of manufacturing sutures disclosed herein are suited to a variety of diameters, including without limitation 7, 6, 5, 4, 3, 2, 1, 0, 1-0, 2-0, 3-0, 4-0, 5-0, 6-0, 7-0, 8-0, 9-0, 10-0, 11-0 and 12-0.

"Suture deployment end" refers to an end of the suture to be deployed into tissue; one or both ends of the suture may be suture deployment ends. The suture deployment end may be attached to a deployment device such as a suture needle, or may be sufficiently sharp and rigid to penetrate tissue on its own.

"Needle attachment" refers to the attachment of a needle to a suture requiring same for deployment into tissue, and can include methods such as crimping, swaging, using adhesives, and so forth. The suture thread is attached to the suture needle using methods such as crimping, swaging and adhesives. Attachment of sutures and surgical needles is described in U.S. Patent Nos. 3,981,307, 5,084,063, 5,102,418, 5,123,911, 5,500,991, 5,722,991, 6,012,216, and 6,163,948, and U.S. Patent Application Publication No. US 2004/0088003). The point of attachment of the suture to the needle is known as the swage. "Armed suture" refers to a suture having a suture needle on at least one suture deployment end.

"Suture needle" refers to needles used to deploy sutures into tissue, which come in many different shapes, forms and compositions. There are two main types of needles, traumatic needles and atraumatic needles. Traumatic needles have channels or drilled ends (that is, holes or eyes) and are supplied separate from the suture thread and are threaded on site. Atraumatic needles are eyeless and are attached to the suture at the factory by swaging or other methods whereby the suture material is inserted into a channel at the blunt end of the needle which is then deformed to a final shape to hold the suture and needle together. As such, atraumatic needles do not require extra time on site for threading and the suture end at the needle attachment site is generally smaller than the needle body. In the traumatic needle the thread comes out of the needle's hole on both sides and often the suture rips the tissues to a certain extent as it passes through. Most modern sutures are swaged atraumatic needles. Atraumatic needles may be permanently swaged to the suture or may be designed to come off the suture with a sharp straight tug. These "pop-offs" are commonly used for interrupted sutures, where each suture is only passed once and then tied. For barbed sutures that are uninterrupted, these atraumatic needles are preferred.

Suture needles may also be classified according to the geometry of the tip or point of the needle. For example, needles may be (i) "tapered" whereby the needle body is round and tapers smoothly to a point; (ii) "cutting" whereby the needle body is triangular and has sharpened cutting edge on the inside; (iii) "reverse cutting" whereby the cutting edge is on the outside; (iv) "trocar point" or "taper cut" whereby the needle body is round and tapered, but ends in a small triangular cutting point; (v) "blunt" points for sewing friable tissues; (vi) "side cutting" or "spatula points" whereby the needle is flat on top and bottom with a cutting edge along the front to one side (these are typically used for eye surgery).

Suture needles may also be of several shapes including, (i) straight, (ii) half curved or ski, (iii) 1/4 circle, (iv) 3/8 circle, (v) 1/2 circle, (vi) 5/8 circle, (v) and compound curve. The sutures described herein may be deployed with a variety of needle types (including without limitation curved, straight, long, short, micro, and so forth), needle cutting surfaces (including without limitation, cutting, tapered, and so forth), and needle attachment techniques (including without limitation, drilled end, crimped, and so forth). Moreover, the sutures described herein may themselves include sufficiently rigid and sharp ends so as to dispense with the requirement for deployment needles altogether. Suturing needles are described, for example, in U.S. Patent Nos. 6,322,581; 6,214,030; 5,464,422; 5,941,899; 5,425,746; 5,306,288; 5,156,615; 5,312,422; 7,063,716; 6,129,741; 5,897,572; 5,676,675; and 5,693,072.

"Needle diameter" refers to the diameter of a suture deployment needle at the widest point of that needle. While the term "diameter" is often associated with a circular periphery, it is to be understood herein to indicate a cross-sectional dimension associated with a periphery of any shape. In preferred embodiments of self-retaining suture, the needle diameter is less than the maximum diameter/cross-sectional dimension of the retainers on the suture.

"Wound closure" refers to a surgical procedure for closing of a wound. An injury, especially one in which the skin or another external or internal surface is cut, torn, pierced, or otherwise broken is known as a wound. A wound commonly occurs when the integrity of any tissue is compromised (e.g., skin breaks or burns, muscle tears, or bone fractures). A wound may be caused by an act, such as a puncture, fall, or surgical procedure; by an infectious disease; or by an underlying medical condition. Surgical wound closure facilitates the biological event of healing by joining, or closely approximating, the edges of those wounds where the tissue has been torn, cut, or otherwise separated. Surgical wound closure directly apposes or approximates the tissue layers, which serves to minimize the volume new tissue formation required to bridge the gap between the two edges of the wound. Closure can serve both functional and aesthetic purposes. These purposes include elimination of dead space by approximating the subcutaneous tissues, minimization of scar formation by careful epidermal alignment, and avoidance of a depressed scar by precise eversion of skin edges.

"Tissue elevation procedure" refers to a surgical procedure for repositioning tissue from a lower elevation to a higher elevation (i.e. moving the tissue in a direction opposite to the direction of gravity). The retaining ligaments of the face support facial soft tissue in the normal anatomic position. However, with age, gravitational effects and loss of tissue volume effect downward migration of tissue, and fat descends into the plane between the superficial and deep facial fascia, thus allowing facial tissue to sag. Face-lift procedures are designed to lift these sagging tissues, and are one example of a more general class of medical procedure known as a tissue elevation procedure. More generally, a tissue elevation procedure reverses the appearance change that results from effects of aging and gravity over time, and other temporal effects that cause tissue to sag, such as genetic effects. It should be noted that tissue can also be repositioned without elevation; in some procedures tissues are repositioned laterally (away from the midline), medially (towards the midline) or inferiorly (lowered) in order to restore symmetry (i.e. repositioned such that the left and right sides of the body "match").

"Medical device" or "implant" refers to any object placed in the body for the purpose of restoring physiological function, reducing/alleviating symptoms associated with disease, and/or repairing and/or replacing damaged or diseased organs and tissues. While normally composed of biologically compatible synthetic materials (e.g., medical-grade stainless steel, titanium and other metals or polymers such as polyurethane, silicon, PLA, PLGA and other materials) that are exogenous, some medical devices and implants include materials derived from animals (e.g., "xenografts" such as whole animal organs; animal tissues such as heart valves; naturally occurring or chemically-modified molecules such as collagen, hyaluronic acid, proteins, carbohydrates and others), human donors (e.g., "allografts" such as whole organs; tissues such as bone grafts, skin grafts and others), or from the patients themselves (e.g., "autografts" such as saphenous vein grafts, skin grafts, tendon/ligament/muscle transplants). Medical devices that can be used in procedures in conjunction with the present invention include, but are not restricted to, orthopedic implants (artificial joints, ligaments and tendons; screws, plates, and other implantable hardware), dental implants, intravascular implants (arterial and venous vascular bypass grafts, hemodialysis access grafts; both autologous and synthetic), skin grafts (autologous, synthetic), tubes, drains, implantable tissue bulking agents, pumps, shunts, sealants, surgical meshes (e.g., hernia repair meshes, tissue scaffolds), fistula treatments, spinal implants (e.g., artificial intervertebral discs, spinal fusion devices, etc.) and the like.

### Self-Retaining Sutures

As discussed above, the present invention provides self-retaining sutures and apparatus and methods for manufacturing self-retaining sutures and methods of using self-retaining sutures in surgical procedures.

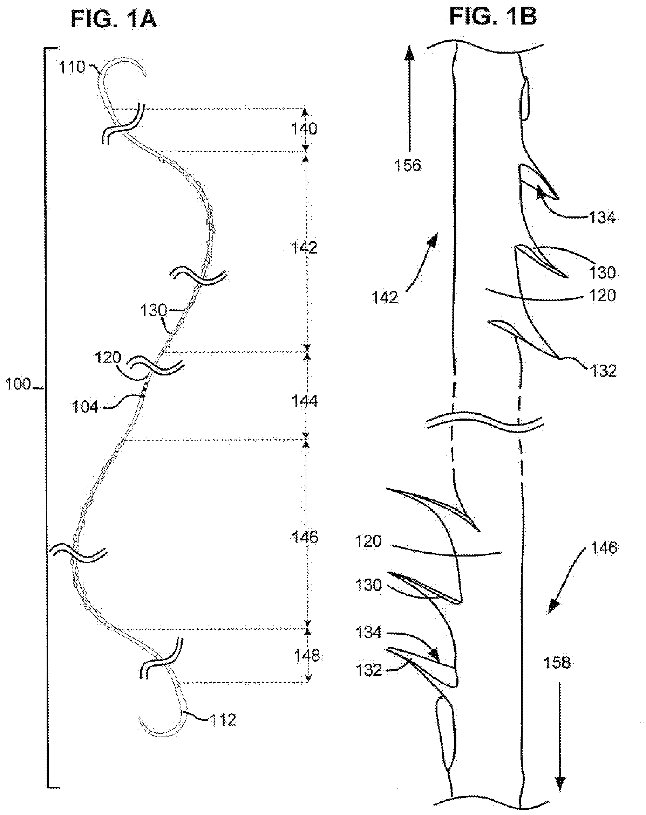

FIG. 1A illustrates an embodiment of a bidirectional self-retaining suture 100. Self-retaining suture 100 includes needles 110, 112 attached to suture thread 120. Self-retaining suture 100 includes a plurality of retainers 130 distributed on the surface of a suture thread 120. The retainers 130 are elevated as shown in FIG. 1A. In lead-in region 140 of suture thread 120 there are no retainers 130. In region 142 of suture thread 120 there are a plurality of retainers 130 arranged such that the suture can be moved through tissue in the direction of needle 110 but resists movement in the direction of needle 112. In transition region 144, there are no retainers 130. In region 146, there is a plurality of retainers 130 arranged such that the suture can be moved through tissue in the direction of needle 112 but resists movement in the direction of needle 110. In lead-in region 148 of suture thread 120 there are no retainers 130. A break is shown in each of regions 140, 142, 144, 146 and 148 to indicate that the length of each region may be varied and selected depending upon the application for which the suture is intended to be used. A self-retaining suture can, in some embodiments, include visible or visualizable markings indicating, for example, the presence, absence and/or orientation of retainers in a region of the suture. Thus, for example, the bidirectional self-retaining suture 100 of FIG. 1A includes visible markings 104 on the transition region 144 which allow a surgeon to identify the location of transition region 144.

Although a bidirectional self-retaining suture 100 is illustrated, the present invention includes self-retaining sutures of a wide variety of suture thread, retainer and needle configurations as described above. In alternative embodiments, for example, a self-retaining suture is provided with an anchor on one end of the suture. The anchor can take the form of a loop, bar, hook, pledget or other structural feature which allows the end of the suture to be fixed to tissue and/or prevents the end of the suture from being drawn through tissue. The anchor can be formed by manipulation of the suture material (e.g. a loop) or can be formed separately and secured to the suture material (e.g. a pledget). Likewise the configuration of each of needles 110 and 112 can be any of the range of different surgical needles developed for use in different applications. Needles 110 and 112 may have the same configuration or different configurations.

FIG. 1B illustrates a magnified view of self-retaining suture 100 in region 142. As shown in FIG. 1B, a plurality of retainers 130 is distributed on the surface of suture thread 120. The affixation of self-retaining sutures after deployment in tissue entails the penetration of retainer tips 132 into the surrounding tissue resulting in tissue being caught between the retainer 130 and the body of suture thread 120. The inner retainer surface 134 of the retainer 130 that is in contact with the tissue that is caught between the retainer 130 and the body of suture thread 120, is referred to herein as the "tissue engagement surface" or "inner retainer surface." As illustrated in FIG. 1B, each retainer 130 has a tip 132 and inner retainer surface 134. When self-retaining suture 100 is moved in the direction of arrow 156, retainers 130 in region 142 lie flat against the body of suture thread 120. However, when self-retaining suture 100 is moved in the direction of arrow 158, tips 132 of retainers 130 in region 142 engage tissue surrounding suture thread 120 and causes retainers 130 to fan out from suture thread 120 and engage the tissue with inner retainer surface 134 thereby preventing movement of the suture in that direction. In region 146, there is a plurality of retainers 130 arranged such that the suture can be moved through tissue in the direction of arrow 158 but resists movement in the direction of arrow 156.

Self-retaining sutures of the present invention may be made by cutting retainers 130 into the surface of a suture thread 120. In specific embodiments, polymeric thread or filaments may be manufactured for the suture body, and the retainers can be subsequently cut or formed on the suture body. The retainers 130 can be cut mechanically using a blade. During cutting either the blade or the suture thread may be moved, or both may be moved, to control the size, shape and depth of cut. The parameters of the cut control the shape of the resulting retainer 130.

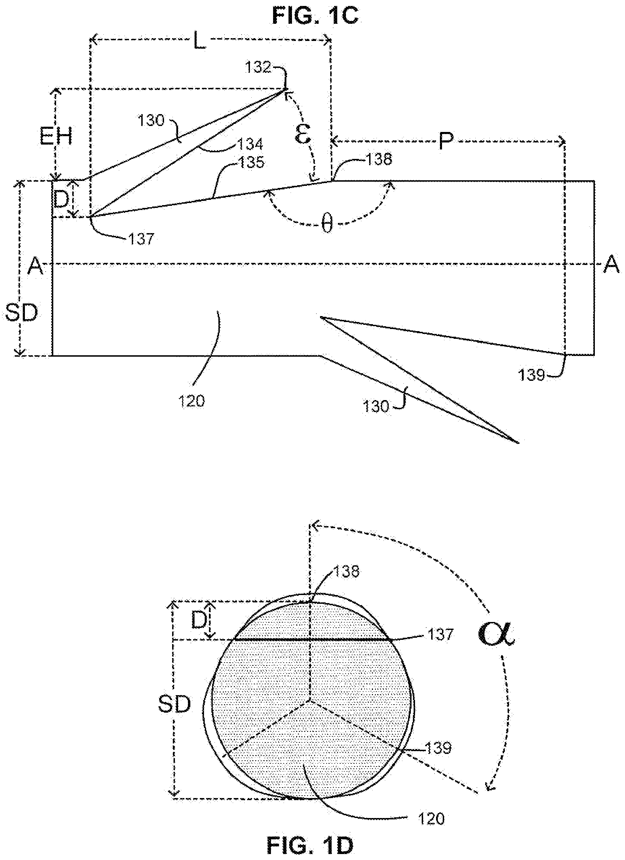

FIG. 1C shows a sectional diagram through a retainer 130. Note that where retainer 130 is cut into suture thread 120 the retainer leaves a cut-out depression 135. The cut-out depression 135 has a cut tip 138 which corresponds with the tip 132 of the retainer 130. Retainer 130 is shown elevated above suture thread 120 in order to show the parameters related to the retainer and elevation of the retainer. The parameters shown in FIG. 1C include the longitudinal axis of the suture A-A, the suture diameter SD, the retainer length L, the retainer cut depth D, the retainer cut angle Θ (theta), the retainer elevation angle ε (epsilon) and the retainer pitch P. The retainer length L is measured along the longitudinal axis of the suture. The pitch P is the distance between adjacent retainers measured along the longitudinal axis; the pitch P can be measured as the distance along the axis of the suture from one cut-tip 138 to the adjacent cut-tip 139. The retainer cut angle Θ is the angle between the cut depression 135 and the longitudinal axis A-A surface of suture thread 120. Retainer elevation angle ε is the angle between the inner retainer surface 134 and the surface of the cut-out depression surface 135. The term aspect ratio can be applied to a retainer to describe the ratio of the retainer length to the depth of cut. Thus, for example, the aspect ratio of retainer 130 is L/D. The spirality angle α is the angle of rotation about the longitudinal axis between adjacent cut tips 138, 139. Where the retainers are on opposite sides of suture thread 120, as shown in FIG. 1C, the spirality angle α is 180 degrees.

FIG. 1D shows a section of an alternative configuration looking along the long axis. As shown in FIG. 1D, the spirality angle α is 120 degrees. FIG. 1D also shows a straight line illustrating the position of the base 137 of cut-out depression 135. For a straight cut such as shown in FIG 1D, the cut depth D is the maximum distance between base 137 and the surface of suture thread 120. The geometry of retainer 130 (retainer cut angle, retainer cut depth, retainer cut length, retainer cut distance, etc.) and/or the spatial arrangement of the retainers 130 may be varied to enhance engagement of tissue by the retainers.

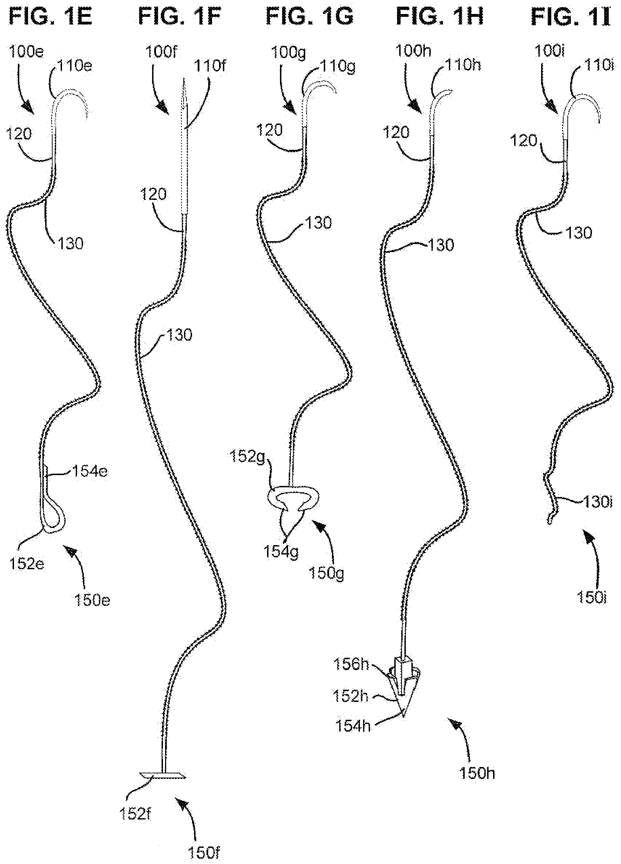

FIG. 1E shows an alternative configuration of a self-retaining suture 100e. Self-retaining suture 100e is an example of a unidirectional self-retaining suture. Self-retaining suture 100e includes a curved needle 110e attached to the proximal end of a suture thread 120. Self-retaining suture 100e includes a plurality of retainers 130 distributed on the surface of a suture thread 120. The retainers 130 are oriented such that the suture thread can be moved through tissue in the direction of needle 110e but the retainers resist movement in the opposite direction. An anchor 150e is formed at the distal end of self-retaining suture 100e. Anchor 150e can take the form of a loop, bar, hook, tack, staple, pledget or other structural feature which allows the end of the suture thread 120 to be fixed to tissue and/or prevents the end of the suture thread 120 from being drawn through tissue. As shown in FIG. 1E, the anchor 150e can be formed by manipulation of the suture thread 120. Anchor 150e is configured as a loop 152e made by folding suture thread 120 back on itself and securing the end 154e of suture thread 120 to itself by, for example, welding, fusing, and/or adhesive.

FIG. 1F shows an alternative configuration of a self-retaining suture 100f. Self-retaining suture 100f is an example of a unidirectional self-retaining suture. Self-retaining suture 100f includes a straight needle 110f attached to the proximal end of a suture thread 120. Self-retaining suture 100f includes a plurality of retainers 130 distributed on the surface of a suture thread 120. The retainers 130 are oriented such that the suture thread can be moved through tissue in the direction of needle 110f but the retainers resist movement in the opposite direction. An anchor 150f is formed at the distal end of self-retaining suture 100f. As shown in FIG. 1F, the anchor 150f comprises a bar 152f connected substantially perpendicular to suture thread 120. Bar is sufficiently large and stiff to prevent the distal end of the suture thread 120 from being drawn through tissue in the direction of needle 110f. Bar 152f can be formed by manipulation of the suture thread 120 or by attaching a separately formed component by, for example, welding, fusing, and/or adhesive.

FIG. 1G shows an alternative configuration of a self-retaining suture 100g. Self-retaining suture 100g is an example of a unidirectional self-retaining suture. Self-retaining suture 100g includes a curved needle 110g attached to the proximal end of a suture thread 120. Self-retaining suture 100g includes a plurality of retainers 130 distributed on the surface of a suture thread 120. The retainers 130 are oriented such that the suture thread can be moved through tissue in the direction of needle 110g but the retainers resist movement in the opposite direction. An anchor 150g is formed at the distal end of self-retaining suture 100g. As shown in FIG. 1G, the anchor 150g comprises a clip/staple 152g connected to suture thread 120. Clip/staple 152g comprises two arms 154g which can be used to engage tissue, for example by penetrating tissue with arms 154g and approximating arms 154g. Clip/staple 152g, by engaging tissue, prevents the distal end of the suture thread 120 from being drawn through tissue in the direction of needle 110g. Clip/staple 152g can be formed by manipulation of the suture thread 120 or by attaching a separately formed component by, for example, welding, fusing, and/or adhesive.

FIG. 1H shows an alternative configuration of a self-retaining suture 100h. Self-retaining suture 100h is an example of a single-armed self-retaining suture. Self-retaining suture 100h includes a curved needle 110h attached to the proximal end of a suture thread 120. Self-retaining suture 100h includes a plurality of retainers 130 distributed on the surface of a suture thread 120. The retainers 130 are oriented such that the suture thread can be moved through tissue in the direction of needle 110h but the retainers resist movement in the opposite direction. An anchor 150h is formed at the distal end of self-retaining suture 100h. As shown in FIG. 1H, the anchor 150h comprises a tack 152h connected to suture thread 120. Tack 152h comprises a pointed end 154h which allows tack to be pushed in to a tissue. Tack 152h includes a plurality of projections 156h which resist removal of tack 152h from the tissue. Tack 152h, by engaging tissue, prevents the distal end of the suture thread 120 from being drawn through tissue in the direction of needle 110h. Tack 152h can be formed by manipulation of the suture thread 120 or by attaching a separately formed component by, for example, welding, fusing, and/or adhesive.

FIG. II shows an alternative configuration of a self-retaining suture 100i. Self-retaining suture 100g is an example of a bidirectional self-retaining suture however, only one end of the suture is provided with the needle. Self-retaining suture 100h includes a curved needle 110i attached to the proximal end of a suture thread 120. Self-retaining suture 100i includes a plurality of retainers 130 distributed on the surface of a suture thread 120. The retainers 130 are oriented such that the suture thread can be moved through tissue in the direction of needle 110g but the retainers resist movement in the opposite direction. An anchor 150g is formed at the distal end of self-retaining suture 100i. As shown in FIG. 1I, the anchor 150i comprises a short length of suture thread 120 having retainers 130i formed thereon oriented to resist movement of the distal end of suture thread 120 through tissue in the direction of needle 110i. Anchor 150i is drawn into the tissue in the direction of needle 110i until sufficient retainers engage tissue to prevent the distal end of the suture thread 120 from being drawn further through tissue in the direction of needle 110i. Retainers 130i can be formed on suture thread 120 in the same process/apparatus as retainers 130.

As shown in FIGS. 1A-1I and described in the accompanying text, the present invention provides a self-retaining sutures of a variety of configurations. Self-retaining sutures of the present invention may be made from suture threads that are small in diameter (for example 2-0, 4-0, 5-0, 6-0, 7-0, 9-0, 10-0, 11-0, 12-0 and smaller sutures). The sutures may be 5-0 size and smaller and for vascular applications are preferably 6-0, 7-0 and smaller. The small size of the suture threads requires special apparatus to allow repeatable creation of retainers. Moreover, where the retainers are individually small, it is desirable to have a high density of retainers in order to enhance the tissue holding force of the self-retaining suture. Thus, it is desirable to reduce the distance (pitch) between adjacent retainers. In preferred embodiments, the pitch P is less than 2 times the retainer length L, in more preferred embodiments, the pitch P is less than 1.5 times the retainer length L. And, in more preferred embodiments, the pitch P is less than 1.2 times the retainer length L. Furthermore, in preferred embodiments the retainers are distributed in a double helix or quadra-helix pattern in which two or four retainers are created within the pitch P. Furthermore, it is also desirable to have longer retainers. In preferred embodiments, utilizing the apparatus of the present invention it is possible to achieve a combination of retainer length L and retainer density such that the combined length of the retainers in a region of the suture bearing retainers is greater than the length of the region. For example it is possible for 25 mm (an inch) of suture thread to bear retainers with a combined length of more than 25 mm (1 inch) more than 38 mm (1.5 inches) and/or more than 50 mm (two inches). Repeatably forming small retainers in such close proximity to other small retainers is made possible by aspects of the novel apparatus described below.

### Apparatus For Manufacturing Self-Retaining Sutures

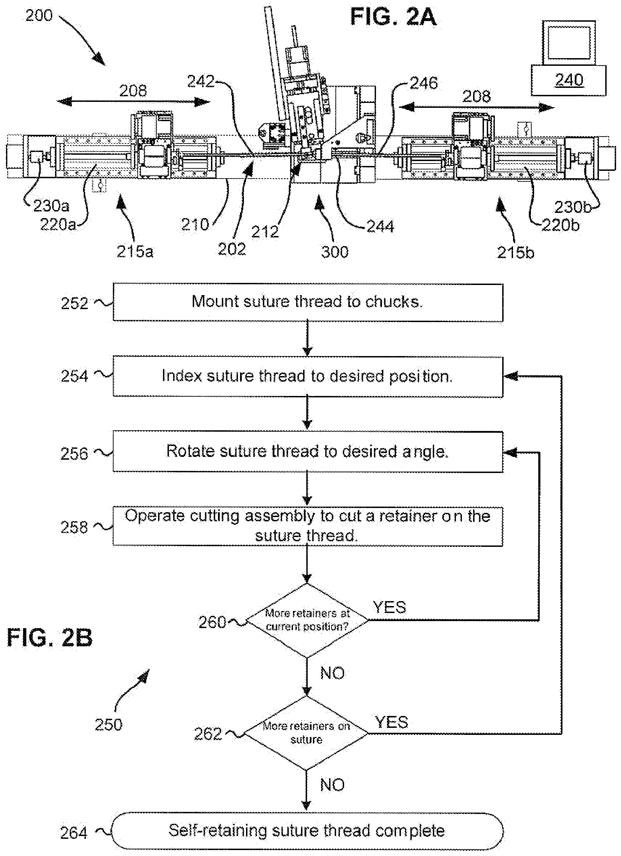

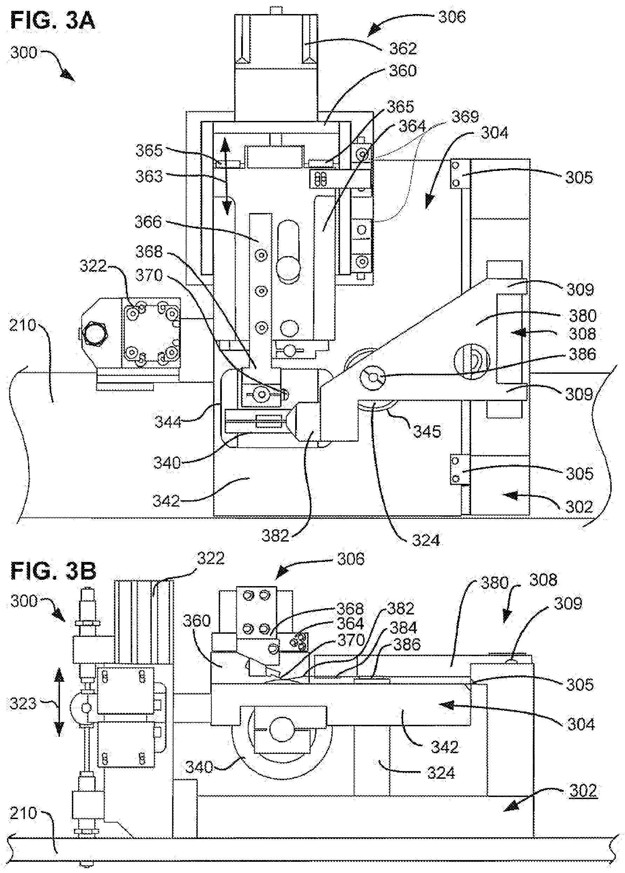

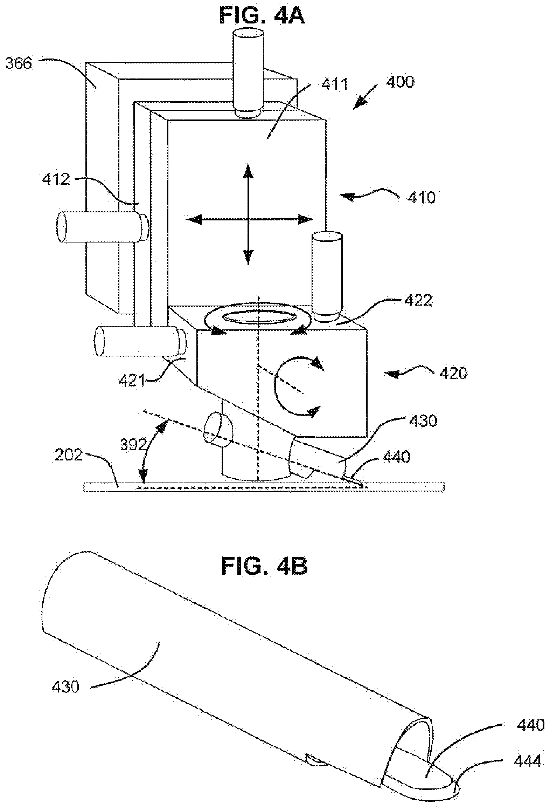

FIG. 2A, shows a schematic diagram of a retainer-cutting machine 200 for making self-retaining sutures. As shown in FIG. 2A, retainer-cutting machine 200 is configured to form retainers on suture thread 202 to create a self-retaining suture. Retainer-cutting machine 200 comprises a table 210 to which the components are mounted. A cutting assembly 300 (See FIGS. 3A-3C) is preferably mounted in a fixed position in the middle of table 210. The cutting assembly 300 includes a retainer-forming head 212 operative to cut retainers upon a suture thread 202. At each end of table 210 is a track 220a, 220b. A chuck assembly 215a, 215b (See FIGS. 4A-4C) is mounted on each track 220a, 220b. Chuck assemblies 215a, 215b clamp the ends of suture thread 202 and hold the suture thread 202 in alignment with the cutting assembly 300. The chuck assemblies 215a, 215b also operate to rotate the suture thread 202 relative to the cutting assembly 300 (around the long axis of the suture). An actuator 230a, 230b, (for example, stepper motors) is associated with each track 220a, 220b for moving each chuck assembly 215a, 215b along the table 210 as shown by arrows 208. The actuators 230a, 230b operate to translate the suture thread 202 relative to the cutting assembly 300.

Cutting assembly 300, chuck assemblies 215a, 215b, and actuators 230a, 230b are under the control of a computer system 240. Computer system 240 coordinates the operation of the cutting assembly 300, chuck assemblies 215a, 215b, and actuators 230a, 230b so that the cutting assembly 300 cuts retainers at the desired locations on the suture thread 202. After suture thread 202 is mounted to chuck assemblies 215a, 215b, the chuck assemblies 215a, 215b translate and rotate suture thread 202 stepwise relative to retainer-forming head 212 under control of computer system 240. At selected positions of suture thread 202, computer system 240, activates retainer-forming head 212 of cutting assembly 300 to form a retainer on suture thread 202. The process is repeated stepwise until, for example, suture thread 202 is a bidirectional self-retaining suture having a first region 242 having a plurality of retainers oriented in a first direction; a second region 246 having a plurality of retainers oriented in a second direction; and a transition region 244 having no retainers and positioned between the first region 242 and the second region 246.

FIG. 2B, shows a flow chart of an example of a process 250 for creating a self-retaining suture with retainers at selected locations on the suture thread. In step 252, the suture thread is mounted in the retainer-cutting machine and mounted to the chucks.

In step 254, the chuck assemblies 215a, 215b index the suture thread 202 to a desired position relative to the cutting assembly 300 by translating the suture thread 202 relative to the table 210 and cutting assembly.

In step 256, the chuck assemblies 215a, 215b rotate the suture thread 202 to a desired angle relative to its starting position.

In step 258, the cutting assembly 300 cuts a retainer on the suture thread.

In step 260, if further retainers are to be cut at this position along the suture thread 202, the process returns to step 256 in order to rotate the suture thread to a new angle. For example, the suture thread can be rotated once by 180 degrees if two retainers are to be formed at the same position along the length of the suture thread. Alternatively, the suture thread can be rotated three times by 90 degrees each if four retainers are to be formed at the same position along the length of the suture thread. If no further retainers are to be cut at this position along the suture thread 202, the process continues to step 262.

In step 262, if no more retainers need to be cut on the suture thread, the self-retaining suture is complete (step 264). However, further if retainers are to be cut at a different position on the suture thread, the process returns to step 254 for actuators 230a, 230b to move chuck assemblies 215a, 215b to index the suture thread 202 to a new position relative to the cutting assembly 300. The actuators 230a, 230b move chuck assemblies 215a, 215b along tracks 220a, 220b to translate the suture thread 202 along its longitudinal axis. For example, suture thread 202 is translated by a distance equal to the desired axial distance between retainers which is, in some distribution patterns, equal to the pitch (See FIG. 1C).

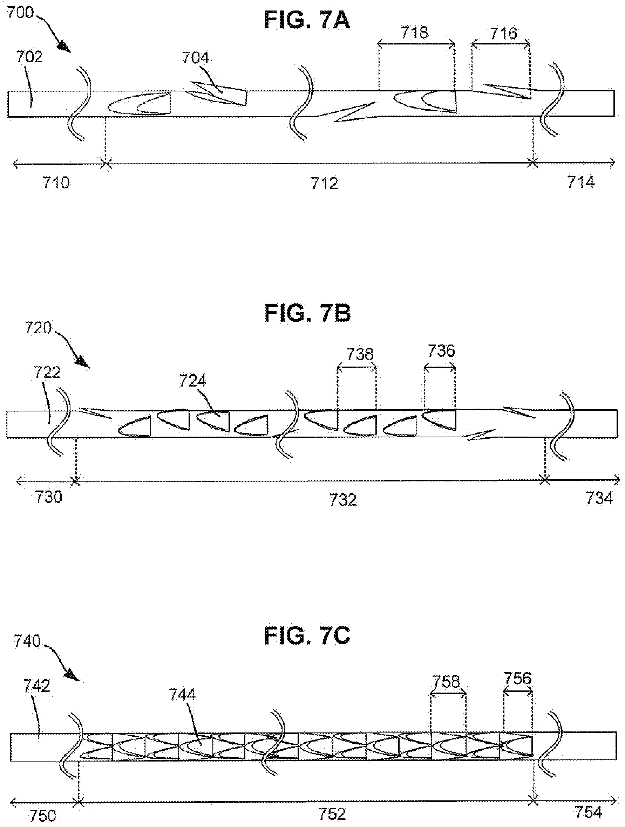

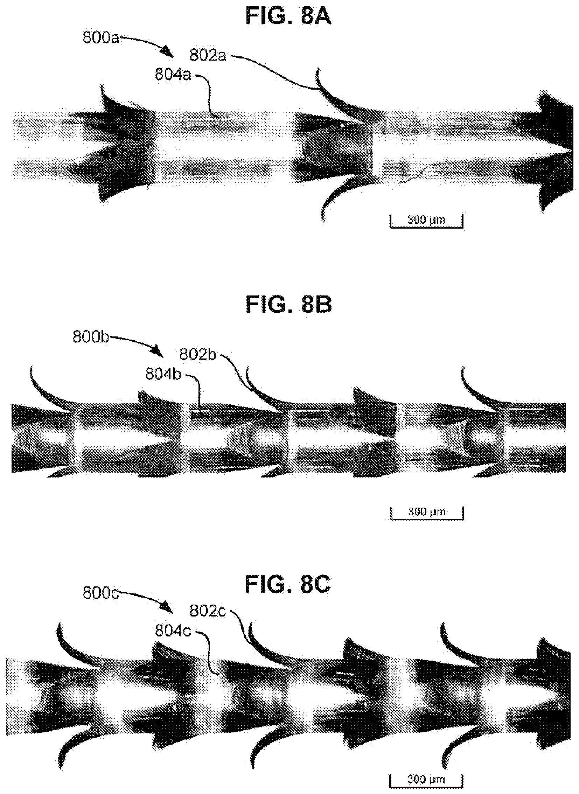

Returning to step 256, the chuck assemblies 215a, 215b rotate the suture thread about its longitudinal axis to create the desired spirality angle between the first retainer (set of retainers) and the second retainer (set of retainers). For example, the spirality angle can be 45 degrees in a quadra-helix pattern as shown, for example in FIG. 7C and 8A. Another retainer is then cut at step 258 and the method proceeds until all of the desired retainers have been cut. The suture thread can then be removed from the chucks, trimmed to the desired length, attached to the selected needles and/or anchors, packaged and sterilized.

### Cutting Assembly

FIGS. 3A-3H show views of cutting assembly 300. FIG. 3A shows a top view of cutting assembly 300. FIG. 3B shows a side view of cutting assembly 300. As shown in FIGS. 3A and 3B, cutting assembly 300 includes four subassemblies: base assembly 302 which is secured to table 210; anvil assembly 304 which is secured to base assembly by a hinge 305; blade assembly 306 which is adjustably mounted to the anvil assembly 304; and spring assembly 308 which is secured to base assembly 302 by a hinge 309.

Base assembly 302 comprises a pneumatic actuator 322 for raising and lowering anvil assembly 304 relative to base assembly 302. (See arrow 323 of FIG. 3B). Base assembly 302 also comprises a column 324 which passes through an aperture in anvil assembly 304.

Anvil assembly 304 includes an anvil 340 adapted to support a suture thread during cutting of retainers (as further described below). Anvil 340 is mounted beneath anvil plate 342. Anvil plate 342 has an anvil aperture 344 through which a portion of anvil 340 protrudes. Anvil plate 342 also has a column aperture 345 (see FIG. 3A) through which column 324 protrudes.

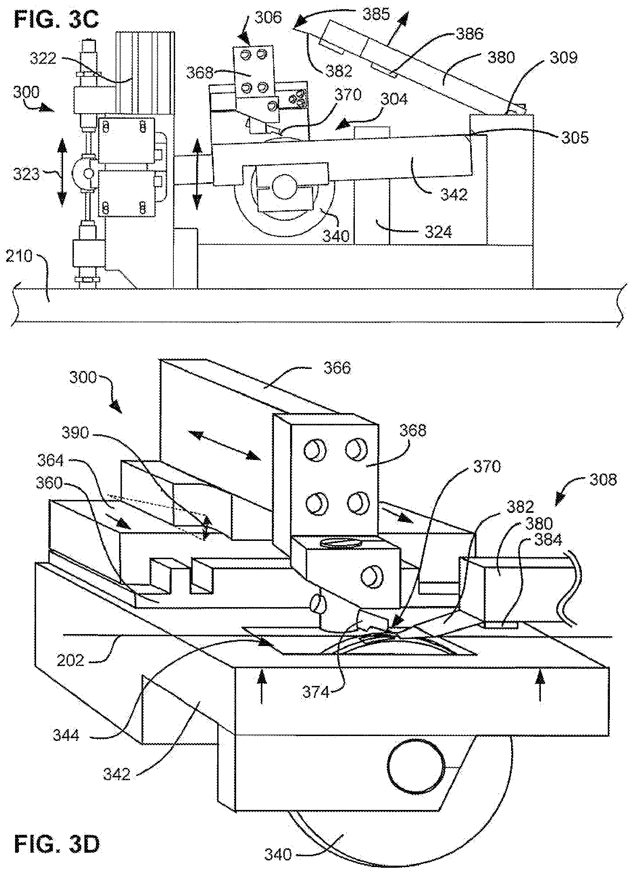

Blade assembly 306 comprises a blade frame 360. A blade slide 364 is mounted to blade frame 360 such that blade slide 364 can slide relative to blade frame 360. Adjustable stops 369 on blade frame 360 are used to control the range of movement of blade slide 364 relative to blade frame 360. A blade actuator 362 is mounted to blade frame 360. The blade actuator can include, for example, an electric motor. Operation of blade actuator 362 cause blade slide 364 to slide relative to blade frame 360. (See arrow 363 of FIG. 3A). A blade arm 366 is mounted at one end by hinges 365 to blade slide 364. A pair of hinges 365 secure blade arm 366 to blade slide 364 in a manner which allows for adjustment of the angle between blade arm 366 and blade slide 364 (see also FIG 3D). At the other end of blade arm 366 is cutting head 368. Blade 370 is mounted to cutting head 368. Blade frame 360 is adjustably secured by clamps to anvil plate 342 of anvil assembly 304 such that the angle between the movement axis of blade slide 364 (and hence blade 370) can be adjusted relative to anvil 340. The position of blade slide 364 relative to anvil 340 can also be adjusted. However, in a preferred embodiment, the movement axis of blade slide 364 (and hence blade 370) is maintained parallel to the surface of anvil 340.

Spring assembly 308, comprises spring arm 380 which is connected at one end by hinge 309 to base assembly 302. At the other end of spring arm 380 is spring 382 which is secured in place by spring mount 384. A spring-arm adjuster 386 is mounted through spring arm 380 and at its lowest point contacts column 324 of base assembly 302. Spring-arm adjuster 386 can be used to adjust the height of spring mount 384 above anvil 340.

FIG. 3C shows a side view of cutting assembly 300 with anvil assembly 304 lowered by operation of actuator 322. Anvil assembly 304 is lowered during translation or rotation of the suture thread. Lowering of anvil assembly 304 releases the suture thread from entrapment between the anvil 340 and the spring 382. Note that the blade assembly 306 is also lowered at the same time because the blade assembly 306 is mounted on the anvil assembly 304. To make sure the suture is not damaged the cutting head 368 and blade 370 must be moved clear of the suture thread prior to lowering the anvil assembly 304 (and cutting assembly 300).

FIG. 3C also shows the movement of spring assembly 308. Spring arm 380 can rotate around hinge 309 to lift spring 382 away from the suture thread. It is not, however, necessary to move spring arm 380 for each translation and rotation of the suture thread. Spring arm 380 is typically raised as shown in FIG. 3C in order to allow mounting of a new suture thread within the cutting assembly mounted on the anvil assembly.

FIG. 3D shows a partial perspective view of cutting assembly 300 showing the relationship between the anvil 340, blade 370 and spring 382. As shown in FIG. 3D, anvil 340 is mounted to anvil plate 342, below anvil plate 342. However, anvil 340 protrudes through anvil aperture 344 in anvil plate 342. The suture thread 202 passes between spring 382 and anvil 340. When the suture thread 202 is correctly positioned for cutting a retainer, anvil 340 is lifted relative to spring 382 and suture thread 202. The movement of anvil 340 traps suture thread 202 between anvil 340 and spring 382. Anvil 340 and spring 382 thereby secure suture thread 202 for cutting. Arrow 390 shows the effect of varying the angle between blade arm 364 and blade slide 364 about hinges 365 (not shown but see FIG. 3B). This angle can be adjusted, if necessary, to ensure that the blade 370 moves parallel to the surface of anvil 340.

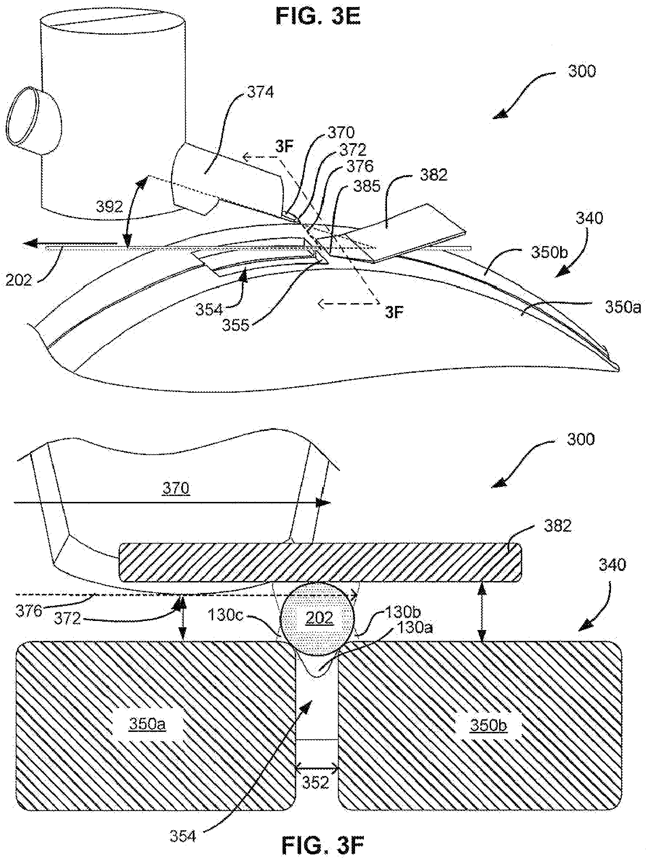

FIGS. 3E and 3F show an enlarged view of the cutting region. As shown in FIG. 3E, the suture thread 202 passes between spring 382 and anvil 340. When the suture thread 202 is correctly positioned for cutting a retainer, anvil 340 is lifted relative to spring 382 and suture thread 202 is trapped between anvil 340 and spring 382 as shown. Anvil 340 comprises two anvil segments 350a, 350b separated by an adjustable anvil gap 352 which is less than one suture diameter (<1 SD) in size. In embodiments anvil gap 352 (see FIG. 3F) is between 0.4 and 0.6 suture diameters in size (0.4-0.6 SD). In a preferred embodiment anvil gap 352 is approximately 0.5 suture diameters in size (0.5 SD). Anvil gap 352, in some embodiments, is a fixed gap selected based on the suture diameter. Anvil gap 352 is, in alternative embodiments, adjustable in increments of the order of a micrometer (µm). The purpose of gap 352 between anvil segments 350a, 350b is to help secure suture thread 202 during cutting of a retainer and also to provide space for a retainer on the opposite side of the suture thread 202 from the retainer being cut.

Anvil gap 352 is one of the features of retainer cutting machine 200 which enable the creation of high density self-retaining sutures. A previously cut retainer can be positioned within gap 352 and thereby avoid interference with anvil 340 during cutting of a new retainer even though the previously cut retainer is not clear of anvil 340 (by, for example being positioned over relief 354). Thus, the presence of anvil gap 352 allows, in some embodiments, for the creation of two or four retainers at which substantially the same axial position along the suture. The presence of anvil gap 352 also allows, in some embodiments, for the creation of two or four retainers which are axially displaced from each other by distances less than the length of a retainer. To put it another way, the presence of relief 354 allows for creation of retainers in double-helix and quadra-helix distribution patterns where the helices are in-phase or out of phase by less than a retainer length (see examples below).

As shown in FIG. 3E, anvil 340 also comprises a relief 354. Relief 354 comprises a step 355 adjacent trailing edge 385 of spring 382. The distance between step 355 and trailing edge 385 is in some embodiments adjustable by rotation of anvil 340. In alternative embodiments, anvil 340 and/or relief 354 are machined with relief 354 in a fixed position. In a preferred embodiment, step 355 is positioned within one retainer length (1 L) of the path of the blade tip 372. Thus, step 355 is, in certain embodiments, positioned within one to three suture diameters (≤ 3 SD) of the path of the blade tip 372. The trailing edge 385 of the spring 382 is within approximately 1 suture diameter (1 SD) of the path of the blade tip 372. Thus, step 355 is, in certain embodiments, positioned within two to four suture diameters (≤ 4 SD) of the trailing edge 385 of spring 382.

Relief 354 is one of the features of retainer cutting machine 200 which enable the creation of high density self-retaining sutures. The previously cut retainers are positioned over the relief 354 and thereby avoid interference with anvil 340 during cutting of new retainers. The presence of relief 354 thus allows the cutting of retainers in very close proximity to retainers that have previously been cut. To put it another way, the presence of relief 354 allows for a small retainer pitch - the distance measured axially along the suture between one retainer of a pattern and the adjacent retainer in the pattern measured between identical points of the retainer (i.e. tip to tip or base to base). For example, the position of relief 354 can be adjusted to enable creation of retainers at a retainer pitch which is less than two retainer lengths, less than 1.5 retainer lengths, less than 1.2 retainer lengths and, in some cases, approximately equal to the retainer length.

Referring again to FIG. 3E, in a preferred embodiment, blade 370 (mounted in blade holder 374) cuts retainers in suture thread 202 in the region between step 355 and the trailing edge 385 of spring 382. The suture thread 202 is secured between anvil 340 and the trailing edge 385 of spring 382 on one side of this region while the suture thread passes over step 355 on the other side of this region. Tip 372 of blade 370 passes through suture thread 202 along a path 376 between step 355 and the spring 382. The proximity of the step 355 to the trailing edge 385 of the spring 382 allows for creation of retainers at high-density by providing support of the suture adjacent the cutting region while preventing interference between already-formed retainers and the anvil 340. The distance between the step 355 and the trailing edge 385 of the spring 382 can be adjusted by, for example rotating anvil 340. Note that in preferred embodiments, the distance between step 355 and the trailing edge 385 of spring 382 is less than or equal to the pitch of the retainers. The pitch of the retainers is, in some embodiments less than or equal to 60µm.

FIG. 3E also illustrates the cutting blade angle parameter 392. The cutting blade angle is the angle between the plane of blade 370 and the axis of suture 202. As shown in FIG. 3E, the cutting blade angle 392 can be adjusted. In embodiments, the cutting blade angle is fixed by the construction of the blade holder and can be adjusted by selecting a blade holder establishing the desired cutting blade angle 392. In alternative embodiments the cutting blade angle is adjustable utilizing a rotary motion stage built into the cutting blade holder (see, e.g. rotary stage 420 of FIG 4A). In general, the cutting blade angle affects the angle of the cut of the retainers. Thus, a smaller cutting blade angle results in a longer retainer for a given cut depth (all other factors being equal). In other words, a smaller cutting blade angle results in a retainer with a higher aspect ratio and a larger cutting blade angle results in a retainer with a smaller aspect ratio (all other factors being equal). However, the aspect ratio is also affected by the plough angle and cutting stage angle as described below.

FIG. 3F shows a partial sectional view through cutting assembly 300 along the line 3F-3F of FIG. 3E. As shown in FIG. 3F, suture thread 202 is trapped between spring 382 and segments 350a and 350b of anvil 340 and immediately adjacent blade 370. The spring 382 is less than one suture diameter (<1 SD) above the surface of anvil 340. As shown in FIG. 3F, the blade tip 372 passes through suture thread 202 along a path 376 parallel to the surface of anvil 340 and at a fixed distance above the surface of the anvil 340. The distance between blade tip 372 and anvil 340 is adjustable. The distance between blade tip 372 and anvil 340 is selected based on the depth of cut desired for the retainer. The distance between blade tip 372 and anvil 340 is less than one suture diameter (<1 SD). In embodiments, the distance between blade tip 372 and anvil 340 is between 0.6 and 0.9 suture diameters. In preferred embodiments the distance between blade tip 372 and anvil 340 is between 0.7 and 0.8 suture diameters. For example, in one embodiment, the suture diameter is 50µm. Moreover, in some embodiments, the suture diameter is less than or equal to 50µm.

As best shown in FIG. 3F, the purpose of relief 354 is to prevent interference between the anvil and retainers adjacent the retainers being cut. Depending on the spirality angle, these retainers may be positioned such that they would be crushed by contact with anvil segments 350a, 350b. For example, FIG. 3F, shows the cutting of a quadra-helix pattern with a 45 degree spirality angle. As shown In FIG. 3F, one retainer 130a has already been cut at the current position of the suture. Retainer 130a is positioned between anvil segments 350a, 350b for cutting of a retainer opposite retainer 130a. In the background are retainers cut at the immediately adjacent position to retainer 130a. Note that because suture thread 202 has been rotated by 45 degrees between the positions, retainers 130b and 130c would be in contact with anvil segments 350a, 350b. However, the suture thread 202 has been translated such that retainers 130b, 130c have passed beyond step 355 of relief 354 (see FIG. 3D). Thus retainers 130b, 130c are positioned above relief 354 (see FIG. 3E) and are not in contact with anvil segments 350a, 350b.

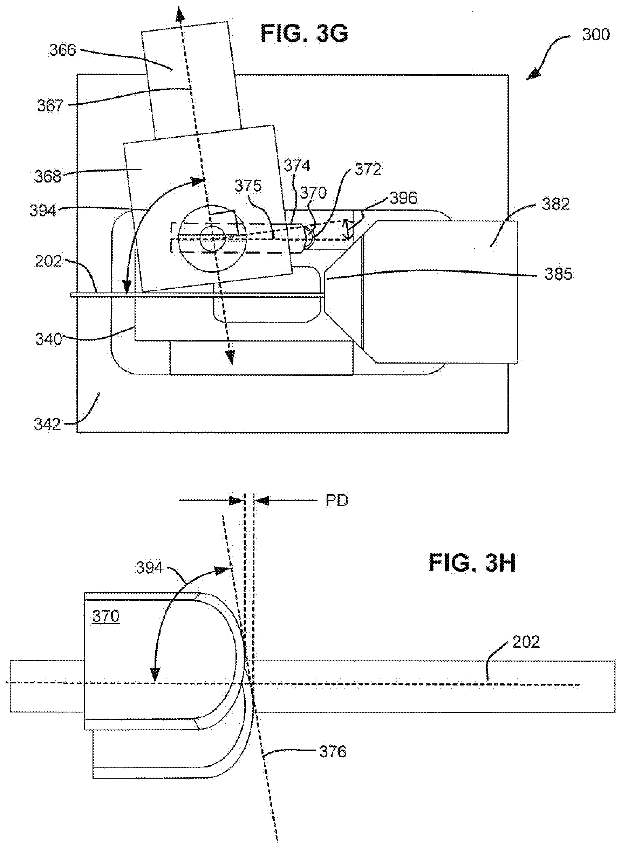

FIG. 3G shows an enlarged top down view of the cutting region of cutting assembly 300. As shown in FIG. 3G, the movement axis 367 of blade slide 364 is adjustable and is not required to be perpendicular to the axis of suture thread 202. In a preferred embodiment, the movement axis 367 of slide 364 is at an acute angle with the axis of suture thread 202. The acute angle between the movement access 367 of slide 364 and the axis of suture thread 202 is termed the cutting stage angle 394.

Referring again to FIG. 3G, The orientation of blade holder 374 with respect to the movement axis 367 of slide 364 is also adjustable. The blade holder 374 has a longitudinal axis 375. The orientation of blade holder 374 is used to adjust the orientation of the axis 395 of blade tip 372. In a preferred embodiment, with the movement axis 367 of slide 364 oriented at cutting stage angle 394 from perpendicular with the axis of suture thread 202, the longitudinal axis 375 of blade holder 374 is oriented at an acute angle termed the plough angle 396 from perpendicular to the movement axis 367 of slide 364. In general it is desirable that the sum of the plough angle 396 and cutting stage angle 394 is equal to 90°. Where the sum of the cutting stage angle 394 plus the plough angle 396 equals 90°, the longitudinal axis 395 of blade holder 374 is kept substantially parallel to the axis of the suture thread 202. In alternative embodiments it can be desirable for the longitudinal axis 395 of blade holder 374 to be other than parallel to the axis of the suture 202 - in such case the sum of the plough angle 396 and cutting stage angle 394 can be selected to be less than or greater than 90°.

The cutting stage angle 394 affects the shape and elevation of the retainers cut by blade 370. The cutting stage angle 394 determines the movement path 376 of the blade 370 as it passes through suture thread 202. As shown in FIG. 3H, when the blade 370 moves across the suture thread 202, the blade advances by a distance PD dependent upon cutting stage angle 394. In general, the smaller is cutting stage angle 394, the greater the amount of advance PD. The amount of advance PD generally increases the length of the retainers formed (for a given cut depth) and also increases the elevation of the retainers formed. In other words, the smaller is the cutting stage angle 394, the greater is the advance PD and the larger is the aspect ratio of retainers formed (all other factors being equal). Conversely, the closer the cutting stage angle 394 is to 90°, the smaller is the advance PD and the smaller is the aspect ratio of retainers formed (all other factors being equal). The advance PD of blade 370 during cutting in combination with the angle and shape of blade 370 can thus be used to control the shape and elevation of retainers formed on suture thread 202. Changes in cutting stage angle 394 (and retainer length) were found to have significant effects on tissue holding force in sutures made on the present retainer-cutting machine.

### Cutting Head and Cutting Blades

FIG. 4A shows one embodiment of a cutting head 400 for use in embodiments of the present invention. Cutting head 400 is mounted on the end of the blade arm 366 (See e.g. cutting head 368 of FIG. 3A). A blade 440 is attached to cutting head 400. Cutting head 400 allows for adjustment of the position and orientation of blade 440 relative to blade arm 366. According to one embodiment cutting head 400 includes a two degree of freedom linear (DOF) stage 410 and a two DOF rotary stage 420 and a blade mount 430. In alternative embodiments, a cutting head need not be adjustable, but is instead manufactured to hold a blade at the desired position and orientation. A different head is created and selected based upon the desired cutting parameters for a given self-retaining suture.

Two DOF linear stage 410 allows for adjustment of the position of the blade 440 relative to the anvil 340 and thus the suture thread 202. A first, linear stage 411 allows for adjustment of the height of the blade above the anvil. The height of the blade above the anvil and thus can be used to control the depth of cut. A second, the linear stage 412 allows for adjustment of the position of the blade along the axis of the suture thread. The second, linear stage 412 allows for adjustment of the relative positions of the path of the blade and the spring (see FIG. 3E). The other remaining linear degree of freedom of blade 440 is the movement axis across suture thread 202. Movement along this axis is controlled by actuator 362 moving blade slide 364 relative to blade frame 360 within the range of motion constrained by adjustable stops 369 (See FIG. 3A).

Two DOF rotary stage 420 can be used to allow for adjustment of the orientation of the blade 440 relative to the movement axis of blade arm 366 and the suture thread 202. A first rotary stage 421 allows for adjustment of the angle of the blade relative to the suture thread 202 in the plane of the anvil 340 not shown. The first rotary stage 421 adjusts the angle of the cut into the suture thread 202. This rotary stage can therefore be used to adjust the cutting blade angle.

The second rotary stage 422 allows for adjustment of the orientation of the blade relative to the movement axis of the blade arm 366. That is, the second rotary stage 422 allows for adjustment of the blade orientation relative to the direction of cutting. This second rotary stage 422 allows the blade orientation to compensate for any cutting stage angle 394 (not shown but see FIG. 3G) applied to the slide orientation. The secondary rotary stage thus allows adjustment of the cutting stage angle 394 (not shown but see FIG. 3G). The final rotary degree of freedom of blade 440 is the rotation of blade 440 about its axis. Blade 440 should be maintained parallel to the plane of anvil 340. This can be accomplished by adjusting the attachment of blade mount 430 to rotary stage 420. Rotary stages are readily available with resolutions of 0.002 degrees or less.

The linear or rotary stages of the cutting head are in some embodiments manually controlled. For example, where the same shape retainers will be formed at all positions along a suture thread it is only necessary to set these parameters before commencement of the retainer cutting. Thereafter, so long as there is no drift, the parameters need not be changed. In alternative embodiments, the linear and rotary stages of the cutting head are controlled by actuators such as piezo-electric actuators, servomotors ultrasonic motors and the like. Computer control of the linear and rotary stages allows for adjustment of the retainer cutting parameters at different positions along a suture thread. Thus, for example, the cut depth can be greater at some positions along a suture thread than at other positions. Also, computer control of the linear and rotary stages can also allow the computer system to adjust the position and/or orientation of the blade in response to drift in the parameters over time, caused for example by temperature changes of wear in the blade. Piezo-electric linear stages are readily available with uni-directional repeatability of 0.05µm and encoder resolutions of 5nm.

In a preferred embodiment, the blade 440 is a sapphire blade. Sapphire has a hardness of 9.0 Mohs. Sapphire blades are ceramic blades typically having an edge radius one or two magnitude lower than an edge radius of a steel blade thus allowing the accurate cutting of retainers on suture threads of size USP 2-0, 4-0, 6-0, 8-0, 9-0, 10-0, 11-0, 12-0 and smaller. Further, sapphire blades generally maintain their mechanical characteristics over the temperature ranges desirable for cutting polymer and co-polymer materials. Maintaining mechanical characteristics (i.e., geometry of a cut produced) can be desired where the retainers are extremely small and therefore sensitive to small changes. Further, sapphire blades are more abrasion resistant than, for example, typical steel blades, providing more repeatable results over long term use. Further, sapphire blades can be sharpened more effectively than steel blades. In alternative embodiments, the blade 440 may be metal, mineral or ceramic blades which are hard coated, mineral coated, ceramic coated and/or carbon coated blades. For example, synthetic diamond/black diamond blades are commercially available for use in ophthalmic applications. For example, the blades may have: carbon coating, diamond coating, diamond-like coating, nano-ceramic coating, ceramic coating, sapphire coating and/or yttriated zirconia coating or a ceramic material having the desired sharpness and durability or other hardened blades or hard coated blades.

In some embodiments, the blade 440 is temperature controlled to optimize formation and elevation of retainers. To control the temperature of the blade, the blade is or otherwise placed in conductive communication with a temperature-controlled copper plate. The copper plate can effectively heat or cool the blade to the desired temperature through conduction. The copper plate is temperature controlled utilizing a solid state or liquid heat transport system and a closed-loop temperature controller. The temperature of the blade can thus be tightly controlled to a desired temperature range to provide satisfactory retainer formation.

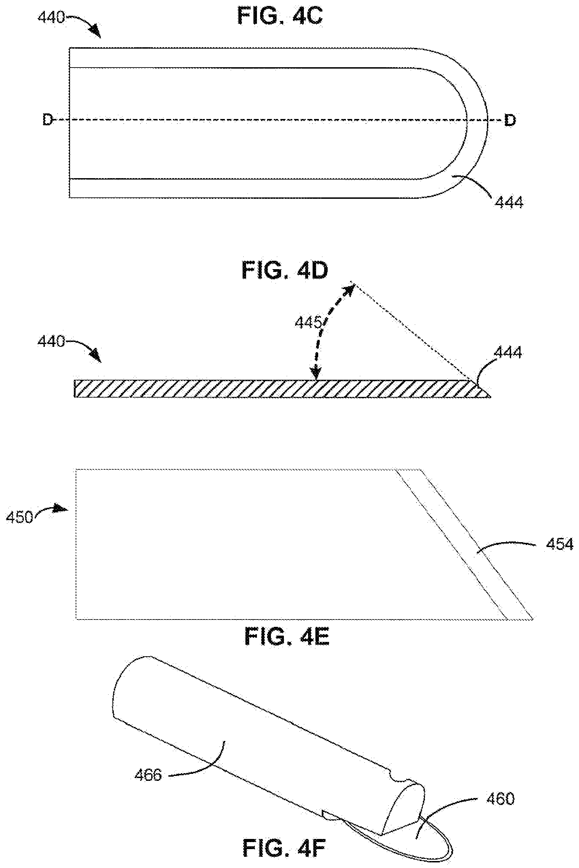

FIGS. 4B, 4C and 4D show enlarged views of sapphire blade 440. FIG. 4A shows a perspective view of a curved sapphire blade 440 secured to a blade mount 430. Blade mount 430 is generally tubular. Sapphire blade 440 is secured inside blade mount 430. Blade mount 430 is secured to blade 440 of cutting head 400 in a manner that allows removal and replacement and angular adjustments of the blade 440. FIG. 4C shows an enlarged plain view of sapphire blade 440. As shown in FIG. 4C, cutting edge 444 is crescent-shaped. In a preferred embodiment, cutting edge 444 is 2.8 mm wide and 7.6 mm long and the cutting edge has a 1.4mm radius of curvature. FIG. 4D shows a sectional view of blade 440 along the line D-D of FIG. 4C. In the preferred embodiment, the thickness of blade 440 is 0.3 mm and the angle 445 of cutting edge 444 is 40 degrees. Suitable blades are commercially available for ophthalmic applications.

In alternative embodiments, a sapphire or diamond blade can be straight, angled or curved and may have be hemispherical, parabolic, or any other shape of cutting edge suitable for cutting the retainer. FIG. 4E shows an alternative sapphire blade 450 having a straight cutting edge 454. FIG. 4F shows an alternative circular blade 460 mounted to a block 466. Circular blade 460 can be made, for example from sapphire, synthetic diamond and/or steel. Block 466 mounts to cutting head 400 in a manner which allows removal and replacement of the blade 460. Block 466 preferably mounts to cutting head 400 in a manner which allows angular adjustment of blade 460. The retainers of self-retaining suture may also be cut with cutting wheels, grinding wheels and/or microcutting tools. Such cutting devices can be substituted for the blade of the present embodiment. That is to say that such cutting devices can be incorporated in place of blade 370 in the embodiment shown in the various figures hereof.

Additionally, the blade or other cutting device can be mounted to an ultrasound generator\vibration generator in order to facilitate cutting. In some embodiments, for example, vibrational energy at a frequency within the ranges, 1 to 100 kHz, 10 to 90 kHz, and 15 to 50kHz is applied by a horn to a converter configured to support the blade or other cutting tool.

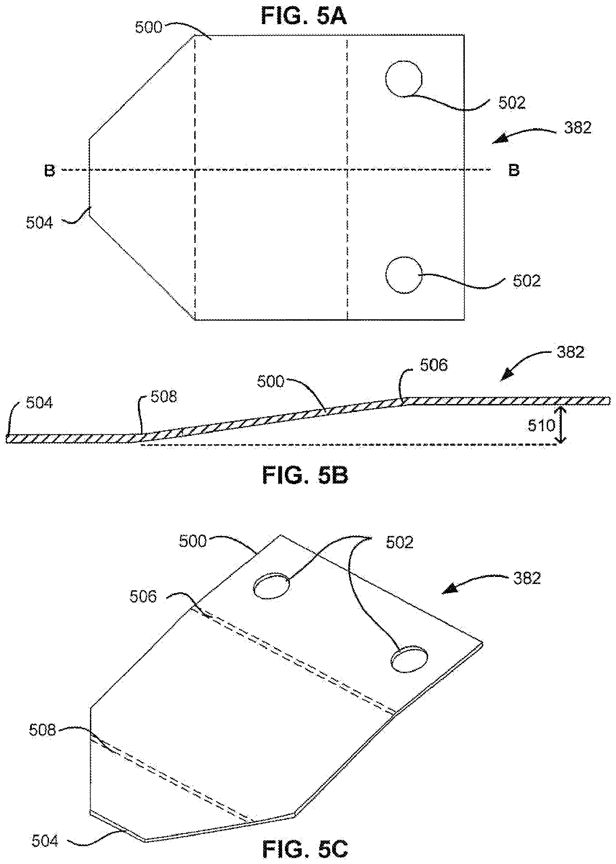

FIGS. 5A-5C show details of the spring 382. As shown in FIGS. 3E and 3F, spring 382 is used to hold suture thread 202 to anvil 340 during cutting of a retainer. As shown in FIGS. 3A, 3B, spring 382 is mounted to spring arm 380 by a spring mount 384. FIG. 5A shows a plain view of spring 382. Spring 382 includes a flexible sheet 500. Two mounting holes 502 allow the spring 382 to be secured to spring arm 380 (not shown). At the opposite side of flexible sheet 500, spring 382 tapers to tip 504. FIG. 5B shows a sectional view of spring 382 along the line B-B of FIG. 5A. As shown in FIG. 5B, spring 382 has two bends 506, 508 which displace tip 504 below the portion of sheet 500 where the mounting holes 502 are located. In a preferred embodiment, the displacement 510 is approximately 1.5 mm. However, the displacement should be selected so as to ensure that tip 504 is the only portion of the spring assembly which makes contact with the suture (not shown). FIG. 5C shows a perspective view of spring 382 illustrating flexible sheet 500, mounting holes 502, bends 506, 508 and tip 504. In one preferred embodiment, spring 382 is made of stainless steel 0.1 mm in thickness.

The spring is used to develop holding force on the suture to hold it in place during cutting. The holding force should be selected to secure the suture without unduly deforming the suture. If the holding force selected is too high, the suture thread will be pushed into the gap of the anvil. If the holding force is too low, the suture thread will move during retainer formation and the quality of the retainers will be impaired. A holding force of less than 1 Newton is sufficient to hold a suture in place. In a preferred embodiment, a holding force of approximately 0.1N is sufficient for holding a 2-0 suture during retainer formation. For smaller diameter sutures, spring 382 is made of thinner stainless steel stock 0.030 mm in thickness. The thinner steel stock facilitates application of a lower holding force to the smaller diameter sutures. The amount of force applied by the spring is determined by the spring constant of the spring and the deflection of the tip of the spring when pushed into contact with the suture. As previously discussed the deflection of the spring is adjustable using the spring-arm adjuster 386 (See FIGS. 3A, 3B).

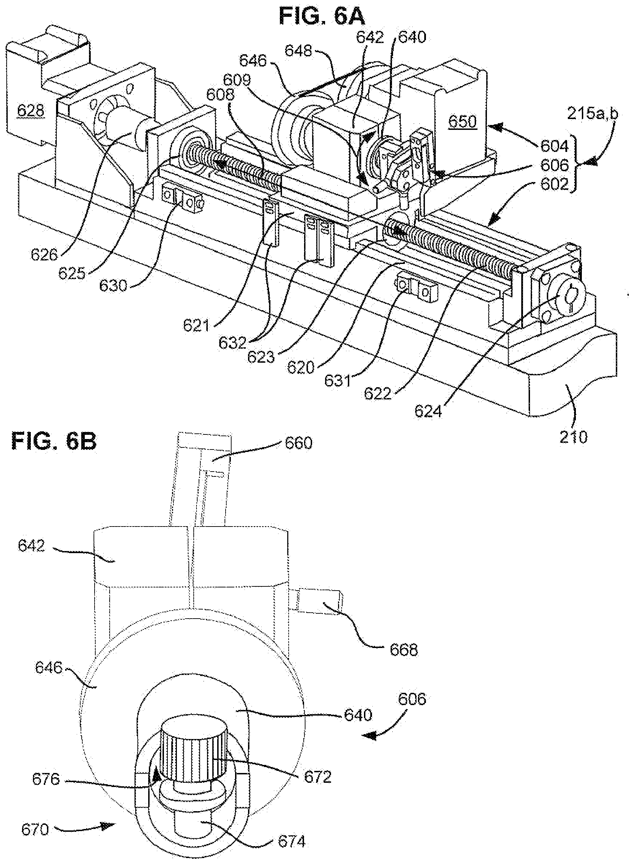

FIGS. 6A-6C show details of the chuck and chuck assembly. FIG. 6A shows a perspective view of chuck assembly 215a, 215b at one end of table 210. As shown in FIG. 6A, chuck assembly 215a, 215b has three main components: linear stage 602, rotary stage 604 and chuck 606. Linear stage 602 is mounted directly on to table 210. Rotary stage 604 is mounted on linear stage 602. Linear stage 602 is configured to move rotary stage 604 along table 210 as shown by arrow 608. Chuck 606 is mounted on rotary stage 604. Rotary stage 604 is configured to rotate chuck 606 to any desired angle. Chuck 606 is configured to hold one end of a suture thread 202. Chuck 606 has features which allow adjustment of the suture thread position to ensure that the suture thread 202 is precisely aligned with the axis of rotation of rotary stage 604 and chuck 606.

Referring again to FIG. 6A, linear stage 602 includes a linear track 620 to which a carriage 621 is mounted. A threaded rod 622 runs along the center of linear track 620 and through a bore 623 in carriage 621. A portion of bore 623 is threaded to engage threaded rod 622. One end of threaded rod 622 is mounted in a bushing 624. The other end of threaded rod 622 passes through a bushing 625 to a coupling 626 which connects threaded rod 622 to a high precision stepper motor 628. Operation of stepper motor 628 rotates threaded rod 622 in a precisely controllable manner. Because a portion of bore 623 is threaded to engage threaded rod 622, rotation of threaded rod 622 causes carriage 621 to translate along linear track 620 in a precisely controlled manner. A pair of limit switches 630, 631 is mounted to linear track 620. One or more tabs 632 are mounted on carriage 621 in such a way that they engage limit switches 630, 631 to prevent movement of carriage 621 beyond a desired range. In preferred embodiments, the position of the limit switches 630, 631 and/or tabs 632 is adjustable.

Rotary stage 604 is mounted on the upper surface of carriage 621. Rotary stage 604 includes a shaft 640 mounted through a bushing 642. Chuck 606 is mounted on one end of shaft 640. A gear 646 is mounted on the other end of shaft 640. Gear 646 is driven by gear 648 attached to a high precision stepper motor 650. Operation of stepper motor 650 rotates gears 648, 646 and shaft 640 in a precisely controllable manner thereby turning chuck 606 as shown by arrow 609.

FIG. 6B shows an enlarged perspective view of the outer end of chuck 606. As shown in FIG. 6B, chuck 606 is mounted on shaft 640 which is supported by bushing 642. A gear 646 is attached to the outer end of the shaft 640. Gear 646 is driven by gear 648 (see FIG 6A). In a preferred embodiment a chain is used to couple gear 648 to gear 646, however in alternative embodiments, a belt or gear-drive can be used. A tensioner 670 for holding a suture thread 202 is mounted to the outer end of shaft 640. A suture thread 202 is mounted to tensioner 670. Tensioner 670 has a rotary actuator 672 connected to a spool 674 to which the suture is mounted. Operation of rotary actuator 672 pulls the suture linearly through a bore 676 in shaft 640 onto spool 674. Rotary actuator 672 can thus be operated to control the tension and (in some cases) the longitudinal position of the suture thread.

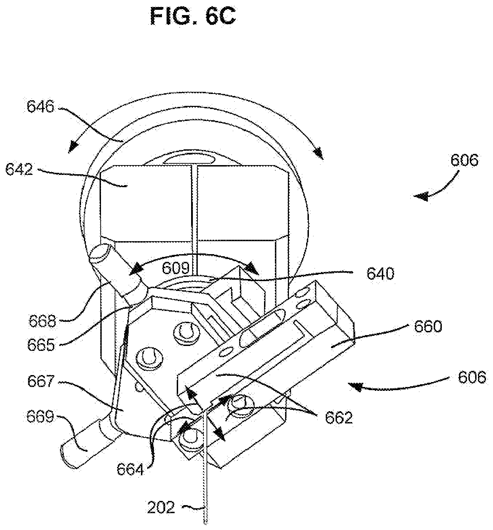

FIG. 6C shows an enlarged perspective view of the inner end of chuck 606. As shown in FIG. 6B, chuck 606 is mounted on shaft 640 which is supported by bushing 642. Chuck 606 includes a v-clamp 660 for positioning a suture thread. V-clamp 660 is mounted on the opposite end of shaft 640 as tensioner 670. A suture thread passes from tension 670 through aperture 676 in shaft 640 and then passes between jaws 662 of v-clamp 660. It is desirable to ensure that the suture thread 202 is accurately aligned with the axis of rotation of shaft 640 so that the suture thread does not oscillate vertically or horizontally when shaft 640 rotates. Thus, chuck 606 includes XY micrometer stages 665, 667 for adjusting the alignment of suture thread relative to the axis of rotation of shaft 640 as shown by arrows 664. V-clamp 660 is mounted on stage 665 which is mounted on stage 667 which is mounted to shaft 640. In the embodiment shown in FIG. 6B, micrometer stages 665, 667 are manually operated by rotation of actuators 668, 669, however, the manual XY micrometer stages 665, 667 are, in other embodiments replaced with an automatically controlled XY stage which senses the alignment of the suture and operates a motorized XY platform to align the suture thread 202 with the rotation axis.

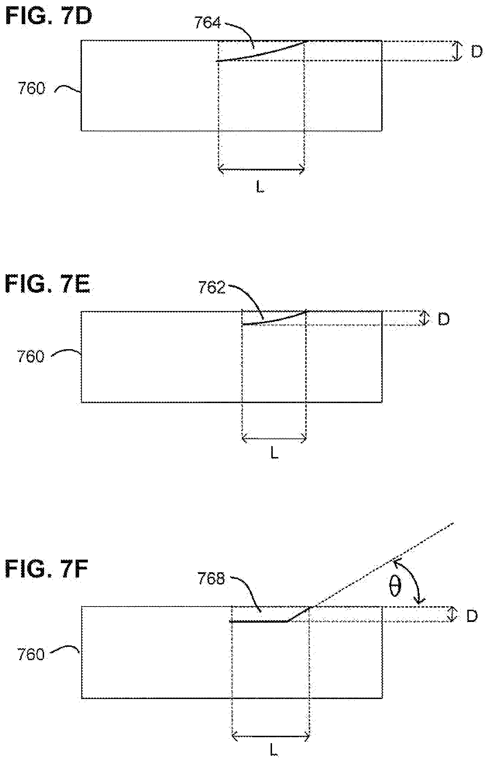

FIGS. 7A, 7B, and 7C show a range of retainer distributions and patterns that can be used in conjunction with a self-retaining suture. FIGS. 7D, 7E, and 7F show a range of retainer shapes that can be used in conjunction with a self-retaining suture. FIG. 7A shows a single helix distribution of retainers on a self-retaining suture according to an embodiment of the invention. FIG. 7B shows a double helix distribution of retainers on a self-retaining suture according to an embodiment of the invention. FIG. 7C shows a high quadra-helix density distribution of retainers on a drug-eluting self-retaining suture according to an embodiment of the invention.

Referring first to FIG. 7A which shows a single helix distribution of retainers 704 on a self-retaining suture. As shown in FIG. 7A, the self-retaining suture 700 has a suture thread 702 which is of USP 2-0, 4-0, 6-0, 7-0, 8-0, 9-0, 10-0, 11-0, 12-0 or below. As shown in FIG. 7A, the suture thread is a 4-0 suture having a diameter of 250µm. The self-retaining suture 700 includes a plurality of retainers 704 arranged in a helical pattern around and along the suture thread 702. As shown in FIG. 7A, the helix makes 5.7 twists per 25 mm (inch). In an embodiment the self-retaining suture has a barbed section 712 at least 60 mm in length and a 100 mm unbarbed lead 710, 714 on either side of the barbed section 712. The barbed section 712 may have retainers 704 in one orientation or in different orientations. Each retainer is 500µm from tip of depression to base of cut - measured axially - see arrow 716. The distance between the base of one retainer and the base of the adjacent retainer in the same helix (pitch) is 600µm - measured axially - see arrow 718.

In the embodiment of FIG. 7A, the pitch is 120% of the retainer length. In preferred embodiments, the pitch is less than 200% of the retainer length, more preferably less than 150% of the retainer length and even more preferably less than about 120% of the retainer length thereby enhancing retainer density and the tissue holding ability of a self-retaining suture. In the embodiment shown in FIG. 7A, retainers 704 are distributed at a density of 17 retainers per cm (42 retainers per inch) or 0.50 retainers per suture diameter in axial length. The retainer density of retainers in retainers per inch = n*25400/pitch (where n= no. of retainers in pattern e.g. n=1 for single helix, n=2 for double helix, n=4 for quadra-helix and wherein 25400 is the number of micrometers per inch). The retainer density of retainers in retainers per suture diameter in axial length = n*(suture diameter)/pitch (where n= no. of retainers in pattern e.g. n=1 for single helix, n=2 for double helix, n=4 for quadra-helix and wherein 25400 is the number of micrometers per inch). Note that it is not necessary that retainers be provided over 255 mm (one inch) of suture thread. The ratio of combined retainer length to suture length can be calculated by the formula n*(retainer length)/pitch and in FIG. 7A the ratio is 1*500µm/600µm or 0.83. In some preferred embodiments of the present invention the ratio of combined retainer length in a region bearing retainers to the length of the region is approximately 0.8 or greater.

Referring now to FIG. 7B which shows a double helix distribution of retainers 724 on a self-retaining suture 720. As shown in FIG. 7B, the self-retaining suture 720 has a suture thread 722 which is of USP 2-0, 4-0, 6-0, 7-0, 8-0, 9-0 10-0, 11-0, 12-0 or below. As shown in FIG. 7B, the suture thread is a 4-0 suture having a diameter of 250µm. The self-retaining suture 720 includes a plurality of retainers 724 arranged in a double helical pattern (n=2) around and along the suture thread 722. As shown in FIG. 7B, each helix makes 4.2 twists per 25 mm (inch). The helixes are also shifted axially by 0.49 mm relative to one another. In an embodiment, the self-retaining suture 720 has a barbed section 732 at least 100 mm in length and a 100 mm unbarbed lead 730, 734 on either side of the barbed section 732. The barbed section 732 may have retainers 724 in one orientation or in different orientations. Each retainer is 310µm from tip of depression to base of cut - measured axially - see arrow 736. The distance between the base of one retainer and the base of the adjacent retainer in the same helix (pitch) is 410µm - measured axially - see arrow 738.

In the embodiment of FIG. 7B, the pitch is 132% of the retainer length. In preferred embodiments, the pitch is less than 200% of the retainer length, more preferably less than 150% of the retainer length and even more preferably less than about 120% of the retainer length thereby enhancing retainer density and the tissue holding ability of a self-retaining suture. In the embodiment shown in FIG. 7B, the retainers 724 are distributed at a density of 48 retainers per cm (123 retainers per inch) or 1.21 retainers per suture diameter in axial length. The ratio of combined retainer length to suture length can be calculated by the formula n*(retainer length)/pitch and in FIG. 7B the ratio is 2*310µm/410µm or 1.51. The ratio of combined retainer length to suture length in the pattern of FIG. 7B, is about 1.51 i.e. the combined length of retainers (number of retainers times length of each retainer) in a portion of suture having retainers is 1.51 times larger than the length of the portion of suture. In some preferred embodiments of the present invention the ratio of combined retainer length in a region bearing retainers to the length of the region is greater than 1 and more preferably greater than 1.2 and more preferably greater than approximately 1.5.

Referring now to FIG. 7C which shows a high density distribution of retainers 744 on a self-retaining suture 740. As shown in FIG. 7C, the self-retaining suture 740 has a suture thread 742 which is of USP 2-0, 4-0, 6-0, 7-0, 8-0, 9-0 10-0, 11-0, 12-0 or below. As shown in FIG. 7C, the suture thread is a 4-0 suture 250µm nominal diameter. The self-retaining suture 740 includes a plurality of retainers 744 arranged in groups of four retainers in one plane (n=4), each arranged at 90 degrees spacing - a quadra-helix distribution. Each adjacent set of four retainers is offset to the adjacent sets by 45 degrees. In an embodiment, the self-retaining suture has a barbed section 752 at least 60 mm in length and a 100 mm unbarbed lead 750, 754 on either side of the barbed section 752. The barbed section 752 may have retainers 744 in one orientation or in different orientations. Each retainer is 180µm from tip of depression to base of cut - measured axially - see arrow 756. The distance between the base of the retainer in one set and the base of the adjacent retainers (pitch) is 280µm - measured axially - see arrow 758.

In the embodiment of FIG. 7B, the pitch is 155% of the retainer length. In preferred embodiments, the pitch is less than 200% of the retainer length, more preferably less than about 155% of the retainer length and even more preferably less than about 120% of the retainer length thereby enhancing retainer density and the tissue holding ability of a self-retaining suture. In the embodiment shown in FIG. 7C, the retainers 744 are distributed at a density of 143 retainers per cm (362 retainers per inch) or 3.57 retainers per suture diameter in axial length. The ratio of combined retainer length to suture length can be calculated by the formula n*(retainer length)/pitch and in FIG. 7C the ratio is 4*180µm/280µm or 2.57. The ratio of combined retainer length to suture length in the pattern of FIG. 7C, is about 2.57 i.e. the combined length of retainers (number of retainers times length of each retainer) in a portion of suture having retainers is 2.57 times larger than the length of the portion of suture. In some preferred embodiments of the present invention the ratio of combined retainer length in a region bearing retainers to the length of the region is greater than 2 and more preferably greater than approximately 2.5.

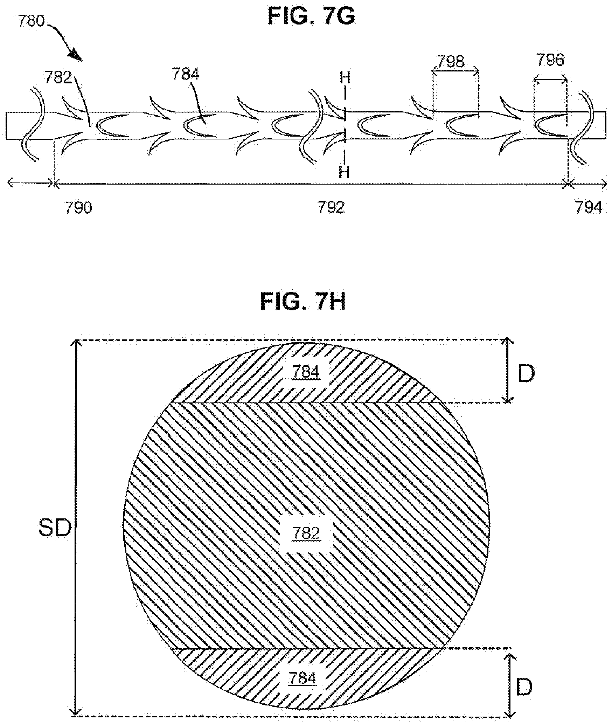

FIGS. 7D, 7E, and 7F show a range of retainer shapes that can be used in the distribution patterns described above and in FIG 7G and accompanying text. The retainer shapes can be controlled by adjustments to blade angle, blade shape, and plough angle and other parameters. For each retainer, shown in FIGS. 7D-7F, a USP 4-0 suture thread 760 having a diameter of about 250µm is used. However the retainer shapes can be scaled for other suture diameters from 1000 to 50µm and smaller for example.

Referring first to FIG. 7D which shows a parabolic retainer 764 for use with either the single-helix (FIG. 7A), double helix (FIG. 7B) or quadra-helix (FIG. 7C) distribution patterns. The depth of cut D (measured transversely) is 60µm. Note that the depth of cut is within the range of between 5% and 35% of the suture thread diameter. The length of cut L (measured axially) is 250µm. Note that the length of cut is within the range of 200% to 800% of the depth of cut. The term aspect ratio can be applied to a retainer to describe the ratio of the retainer length to the depth of cut. Thus, for example, the aspect ratio of parabolic retainer 764 is L/D which equals 4.1 in this embodiment.

FIG. 7E shows a parabolic retainer 762 shape for use with the high density (FIG. 7C) distribution patterns. The depth of cut D (measured transversely) is 36µm which is within the range of between 5% and 35% of the suture thread diameter. The length of cut L (measured axially) is 170µm s within the range of 200% to 800% of the depth of cut. The aspect ratio of parabolic retainer 762 is 4.7 in this embodiment.

FIG. 7F shows an alternative retainer 768 having a 30 degree entry (angle θ) and then running parallel to the axis of the suture. The depth of cut D (measured transversely) is 36µm. The length of cut L (measured axially) is 234µm. The angle of entry θ is initially 30 degrees from the suture axis. The aspect ratio of retainer 768 is increased relative to the parabolic retainer shapes - the aspect ratio of retainer 768 is 6.5 in this embodiment.

The retainer shapes described in FIGS. 7D-7F and other retainer shapes can be used to accommodate the density and distribution of retainers desired for particular applications and with particular suture thread diameters. The shape of the retainers can be configured by adjusting the parameters of the cutting assembly previously described. Alternative retainer shapes and distribution patterns are disclosed in U.S. Patent Application 12/101885 titled "Self-Retaining Systems For Surgical Procedures" filed April 11, 2008 (Atty. Dkt. No. ANGIO-01000US7).

Referring now to FIG. 7G which shows a double helix distribution of retainers 784 on a self-retaining suture 780. FIG. 7G illustrates a particular case of the double helix distribution in which the retainers are in-phase, i.e. the retainers of both helices are formed at substantially the same positions axially along the suture. In the embodiment shown the retainers are formed on opposite sides of the suture thread from one another. As shown in FIG. 7G, the self-retaining suture 780 has a suture thread 782 which is of USP 2-0, 4-0, 6-0, 7-0, 8-0, 9-0 10-0, 11-0, 12-0 or below. As shown in FIG. 7G, the suture thread is a 2-0 suture having a diameter of about 330µm. The self-retaining suture 780 includes a plurality of retainers 784 arranged in a double helical pattern (n=2) around and along the suture thread 782. The helices are in-phase with one another so there is little or no axial shift between the helices/retainer. In the embodiment shown, adjacent pairs of retainers are rotated 90° relative to the adjacent pairs of retainers. The helical pattern is equivalent to 12.7 twists per 25 mm (inch) of the suture. The distance between the base of one retainer and the base of the adjacent retainer in the same helix (pitch) is 500µm - measured axially - see arrow 798. Each retainer is 420µm from tip of depression to base of cut - measured axially - see arrow 796. In an embodiment, the self-retaining suture 780 has a barbed section 792 at least 100 mm in length and a 100 mm unbarbed lead 790, 794 on either side of the barbed section 792. The barbed section 792 may have retainers 784 in one orientation or in different orientations. The suture can also include a transition region - having no retainers - between regions having retainers oriented in opposite directions.

In the embodiment of FIG. 7G the pitch is 119% of the retainer length. In preferred embodiments, the pitch is less than 200% of the retainer length, more preferably less than 150% of the retainer length and even more preferably less than about 120% of the retainer length thereby enhancing retainer density and the tissue holding ability of a self-retaining suture. In this configuration, the self-retaining suture has two retainers every 500µm yielding a retainer density of 40 retainers per cm (102 retainers per inch) or 1.32 retainers per suture diameter in axial length. The ratio of combined retainer length to suture length can be calculated by the formula n*(retainer length)/pitch and in FIG. 7G the ratio is 2*420µm/500µm or 1.68. Thus, the ratio of combined retainer length to suture length in the barbed section 792 of FIG. 7G, is about 1.68 i.e. the combined length of retainers (number of retainers times length of each retainer) in a portion of suture is 1.68 times larger than the length of the portion of suture. In some preferred embodiments of the present invention the ratio of combined retainer length in a region bearing retainers to the length of the region is greater than 1 and more preferably greater than approximately 1.5.

FIG. 7H shows a sectional view of the self-retaining suture of FIG. 7G along the line H-H which coincides with maximum depth of cut of the retainers 784. FIG. 7G shows a central uncut portion of the suture thread 782 responsible for the tensile strength of the suture and two segments of the suture forming the base of retainers 784. In the embodiment described in FIG. 7G, the depth of cut D is 70µm whereas the suture diameter 330µm. Calculating the area of the two segments representing the retainers 784, demonstrates that approximately 70% of the cross-section of the suture thread 782 remains to provide the tensile strength of the suture thread 782. In preferred embodiments, in this and other distribution patterns, 70% or more of the suture section remains after cutting the retainers to provide tensile strength. The retainer distribution pattern and retainer shape (including depth of cut) can be selected to achieve this result. Similarly, in the embodiment of FIG. 7C, where four retainers are cut in the same plane, a shallow depth of cut is chosen so as to leave a sufficient suture section to provide tensile strength to the self-retaining suture 740.

### Self-Retaining Suture Examples

A plurality of self-retaining sutures have been made on the apparatus described above. The sutures have been formed on a range of stock suture threads with different retainer configurations and distributions. In some cases, the tissue holding strength of the retainers has been assessed to evaluate the function of retainer shape and bar distribution. Factors such as retainer length and plow angle had the most effect on retainer retention performance in addition to retainer density for a particular diameter of suture. Note however, that the optimum retainer length and distribution can differ dependent upon the tissue in which the suture is used and the manner in which the suture is to be deployed.

### Example A. Lot 36

FIG. 8A shows an image of a self-retaining suture 800a made utilizing the cutting apparatus previously described. Self-retaining suture 800a was formed by cutting retainers 802a in a quadra-helix pattern (n=4) into a polypropylene suture thread 804a of USP 2-0 (300µm nominal diameter) using a 28mm diameter circular blade. Four retainers were cut at each axial position on the suture - the suture was rotated 90° between each cut. The suture was then translated axially by 1000µm and rotated 45° before commencing cutting of the next four retainers. To form the retainers 802a on the polypropylene suture thread 804a; the parameters of the cutting assembly were set as follows: blade angle 38.89°, plow angle 10°, cutting stage angle 78°, depth of cut 50µm, and pitch 1000µm. The resulting retainer length was 359µm at a density of approximately 40 retainers per cm (102 retainers per inch) or 1.20 retainers per suture diameter in axial length. The retainer aspect ratio (retainer length/cut depth) was approximately 7.2. The resulting ratio of combined retainer length to suture length was 1.4 i.e. the combined length of retainers (number of retainers times length of each retainer) in a portion of suture having retainers was 1.4 times larger than the length of the portion of suture. The self-retaining suture 800a was tested for holding strength with a straight tensile pull test through pork skin fat layer (see methods below). The mean force maximum over 15 tests was 4.045N.

### Example B. Lot 37

FIG. 8B shows an image of a self-retaining suture 800b made utilizing the cutting apparatus previously described. Self-retaining suture 800b was formed by cutting retainers 802b in a quadra-helix pattern (n=4) into a polypropylene suture thread 804b of USP 2-0 (300µm nominal diameter) using a 28mm diameter circular blade. Four retainers were cut at each axial position on the suture - the suture was rotated 90° between each cut. The suture was then translated axially by 500µm and rotated 45° before commencing cutting of the next four retainers. To form the retainers 802b on the polypropylene suture thread 804b, the parameters of the cutting assembly were set as follows: blade angle 38.89°, plow angle 10°, cutting stage angle 78°, depth of cut 50µm, and pitch 500µm. The resulting retainer length was 331µm at a density of approximately 80 retainers per cm (204 retainers per inch) or 2.4 retainers per suture diameter in axial length. The retainer aspect ratio was approximately 6.6. The resulting ratio of combined retainer length to suture length was 2.6. The self-retaining suture 800b was tested for holding strength with a straight tensile pull test through pork skin fat layer (see methods below). The mean force maximum over 15 tests was 5.045N.

### Example C. Lot 38

FIG. 8C shows an image of a self-retaining suture 800c made utilizing the cutting apparatus previously described. Self-retaining suture 800c was formed by cutting retainers 802c in a quadra-helix pattern (n=4) into a polypropylene suture thread 804c of USP 2-0 (300µm nominal diameter) using a 28mm diameter circular blade. Four retainers were cut at each axial position on the suture - the suture was rotated 90° between each cut. The suture was then translated axially by 440µm and rotated 45° before commencing cutting of the next four retainers. To form the retainers 802c on the polypropylene suture thread 804c, the parameters of the cutting assembly were set as follows: blade angle 38.89°, plow angle 8°, cutting stage angle 78°, depth of cut 50µm, and pitch 440µm. The resulting retainer length was 375µm at a density of 91 retainers per cm (230 retainers per inch) or 2.73 retainers per suture diameter in axial length. The retainer aspect ratio was approximately 7.5. The resulting ratio of combined retainer length to suture length was 3.4. The self-retaining suture 800c was tested for holding strength with a straight tensile pull test through pork skin fat layer (see methods below). The mean force maximum over 15 tests was 4.651N.

### Example D. Lot 39

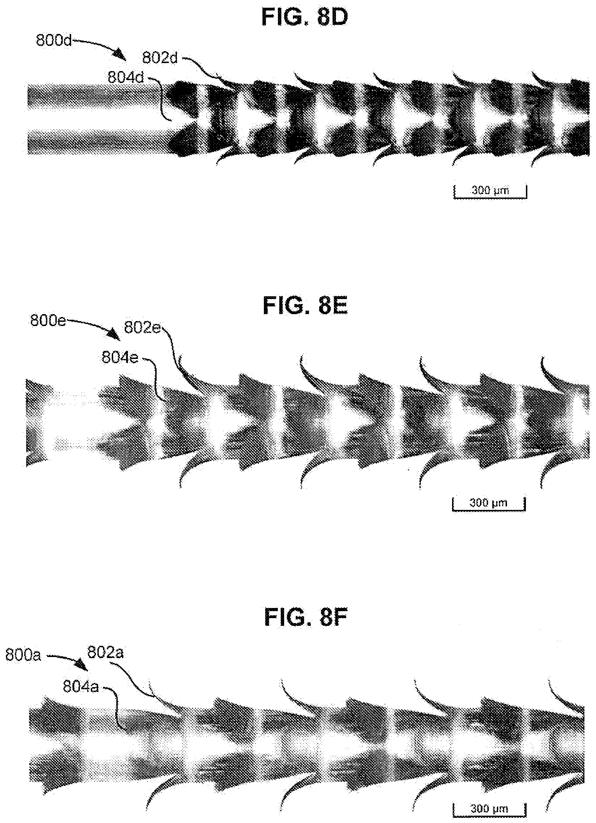

FIG. 8D shows an image of a self-retaining suture 800d made utilizing the cutting apparatus previously described. Self-retaining suture 800d was formed by cutting retainers 802d in a quadra-helix pattern (n=4) into a polypropylene suture thread 804d of USP 2-0 (300µm nominal diameter) using a 28mm diameter circular blade. Four retainers were cut at each axial position on the suture - the suture was rotated 90° between each cut. The suture was then translated axially by 170µm and rotated 45° before commencing cutting of the next four retainers. To form the retainers 802d on the polypropylene suture thread 804d, the parameters of the cutting assembly were set as follows: blade angle 38.89°, plow angle 8°, cutting stage angle 86°, depth of cut 50µm, and pitch 170µm. The resulting retainer length was 156µm at a density of235 retainers per inch (596 retainers per inch) or 7.06 retainers per suture diameter in axial length. The retainer aspect ratio was approximately 3.1. The resulting ratio of combined retainer length to suture length was 3.6. The self-retaining suture 800d was tested for holding strength with a straight tensile pull test through pork skin fat layer (see methods below). The mean force maximum over 15 tests was 3.280N.

### Example E. Lot 40

FIG. 8E shows an image of a self-retaining suture 800e made utilizing the cutting apparatus previously described. Self-retaining suture 800e was formed by cutting retainers 802e in a quadra-helix pattern (n=4) into a polypropylene suture thread 804e of USP 2-0 (300µm nominal diameter) using a 28mm diameter circular blade. Four retainers were cut at each axial position on the suture - the suture was rotated 90° between each cut. The suture was then translated axially by 270µm and rotated 45° before commencing cutting of the next four retainers. To form the retainers 802e on the polypropylene suture thread 804e, the parameters of the cutting assembly were set as follows: blade angle 38.89°, plow angle 4°, cutting stage angle 86°, depth of cut 50µm, and pitch 270µm. The resulting retainer length was 255µm at a density of 148 retainers per cm (376 retainers per inch) or 4.44 retainers per suture diameter in axial length. The retainer aspect ratio was approximately 5.1. The resulting ratio of combined retainer length to suture length was 3.8. The self-retaining suture 800e was tested for holding strength with a straight tensile pull test through pork skin fat layer (see methods below). The mean force maximum over 15 tests was 5.159N.

### Example F. Lot 41

FIG. 8F shows an image of a self-retaining suture 800f made utilizing the cutting apparatus previously described. Self-retaining suture 800fwas formed by cutting retainers 802f in a quadra-helix pattern (n=4) into a polypropylene suture thread 804f of USP 2-0 (300µm nominal diameter) using a 28mm diameter circular blade. Four retainers were cut at each axial position on the suture - the suture was rotated 90° between each cut. The suture was then translated axially by 330µm and rotated 45° before commencing cutting of the next four retainers. To form the retainers 802f on the polypropylene suture thread 804f, the parameters of the cutting assembly were set as follows: blade angle 30.56°, plow angle 4°, cutting stage angle 86°, depth of cut 50µm, and pitch 330µm. The resulting retainer length was 301µm at a density of 121 retainers per cm (308 retainers per inch) or 3.64 retainers per suture diameter in axial length. The retainer aspect ratio was approximately 6.0. The resulting ratio of combined retainer length to suture length was 3.6. The self-retaining suture 800f was tested for holding strength with a straight tensile pull test through pork skin fat layer (see methods below). The mean force maximum over 15 tests was 5.432N. The testing identified lot 41 as having the best retainer retention strength from among lots 36-43 under the test conditions.

### Example G. Lot 42

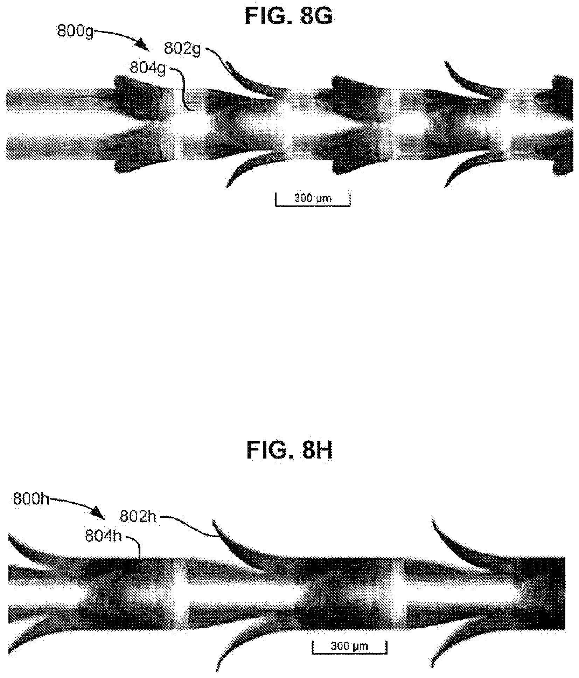

FIG. 8G shows an image of a self-retaining suture 800g made utilizing the cutting apparatus previously described. Self-retaining suture 800g was formed by cutting retainers 802g in a quadra-helix pattern (n=4) into a polypropylene suture thread 804g of USP 2-0 using a 28mm diameter circular blade. Four retainers were cut at each axial position on the suture - the suture was rotated 90° between each cut. The suture was then translated axially by 500µm and rotated 45° before commencing cutting of the next four retainers. To form the retainers 802g on the polypropylene suture thread 804g, the parameters of the cutting assembly were set as follows: blade angle 38.89°, plow angle 10°, cutting stage angle 78°, depth of cut 50µm, and pitch 500µm. The resulting retainer length was 356µm at a retainer density of 80 retainers per cm (204 retainers per inch) or 2.40 retainers per suture diameter in axial length. The retainer aspect ratio was approximately 7.1. The resulting ratio of combined retainer length to suture length was 2.8. The self-retaining suture 800g was tested for holding strength with a straight tensile pull test through pork skin fat layer (see methods below). The mean force maximum over 50 tests was 5.112N.

### Example H. Lot 43

FIG. 8H shows an image of a self-retaining suture 800h made utilizing the cutting apparatus previously described. Self-retaining suture 800h was formed by cutting retainers 802h in a double helix pattern (n=2) into a polypropylene suture thread 804h of USP 2-0 (300µm nominal diameter) using a 28mm diameter circular blade. As described above with respect to FIG. 7G, the two helices are in-phase, i.e. the retainers of both helices are formed at substantially the same positions axially along the suture. The retainers are also formed on opposite sides of the suture thread from one another. Each pair of retainers is rotated 90° relative to the adjacent pairs of retainers. Two retainers were cut at each axial position on the suture - the suture was rotated 180° between cutting the two retainers. The suture was then translated axially by 500µm and rotated 90° before commencing cutting of the next two retainers. To form the retainers 802h on the polypropylene suture thread 804h, the parameters of the cutting assembly were set as follows: blade angle 38.89°, plow angle 10°, cutting stage angle 78°, depth of cut 50µm, and pitch 500µm. The resulting retainer length was 435µm at a retainer density of 40 retainers per cm (102 retainers per inch) or 1.20 retainers per suture diameter in axial length. The retainer aspect ratio was approximately 8.7. The resulting ratio of combined retainer length to suture length was 1.7. The self-retaining suture 800h was tested for holding strength with a straight tensile pull test through pork skin fat layer (see methods below). The mean force maximum over 50 tests was 5.060N.

### Example I. Lot 45

FIG. 8I shows an image of a self-retaining suture 800i made utilizing the cutting apparatus previously described. Self-retaining suture 800i was formed by cutting retainers 802i in a bidirectional double helix pattern (n=2) into a polypropylene suture thread 804i of USP 6-0 (96µm nominal diameter) using a sapphire blade. In this embodiment the helices are out of phase, i.e. the retainers of one helix are axially displaced along the suture from the retainers of the other helix. In the embodiment shown, the axial displacement is approximately equal to the axial retainer length. Each retainer in each helix is rotated 90° relative to the adjacent retainers in the same helix. To form the retainers 802i on the polypropylene suture thread 804i, the parameters of the cutting assembly were set as follows: blade angle 30°, plow angle 12°, cutting stage angle 78°, depth of cut 32µm, and pitch 70µm. The suture thread was reversed and re-indexed for cutting retainers in the opposite direction. A transition region 806i, without retainers is located between the retainers oriented in opposition directions. The resulting retainer length was 80µm at a retainer density of286 retainers per cm (726 retainers per inch) or 2.74 retainers per suture diameter in axial length. The retainer aspect ratio was approximately 2.5. The resulting ratio of combined retainer length to suture length was 2.2.

### Example J. Lot 46

FIG. 8J shows an image of a self-retaining suture 800j made utilizing the cutting apparatus previously described. Self-retaining suture 800j was formed by cutting retainers 802j in a quadra-helix pattern (n=4) into a polypropylene suture thread 804j of USP 6-0 (96µm nominal diameter) using a sapphire blade. Four retainers were cut at each axial position on the suture - the suture was rotated 90° between each cut. The suture was then translated axially by 90µm and rotated 45° before commencing cutting of the next four retainers. To form the retainers 802j on the polypropylene suture thread 804j the parameters of the cutting assembly were set as follows: blade angle 30°, plow angle 12°, cutting stage angle 78°, depth of cut 15µm, and pitch 90µm. The resulting retainer length was 65µm at a retainer density of444 retainers per cm (1128 retainers per inch) or 4.27 retainers per suture diameter in axial length. The retainer aspect ratio was approximately 4.3. The resulting ratio of combined retainer length to suture length was 2.9.

### Example K. Lot Test 8-0

FIG. 8K shows an image of a self-retaining suture 800k made utilizing the cutting apparatus previously described. Self-retaining suture 800k was formed by cutting retainers 802k in a quadra-helix pattern (n=4) into a polypropylene suture thread 804k of USP 8-0 (50µm nominal diameter) using a sapphire blade. Four retainers were cut at each axial position on the suture - the suture was rotated 90° between each cut. The suture was then translated axially by 60µm and rotated 45° before commencing cutting of the next four retainers. To form the retainers 802k on the polypropylene suture thread 804k the parameters of the cutting assembly were set as follows: blade angle 30°, plow angle 12°, cutting stage angle 78°, depth of cut 7µm, and pitch 60µm. The resulting retainer length was 40µm at a retainer density greater than 665 retainers per cm (1690 retainers per inch) or 3.33 retainers per suture diameter in axial length. The retainer aspect ratio was approximately 5.7. The resulting ratio of combined retainer length to suture length was 2.7. FIG. 8o shows an enlarged view of a segment of self-retaining suture 800k of FIG 8K.

### Example L. Lot 44

FIG. 8L shows an image of a self-retaining suture 800L made utilizing the cutting apparatus previously described. Self-retaining suture 800L was formed by cutting retainers 802L in a quadra-helix pattern (n=4) into a polypropylene suture thread 804L of USP 6-0 (96µm nominal diameter) using a sapphire blade. Four retainers were cut at each axial position on the suture - the suture was rotated 90° between each cut. The suture was then translated axially by 90µm and rotated 45° before commencing cutting of the next four retainers. To form the retainers 802L on the polypropylene suture thread 804L, the parameters of the cutting assembly were set as follows: cutting stage angle 78°, plow angle 12°, blade angle 30°, depth of cut 15µm, and pitch 90µm. The suture thread was reversed and re-indexed for cutting retainers in the opposite direction. A transition region 806L, without retainers is located between the retainers oriented in opposition directions. The resulting retainer length was 65µm at a density of 444 retainers per cm (1128 retainers per inch) or 4.27 retainers per suture diameter in axial length. The retainer aspect ratio was approximately 4.3. The ratio of combined retainer length to suture length was 2.9.

### Example M. Lot 47

FIG. 8M shows an image of a self-retaining suture 800m made utilizing the cutting apparatus previously described. Self-retaining suture 800m was formed by cutting retainers 802m in a quadra-helix pattern (n=4) into a polypropylene suture thread 804m of USP 6-0 (96µm nominal diameter) using a sapphire blade. Four retainers were cut at each axial position on the suture - the suture was rotated 90° between each cut. The suture was then translated axially by 90µm and rotated 45° before commencing cutting of the next four retainers. To form the retainers 802m on the polypropylene suture thread 804m, the parameters of the cutting assembly were set as follows: cutting stage angle 78°, plow angle 12°, blade angle 30°, depth of cut 15µm, and pitch 90µm. The resulting retainer length was 60µm at a density of 444 retainers per cm (1128 retainers per inch) or 4.27 retainers per suture diameter in axial length. The retainer aspect ratio was approximately 4.0. The ratio of combined retainer length to suture length was 2.7.

### Example N. Lot 48

FIG. 8N shows an image of a self-retaining suture 800n made utilizing the cutting apparatus previously described. Self-retaining suture 800n was formed by cutting retainers 802n in a quadra-helix pattern (n=4) into a polypropylene suture thread 804n of USP 2-0 (300µm nominal diameter) using a 28mm diameter circular blade. Four retainers were cut at each axial position on the suture - the suture was rotated 90° between each cut. The suture was then translated axially by 330µm and rotated 45° before commencing cutting of the next four retainers. To form the retainers 802n on the polypropylene suture thread 804n, the parameters of the cutting assembly were set as follows: cutting stage angle 86°, plow angle 4°, blade angle 30.56° Seventy five suture samples were made in order to test the tissue-holding strength of the suture in a variety of tissues. The retainers were measured in three samples and the average measured retainer parameters were: suture diameter 340µm depth of cut 50µm, pitch 332µm; retainer length was 278µm at a density of 120 retainers per cm (306 retainers per inch) or 4.1 retainers per suture diameter in axial length. The retainer aspect ratio was approximately 5.6. The ratio of combined retainer length to suture length was 3.3. The self-retaining suture 800n was tested for holding strength with a straight tensile pull test through a variety of tissues as described below.

### Example P. Lot 49

FIG. 8P shows an image of a self-retaining suture 800p made utilizing the cutting apparatus previously described. Self-retaining suture 800p was formed by cutting retainers 802p in a double-helix pattern (n=2) into a polypropylene suture thread 804p of USP 2-0 (300µm nominal diameter) using a 28mm diameter circular blade. As described above with respect to FIG. 7G, the two helices are in-phase, i.e. the retainers of both helices are formed at substantially the same positions axially along the suture. The retainers are also formed on opposite sides of the suture thread from one another. Each pair of retainers is rotated 90° relative to the adjacent pairs of retainers. Two retainers were cut at each axial position on the suture - the suture was rotated 180° between cutting the two retainers. The suture was then translated axially by 500µm and rotated 90° before commencing cutting of the next two retainers. To form the retainers 802p on the polypropylene suture thread 804p, the parameters of the cutting assembly were set as follows: cutting stage angle 76°, plow angle 11.5°, blade angle 38.89°. Seventy five suture samples were made in order to test the tissue-holding strength of the suture in a variety of tissues. The retainers were measured in three samples and the average measured retainer parameters were: suture diameter 336µm, depth of cut 76µm, and pitch 499µm, and retainer length 422µm at a density of 40 retainers per cm (102 retainers per inch) or 1.36 retainers per suture diameter in axial length. The retainer aspect ratio was approximately 5.6. The ratio of combined retainer length to suture length was 1.7. The self-retaining suture 800p was tested for holding strength with a straight tensile pull test through a variety of tissues as described below.

### Example Q. Lot 50

FIG. 8Q shows an image of a self-retaining suture 800q made utilizing the cutting apparatus previously described. Self-retaining suture 800q was formed by cutting retainers 802q in a double-helix pattern (n=2) into a polypropylene suture thread 804q of USP 2-0 (300µm nominal diameter) using a 28mm diameter circular blade. As described above with respect to FIG. 7G, the two helices are in-phase, i.e. the retainers of both helices are formed at substantially the same positions axially along the suture. The retainers are also formed on opposite sides of the suture thread from one another. Each pair of retainers is rotated 90° relative to the adjacent pairs of retainers. Two retainers were cut at each axial position on the suture - the suture was rotated 180° between cutting the two retainers. The suture was then translated axially by 500µm and rotated 90° before commencing cutting of the next two retainers. To form the retainers 802q on the polypropylene suture thread 804q, the parameters of the cutting assembly were set as follows: cutting stage angle 76°, plow angle 11.5°, blade angle 38.89°. Seventy five suture samples were made. The retainers were measured in three samples and the average measured retainer parameters were: suture diameter 321µm, depth of cut 71µm, and pitch 498µm, and retainer length 409µm at a density of 40 retainers per cm (102 retainers per inch) or 1.29 retainers per suture diameter in axial length. The retainer aspect ratio was approximately 5.8. The ratio of combined retainer length to suture length was 1.6.

### Example R. Lot 51

FIG. 8R shows an image of a self-retaining suture 800r made utilizing the cutting apparatus previously described. Self-retaining suture 800r was formed by cutting retainers 802r in a double-helix pattern (n=2) into a polypropylene suture thread 804r of USP 2-0 (300µm nominal diameter) using a 28mm diameter circular blade. In this embodiment the helices are out of phase, i.e. the retainers of one helix are axially displaced along the suture from the retainers of the other helix. In the embodiment shown, the axial displacement is approximately equal to the axial retainer length. Each retainer in each helix is rotated 90° relative to the adjacent retainers in the same helix. Two retainers were cut at each axial position on the suture - the suture was rotated 180° between cutting the two retainers. The suture was then translated axially by 430µm and rotated 90° before commencing cutting of the next two retainers. To form the retainers 802r on the polypropylene suture thread 804r, the parameters of the cutting assembly were set as follows: cutting stage angle 76°, plow angle 11.5°, blade angle 38.89°. Fifteen suture samples were made. The retainers were measured in three samples and the average measured retainer parameters were: suture diameter 333µm, depth of cut 99µm, and pitch 431µm, and retainer length 501µm at a density of 46 retainers per cm (118 retainers per inch) or 1.55 retainers per suture diameter in axial length. The retainer aspect ratio was approximately 5.1. The ratio of combined retainer length to suture length was 2.3.

### Example S. Lot 52

FIG. 8S shows an image of a self-retaining suture 800s made utilizing the cutting apparatus previously described. Self-retaining suture 800s was formed by cutting retainers 802s in a quadra-helix pattern (n=4) into a polypropylene suture thread 804s of USP 2-0 (300µm nominal diameter) using a 28mm diameter circular blade. Four retainers were cut at each axial position on the suture - the suture was rotated 90° between cutting the four retainers. The suture was then translated axially by 470µm and rotated 45° before commencing cutting of the next four retainers. To form the retainers 802s on the polypropylene suture thread 804s, the parameters of the cutting assembly were set as follows: cutting stage angle 76°, plow angle 11.5°, blade angle 38.89°. Fifteen suture samples were made. The retainers were measured in three samples and the average measured retainer parameters were: suture diameter 331µm, depth of cut 50µm, and pitch 468µm, and retainer length 348µm at a density of 85 retainers per cm (217 retainers per inch) or 2.84 retainers per suture diameter in axial length. The retainer aspect ratio was approximately 7.0. The ratio of combined retainer length to suture length was 2.9.

### Example T. Lot 53

FIG. 8T shows an image of a self-retaining suture 800t made utilizing the cutting apparatus previously described. Self-retaining suture 800t was formed by cutting retainers 802t in a double-helix pattern (n=2) into a polypropylene suture thread 804t of USP 3-0 (200µm nominal diameter) using a 28mm diameter circular blade. As described above with respect to FIG. 7G, the two helices are in-phase, i.e. the retainers of both helices are formed at substantially the same positions axially along the suture. The retainers are also formed on opposite sides of the suture thread from one another. Each pair of retainers is rotated 90° relative to the adjacent pairs of retainers. Two retainers were cut at each axial position on the suture - the suture was rotated 180° between cutting the two retainers. The suture was then translated axially by 400µm and rotated 90° before commencing cutting of the next two retainers. To form the retainers 802t on the polypropylene suture thread 804t, the parameters of the cutting assembly were set as follows: cutting stage angle 76°, plow angle 9.6°, blade angle 38.89°. Ninety suture samples were made. The retainers were measured in four samples and the average measured retainer parameters were: suture diameter 249µm, depth of cut 53µm, and pitch 401 µm, and retainer length 281 µm at a density of 50 retainers per cm (127 retainers per inch) or 1.24 retainers per suture diameter in axial length. The retainer aspect ratio was approximately 5.3. The ratio of combined retainer length to suture length was 1.4.

### Example U. Lot 54

FIG. 8U shows an image of a self-retaining suture 800u made utilizing the cutting apparatus previously described. Self-retaining suture 800u was formed by cutting retainers 802u in a double-helix pattern (n=2) into a polypropylene suture thread 804u of USP 4-0 (150µm nominal diameter) using a 28mm diameter circular blade. As described above with respect to FIG. 7G, the two helices are in-phase, i.e. the retainers of both helices are formed at substantially the same positions axially along the suture. The retainers are also formed on opposite sides of the suture thread from one another. Each pair of retainers is rotated 90° relative to the adjacent pairs of retainers. Two retainers were cut at each axial position on the suture - the suture was rotated 180° between cutting the two retainers. The suture was then translated axially by 250µm and rotated 90° before commencing cutting of the next two retainers. To form the retainers 802u on the polypropylene suture thread 804u, the parameters of the cutting assembly were set as follows: cutting stage angle 76°, plow angle 9.5°, blade angle 38.89°, Fifteen suture samples were made. The retainers were measured in five samples and the average measured retainer parameters were: suture diameter 196µm, depth of cut 40µm, and pitch 250µm, and retainer length 221µm at a density of 80 retainers per cm (203 retainers per inch) or 1.57 retainers per suture diameter in axial length. The retainer aspect ratio was approximately 5.5. The ratio of combined retainer length to suture length was 1.8.

### Example V. Lot 55

FIG. 8V shows an image of a self-retaining suture 800v made utilizing the cutting apparatus previously described. Self-retaining suture 800v was formed by cutting retainers 802v in a double-helix pattern (n=2) into a polypropylene suture thread 804v of USP 2-0 (300µm nominal diameter) using a 29.9mm diameter circular blade. As described above with respect to FIG. 7G, the two helices are in-phase, i.e. the retainers of both helices are formed at substantially the same positions axially along the suture. The retainers are also formed on opposite sides of the suture thread from one another. Each pair of retainers is rotated 90° relative to the adjacent pairs of retainers. Two retainers were cut at each axial position on the suture - the suture was rotated 180° between cutting the two retainers. The suture was then translated axially by 700µm and rotated 90° before commencing cutting of the next two retainers. To form the retainers 802v on the polypropylene suture thread 804v, the parameters of the cutting assembly were set as follows: cutting stage angle 76°, plow angle 9.5°, blade angle 38.89°. Fifteen suture samples were made. The retainers were measured in one sample and the measured retainer parameters were: suture diameter 325µm, depth of cut 68µm, and pitch 700µm, and retainer length 530µm at a density of 28 retainers per cm (72 retainers per inch) or 0.93 retainers per suture diameter in axial length. The retainer aspect ratio was approximately 7.8. The ratio of combined retainer length to suture length was 1.5.

### Example W. Double Helix 10-0

FIG. 8W shows an image of a 10-0 double-helix self-retaining suture made according to an embodiment of the invention. Self-retaining suture 800w was formed by cutting retainers 802w in a double-helix pattern (n=2) into a polypropylene suture thread 804w of USP 10-0 (32µm nominal diameter) using a sapphire blade. As described above with respect to FIG. 7G, the two helices are in-phase, i.e. the retainers of both helices are formed at substantially the same positions axially along the suture. The retainers are also formed on opposite sides of the suture thread from one another. Each pair of retainers is rotated 90° relative to the adjacent pairs of retainers. Two retainers were cut at each axial position on the suture - the suture was rotated 180° between cutting the two retainers. The suture was then translated axially and rotated 90° before commencing cutting of the next two retainers. To form the retainers 802w on the polypropylene suture thread 804w, the parameters of the cutting assembly were set as follows: cutting stage angle 78°, plow angle 12°, blade angle 22.22°. The resulting pitch was 48µm, cut depth was 7µm and retainer length was 30µm at a density of 420 retainers per cm (1058 retainers per inch) or 1.33 retainers per suture diameter in axial length. The retainer aspect ratio was approximately 4.3. The ratio of combined retainer length to suture length was 1.25. FIG. 8X shows an enlarged view of the suture of FIG. 8W.

### Example Y. Quadra Helix 10-0

FIG. 8Y shows an image of a 10-0 quadra-helix self-retaining suture made according to an embodiment of the invention. Self-retaining suture 800y was formed by cutting retainers 802y in a quadra-helix pattern (n=4) into a polypropylene suture thread 804y of USP 10-0 (32µm nominal diameter) using a 28mm diameter circular blade. Four retainers were cut at each axial position on the suture - the suture was rotated 90° between cutting the four retainers. The suture was then translated axially and rotated 45° before commencing cutting of the next four retainers. To form the retainers 802y on the polypropylene suture thread 804y, the parameters of the cutting assembly were set as follows: cutting stage angle 78°, plow angle 12°, blade angle 22.22°. The pitch was 48µm, the cut depth was 5µm and retainer length was 25µm at a density of833 retainers per cm (2117 retainers per inch) or 2.67 retainers per suture diameter in axial length. The retainer aspect ratio was approximately 5.0. The ratio of combined retainer length to suture length was 2.08. FIG. 8Z shows an enlarged view of the suture of FIG. 8Y.

### Further Examples

Although not all of these examples have been made, the cutting apparatus previously described is capable of creating retainers on suture threads of USP 8-0 (50µm nominal diameter) as well as suture threads of USP 9-0 (30µm nominal diameter), USP 10-0 (20µm nominal diameter), USP 11-0 (10µm nominal diameter) and USP 12-0 (9µm or less nominal diameter). For example, a self-retaining suture can be made by cutting retainers in a quadra-helix pattern into a polypropylene suture thread of USP 9-0 (30µm nominal diameter) using a sapphire blade by setting the cutting parameters: depth of cut 4µm, retainer length 30µm and pitch 40µm, thereby creating retainers at a density of 1000 retainers per cm (2540 retainers per inch). For example, a self-retaining suture can be made by cutting retainers in a quadra-helix pattern into a polypropylene suture thread of USP 10-0 (20µm nominal diameter) using a sapphire blade by setting the cutting parameters: depth of cut 3µm, retainer length 20µm and pitch 30µm, thereby creating retainers at a density of 1333 retainers per cm (3386 retainers per inch). For example, a self-retaining suture can be made by cutting retainers in a quadra-helix pattern into a polypropylene suture thread of USP 11-0 (15µm nominal diameter) using a sapphire blade by setting the cutting parameters: depth of cut 2µm, retainer length 10µm and pitch 25µm, thereby creating retainers at a density of 1600 retainers per cm (4064 retainers per inch). For example, a self-retaining suture can be made by cutting retainers in a quadra-helix pattern into a polypropylene suture thread of USP 12-0 (9µm nominal diameter) using a sapphire blade by setting the cutting parameters: depth of cut 2µm, retainer length 5µm and pitch 20µm, thereby creating retainers at a density of 2500 retainers per cm (6350 retainers per inch). Note that by increasing the pitch for each of the above examples, the retainer density can be reduced from between 2500 and 79 retainers per cm (6350 and 200 retainers per inch) depending upon the distribution pattern and retainer length. However, as illustrated by the barb configuration testing, a lower pitch relative to the barb length is preferred. For example, in preferred embodiments, the pitch is less than two times the barb length. More preferably the pitch is less than 1.5 times the barb length. Even more preferably the pitch is less than about 1.2 times the barb length.

### Tissue Holding Strength Testing

Tissue-holding strength testing was conducted in order to evaluate and compare the performance of self-retaining sutures made with different retainers and retainer distributions. Tissue-holding strength testing was conducted in a range of different tissues. FIG. 9A shows a schematic diagram of the testing jig used for evaluating tissue holding strength. Testing was conducted using a commercially available TA.XTplus Texture Analyzer 900 and Texture Exponent Software available from Stable Microsystems (United Kingdom).

Standardized tissue samples were prepared from swine tissue. The tissues were prepared to present a fixed thickness of tissue to be engaged by the suture. For anisotropic tissues, care was taken to prepare the sample such that the tissue was oriented in the same way from sample to sample. The following tissue samples were prepared: meniscus (10mm); bladder wall (3mm); uterine wall (3mm); soft palate (10mm); vaginal cuff (2.5mm); and joint capsule (1.5mm).

To analyze tissue-holding strength, a sample suture 920 was inserted downwards into standardized sample tissue 940 using a straight needle or a small diameter hypotube crimped to the leading end of the suture. After insertion of the sample suture 920, the needle and protruding suture was severed. The tissue sample 940 was then secured to the base 902 of the TA.XTplus Texture Analyzer 900 using a clamp 904. The trailing end 922 of the suture was secured to the moving arm 906 of the TA.XTplus Texture Analyzer 900 using a pneumatic grip 908 operated by a foot pedal (not shown). Note that the retainers 924 of the sample suture 920 are oriented to resist movement through tissue in the direction of travel 950 of the moving arm 906. The TA.XTplus Texture Analyzer 900 was then operated to drive moving arm 906 in the direction 950 at a constant velocity until the sample suture 920 was pulled out of the sample tissue 940. The Texture Exponent software was utilized to capture load cell data from the TA.XTplus Texture Analyzer 900 thereby measuring the force needed to pull the sample suture 920 from the sample tissue 940 and overcome the tissue retention by the retainers 924. The analysis was repeated for multiple samples of each suture in order to account for variability between samples. Minitab 15 was then used for statistical analysis of the results. The tissue holding strength of commercially available self-retaining sutures - Quill^{TM} Polypropylene 2-0 was also tested.

The results of the analysis are shown in FIGS. 9B and 9C. FIG. 9B is a table showing the mean, standard error margin and standard deviation in Newtons of the retaining force maxima for particular combinations of suture sample and tissue sample. FIG. 9C is a chart showing the relative increase in tissue holding force of Example N - Lot 48 and Example P - Lot 49 as compared to the commercially available Quill^{TM} Polypropylene 2-0. As shown in FIGS. 9B and 9C, Example N - Lot 48 and Example P-Lot 49 exhibited significantly better tissue holding strength than the commercially available Quill^{TM} Polypropylene 2-0. In particular Example P-Lot 49 showed significantly better tissue holding strength across all tissues tested with an increase from 117% (uterus tissue) to 716% (meniscus tissue) when compared to commercially available Quill^{TM} Polypropylene 2-0.

### Materials

Suture threads described herein may be produced by any suitable method, including without limitation, injection molding, stamping, cutting, extrusion, and so forth. In preferred embodiments, the suture threads are drawn polymeric monofilaments having a high strength to diameter ratio. Polymeric suture threads/filaments may be manufactured or purchased for the suture body, and the retainers can be subsequently cut onto the suture body. The suture threads/filaments can be biodegradable of non-degradable as desired for a particular application. The retainers can be mechanically-cut using blades, cutting wheels, grinding wheels, and so forth. During cutting, either the cutting device or the suture thread may be moved relative to the other, or both may be moved, to control the size, shape and depth.

### Clinical Uses

Self-retaining sutures made according to the apparatus and methods described herein described herein may be used in open, endoscopic and robotic surgery. Self-retaining sutures made according to the apparatus and methods described herein described herein may also be used in microsurgical procedures that are performed under a surgical microscope (and thus may be referred to as "self-retaining sutures") including, for example, microsurgery, vascular microsurgery, nerve repair, cosmetic and reconstructive surgery, urogenital microsurgery, and other microsurgery. Such surgical procedures include, but are not limited to, reattachment and repair of peripheral nerves, spinal microsurgery, vascular microsurgery, microsurgery of the hand, various plastic microsurgical procedures (e.g., facial reconstruction), microsurgery of the male or female reproductive systems, and various types of reconstructive microsurgery. Microsurgical reconstruction is used for complex reconstructive surgery problems when other options such as primary closure, healing by secondary intention, skin grafting, local flap transfer, and distant flap transfer are not adequate. Self-retaining sutures have a very small caliber, often as small as USP 9-0 or USP 10-0, and may have an attached needle of corresponding size. The sutures may be degradable or non-degradable. Self-retaining sutures as described herein may be used in similarly small caliber ranges for ophthalmic surgical procedures and thus may be referred to as "ophthalmic self-retaining sutures". Such procedures include but are not limited to keratoplasty, cataract, and vitreous retinal microsurgical procedures. Ophthalmic self-retaining sutures may be degradable or non-degradable, and have an attached needle of correspondingly-small caliber.

The features of the self-retaining sutures described above can be combined to create a variety of sutures, in particular, the various retainer distribution patterns, retainer shapes, retainer densities can be selected from those described and combined in a multitude of ways without departing from the scope of the present invention. Such self-retaining suture thread can be incorporated into unidirectional or bidirectional sutures as desired for a particular application. Such suture thread can be combined with one or more needles and anchors to create a product suitable for a particular application. Additionally, aspects of the suture cutting apparatus can be selected or combined to create suture cutting apparatus without departing from the scope of the invention. Various modifications, omissions, and additions may be made to the disclosed embodiments without materially departing from the novel teachings and advantages of the invention, particularly in light of the foregoing teachings.

Although the present invention has been shown and described in detail with regard to only a few exemplary embodiments of the invention, it should be understood by those skilled in the art that it is not intended to limit the invention to the specific embodiments disclosed. Various modifications, omissions, and additions may be made to the disclosed embodiments without materially departing from the novel teachings and advantages of the invention, particularly in light of the foregoing teachings. Accordingly, it is intended to cover all such modifications, omissions, additions, and equivalents as may be included within the scope of the invention as defined by the following claims.

## Claims

1. A self-retaining suture comprising:
a suture thread (120, 702, 722, 742, 760, 804) with a plurality of retainers (130, 704, 724, 744, 764, 762, 768, 784, 802) distributed along the suture thread;
wherein the plurality of retainers is distributed at a density of about at least 39 retainers per cm (100 retainers per inch) along a length of the suture thread; and
wherein the plurality of retainers is distributed in a pattern selected from: a quadra-helix pattern; a double-helix pattern; and a single helix pattern.

2. The self-retaining suture of claim 1, wherein the pattern has a pitch P and the retainers (130, 704, 724, 744, 764, 762, 768, 784, 802) have a length L and wherein P < 2L; the retainers are distributed at a density of between 79 retainers per cm (200 retainers per inch) and 630 retainers per cm (1,600 retainers per inch); said suture thread (120, 702, 722, 742, 760, 804) is of a size in the range of USP 4-0 to USP 12-0; said retainers comprise a portion of the suture thread having a cut which partially separates a portion of the suture thread into a shape adapted to engage tissue; and wherein the suture thread has a diameter SD and L > 0.6SD.

3. The self-retaining suture of claim 1,
wherein the suture thread (120, 702, 722, 742, 760, 804) has a suture diameter (SD) no greater than about 300µm;
wherein the retainers (130, 704, 724, 744, 764, 762, 768, 784, 802) have a cut depth (C) between 5% and 35% of the suture diameter (SD);
wherein the retainers have a retainer length (L) greater than 50% of the suture diameter (SD); and
wherein the retainers are distributed at a density greater than 2 retainers per suture diameter (SD) in length of suture thread.

4. The self-retaining suture of claim 3, wherein the plurality of retainers (130, 704, 724, 744, 764, 762, 768, 784, 802) is distributed at a density greater than 2 retainers per retainer length (L) of suture thread (120, 702, 722, 742, 760, 804); the retainer length (L) is between 500% and 800% of the cut depth (C); and the suture thread has a suture diameter (SD) no greater than about 100µm.

5. The self-retaining suture of claim 1, wherein:
the suture thread (120, 702, 722, 742, 760, 804) has a suture diameter (SD) no greater than 300µm;
the retainers (130, 704, 724, 744, 764, 762, 768, 784, 802) have a retainer length L greater than 20% of the suture diameter (SD); and
the retainers are distributed at a density greater than 79 retainers per cm (200 retainers per inch).

6. The self-retaining suture of claim 5, wherein the retainers (130, 704, 724, 744, 764, 762, 768, 784, 802) are distributed at a density of between 79 retainers per cm (200 retainers per inch) and 630 retainers per cm (1,600 retainers per inch); and the suture thread (120, 702, 722, 742, 760, 804) has a suture diameter (SD) smaller than 100µm.

7. The self-retaining suture of claim 1, wherein:
the suture thread (120, 702, 722, 742, 760, 804) has a diameter less than about 350µm and greater than about 250µm, the suture thread having a longitudinal axis;
each retainer (130, 704, 724, 744, 764, 762, 768, 784, 802) is formed by an angled cut into a section of the suture thread;
each retainer has a retainer length measured along said axis wherein the retainer length is greater than about 300µm and less than about 500µm;
the retainers are distributed in pairs, each pair comprising a first retainer and a second retainer;
wherein the second retainer of each pair is positioned at substantially the same position along the axis and substantially 180 degrees around said axis from the first retainer of each pair;
wherein for each pair of retainers there is at least one adjacent pair of retainers;
wherein each pair of retainers is displaced by a pitch length along the axis and substantially 90 degrees around said axis relative to the adjacent pair of retainers; and
wherein the pitch length is no less than about 300µm and no greater than about 550µm.

8. The self-retaining suture of claim 7, wherein the retainer length is at least about 400µm and the pitch length is no greater than about 500µm.

9. The self-retaining suture of claim 7, wherein the suture is a polypropylene monofilament of USP 2-0.

10. The self-retaining suture of claim 1, wherein:
the suture thread (120, 702, 722, 742, 760, 804) has a suture diameter and a longitudinal axis;
each retainer (130, 704, 724, 744, 764, 762, 768, 784, 802) is formed by an angled cut into a section of the suture;
each retainer has a retainer length greater than one suture diameter measured along said axis; and
the retainers are distributed in pairs, each pair comprising a first retainer and a second retainer at substantially the same position along the axis but on the opposite side of the suture;
wherein for each pair of retainers there is at least one adjacent pair of retainers; and
wherein the retainers of each pair of retainers are displaced by a pitch length less than two suture diameters along the axis and substantially 90 degrees around said axis relative to the retainers of the adjacent pair of retainers.

## Patentansprüche

1. Selbsthaltende Naht, umfassend:
einen Nahtfaden (120, 702, 722, 742, 760, 804) mit einer Vielzahl von Haltern (130, 704, 724, 744, 764, 762, 768, 784, 802), die entlang des Nahtfadens verteilt sind;
wobei die Vielzahl von Haltern mit einer Dichte von mindestens ungefähr 39 Haltern pro cm (100 Halter pro Zoll) entlang einer Länge des Nahtfadens verteilt ist; und
wobei die Vielzahl von Haltern in einem Muster verteilt ist, das unter Folgenden ausgewählt ist: ein Vierfachhelix-Muster; ein Doppelhelix-Muster; und ein Einfachhelix-Muster.

2. Selbsthaltende Naht nach Anspruch 1, wobei das Muster eine Steigung P hat und die Halter (130, 704, 724, 744, 764, 762, 768, 784, 802) eine Länge L aufweisen, und wobei P<2L; wobei die Halter mit einer Dichte von 79 Haltern pro cm (200 Halter pro Zoll) bis 630 Halter pro cm (1600 Halter pro Zoll) verteilt sind; wobei der Nahtfaden (120, 702, 722, 742, 760, 804) eine Größe im Bereich von USP 4-0 bis USP 12-0 aufweist;
wobei die Halter einen Abschnitt des Nahtfadens umfassen, der einen Schnitt aufweist, der einen Teil des Nahtfadens teilweise in eine für einen Gewebeeingriff angepasste Form trennt; und
wobei der Nahtfaden einen Durchmesser SD und L > 0,6SD aufweist.

3. Selbsthaltende Naht nach Anspruch 1,
wobei der Nahtfaden (120, 702, 722, 742, 760, 804) einen Nahtdurchmesser (SD) von höchstens ungefähr 300 µm aufweist;
wobei die Halter (130, 704, 724, 744, 764, 762, 768, 784, 802) eine Schnitttiefe (C) zwischen 5% und 35% des Nahtdurchmessers (SD) aufweisen;
wobei die Halter eine Halterlänge (L) aufweisen, die größer als 50% des Nahtdurchmessers (SD) ist; und
wobei die Halter mit einer Dichte von mehr als 2 Haltern pro Nahtdurchmesser (SD) in der Länge des Nahtfadens verteilt sind.

4. Selbsthaltende Naht nach Anspruch 3, wobei die Vielzahl von Haltern (130, 704, 724, 744, 764, 762, 768, 784, 802) mit einer Dichte von mehr als 2 Haltern pro Halterlänge (L) des Nahtfadens (120, 702, 722, 742, 760, 804) verteilt ist; wobei die Halterlänge (L) zwischen 500% und 800% der Schnitttiefe (C) beträgt; und wobei der Nahtfaden einen Nahtdurchmesser (SD) von höchstens ungefähr 100 µm aufweist.

5. Selbsthaltende Naht nach Anspruch 1, wobei:
der Nahtfaden (120, 702, 722, 742, 760, 804) einen Nahtdurchmesser (SD) von höchstens 300 µm aufweist;
die Halter (130, 704, 724, 744, 764, 762, 768, 784, 802) eine Halterlänge L aufweisen, die größer als 20% des Nahtdurchmessers (SD) ist; und
die Halter mit einer Dichte von mehr als 79 Haltern pro cm (200 Halter pro Zoll) verteilt sind.

6. Selbsthaltende Naht nach Anspruch 5, wobei die Halter (130, 704, 724, 744, 764, 762, 768, 784, 802) mit einer Dichte von 79 Haltern pro cm (200 Halter pro Zoll) bis 630 Halter pro cm (1600 Halter pro Zoll) verteilt sind; und der Nahtfaden (120, 702, 722, 742, 760, 804) einen Nahtdurchmesser (SD) von weniger als 100 µm aufweist.

7. Selbsthaltende Naht nach Anspruch 1, wobei:
der Nahtfaden (120, 702, 722, 742, 760, 804) einen Durchmesser von weniger als ungefähr 350 µm und mehr als ungefähr 250 µm aufweist, wobei der Nahtfaden eine Längsachse aufweist;
jeder Halter (130, 704, 724, 744, 764, 762, 768, 784, 802) von einem winkelförmigen Schnitt in einen Abschnitt des Nahtfadens gebildet wird;
jeder Halter eine Halterlänge, gemessen entlang der Achse, aufweist, wobei die Halterlänge mehr als ungefähr 300 µm und weniger als ungefähr 500 µm beträgt;
die Halter paarweise verteilt sind, wobei jedes Paar einen ersten Halter und einen zweiten Halter aufweist;
wobei der zweite Halter jedes Paars im Wesentlichen in derselben Position entlang der Achse und im Wesentlichen 180 Grad um die Achse vom ersten Halter jedes Paars angeordnet ist;
wobei es für jedes Halterpaar mindestens ein benachbartes Halterpaar gibt;
wobei jedes Halterpaar durch eine Steigungslänge entlang der Achse und im Wesentlichen um 90 Grad um die Achse bezüglich des benachbarten Halterpaars verschoben ist; und
wobei die Steigungslänge nicht weniger als ungefähr 300 µm und nicht mehr als ungefähr 550 µm beträgt.

8. Selbsthaltende Naht nach Anspruch 7, wobei die Halterlänge mindestens ungefähr 400 µm beträgt und die Steigungslänge nicht mehr als ungefähr 500 µm beträgt.

9. Selbsthaltende Naht nach Anspruch 7, wobei die Naht ein USP-2-0 Polypropylen-Monofilament ist.

10. Selbsthaltende Naht nach Anspruch 1, wobei:
der Nahtfaden (120, 702, 722, 742, 760, 804) einen Nahtdurchmesser und eine Längsachse aufweist;
jeder Halter (130, 704, 724, 744, 764, 762, 768, 784, 802) von einem winkelförmigen Schnitt in einen Abschnitt des Nahtfadens gebildet wird;
jeder Halter eine Halterlänge aufweist, die größer als ein Nahtdurchmesser ist, gemessen entlang der Achse; und
die Halter paarweise verteilt sind, wobei jedes Paar einen ersten Halter und einen zweiten Halter im Wesentlichen an derselben Position entlang der Achse, aber auf der gegenüberliegenden Seite der Naht aufweist;
wobei es für jedes Halterpaar mindestens ein benachbartes Halterpaar gibt;
und
wobei die Halter jedes Halterpaars durch eine Steigungslänge, die kleiner als zwei Nahtdurchmesser ist, entlang der Achse und im Wesentlichen um 90 Grad um die Achse bezüglich der Halter des benachbarten Halterpaars verschoben sind.

## Revendications

1. Suture à auto-retenue comprenant :
un fil de suture (120, 702, 722, 742, 760, 804) ayant une pluralité de dispositifs de retenue (130, 704, 724, 744, 764, 762, 768, 784, 802) répartis le long du fil de suture ;
dans laquelle la pluralité de dispositifs de retenue est répartie à une densité d'environ au moins 39 dispositifs de retenue par cm (100 dispositifs de retenue par pouce) sur toute la longueur du fil de suture ; et
dans laquelle la pluralité de dispositifs de retenue est répartie selon un motif sélectionné parmi : un motif à quatre hélices ; un motif à deux hélices ; et un motif à une seule hélice.

2. Suture à auto-retenue selon la revendication 1, dans laquelle le motif présente un pas (P) et les dispositifs de retenue (130, 704, 724, 744, 764, 762, 768, 784, 802) présentent une longueur (L) et dans laquelle P < 2L ; les dispositifs de retenue sont répartis à une densité comprise entre 79 dispositifs de retenue par cm (200 dispositifs de retenue par pouce) et 630 dispositifs de retenue par cm (1 600 dispositifs de retenue par pouce) ; ledit fil de suture (120, 702, 722, 742, 760, 804) est d'une taille dans la plage allant de 4-0 USP à 12-0 USP ; lesdits dispositifs de retenue comprennent une partie du fil de suture ayant une découpe qui sépare partiellement une partie du fil de suture en une forme conçue pour venir en prise avec un tissu ; et dans laquelle le fil de suture présente un diamètre (SD) et L > 0,6 SD.

3. Suture à auto-retenue selon la revendication 1,
dans laquelle le fil de suture (120, 702, 722, 742, 760, 804) présente un diamètre de suture (SD) inférieur ou égal à environ 300 µm ;
dans laquelle les dispositifs de retenue (130, 704, 724, 744, 764, 762, 768, 784, 802) présentent une profondeur de découpe (C) comprise entre 5 % et 35 % du diamètre de suture (SD) ;
dans laquelle les dispositifs de retenue présentent une longueur de dispositif de retenue (L) supérieure à 50 % du diamètre de suture (SD) ; et
dans laquelle les dispositifs de retenue sont répartis à une densité supérieure à 2 dispositifs de retenue par diamètre de suture (SD) sur la longueur du fil de suture.

4. Suture à auto-retenue selon la revendication 3, dans laquelle la pluralité de dispositifs de retenue (130, 704, 724, 744, 764, 762, 768, 784, 802) est répartie à une densité supérieure à 2 dispositifs de retenue par longueur de dispositif de retenue (L) d'un fil de suture (120, 702, 722, 742, 760, 804) ; la longueur de dispositif de retenue (L) est comprise entre 500 % et 800 % de la profondeur de découpe (C) ; et le fil de suture présente un diamètre de suture (SD) inférieur ou égal à environ 100 µm.

5. Suture à auto-retenue selon la revendication 1, dans laquelle :
le fil de suture (120, 702, 722, 742, 760, 804) présente un diamètre de suture (SD) inférieur ou égal à 300 µm ;
les dispositifs de retenue (130, 704, 724, 744, 764, 762, 768, 784, 802) présentent une longueur de dispositif de retenue (L) supérieure à 20 % du diamètre de suture (SD) ;
et
les dispositifs de retenue sont répartis à une densité supérieure à 79 dispositifs de retenue par cm (200 dispositifs de retenue par pouce).

6. Suture à auto-retenue selon la revendication 5, dans laquelle les dispositifs de retenue (130, 704, 724, 744, 764, 762, 768, 784, 802) sont répartis à une densité comprise entre 79 dispositifs de retenue par cm (200 dispositifs de retenue par pouce) et 630 dispositifs de retenue par cm (1 600 dispositifs de retenue par pouce) ; et le fil de suture (120, 702, 722, 742, 760, 804) présente un diamètre de suture (SD) plus petit que 100 µm.

7. Suture à auto-retenue selon la revendication 1, dans laquelle :
le fil de suture (120, 702, 722, 742, 760, 804) présente un diamètre inférieur à environ 350 µm et supérieur à environ 250 µm, le fil de suture ayant un axe longitudinal ;
chaque dispositif de retenue (130, 704, 724, 744, 764, 762, 768, 784, 802) est formé par une découpe oblique dans une section du fil de suture ;
chaque dispositif de retenue présente une longueur de dispositif de retenue mesurée le long dudit axe dans laquelle la longueur de dispositif de retenue est supérieure à environ 300 µm et inférieure à environ 500 µm ;
les dispositifs de retenue sont répartis en paires, chaque paire comprenant un premier dispositif de retenue et un second dispositif de retenue ;
dans laquelle le second dispositif de retenue de chaque paire est positionné sensiblement à la même position le long de l'axe et sensiblement à 180 degrés autour dudit axe à partir du premier dispositif de retenue de chaque paire ;
dans laquelle, pour chaque paire de dispositifs de retenue, il y a au moins une paire adjacente de dispositifs de retenue ;
dans laquelle chaque paire de dispositifs de retenue est déplacée par une longueur de pas le long de l'axe et sensiblement de 90 degrés autour dudit axe par rapport à la paire adjacente de dispositifs de retenue ; et
dans laquelle la longueur de pas est supérieure ou égale à environ 300 µm et inférieure ou égale à environ 550 µm.

8. Suture à auto-retenue selon la revendication 7, dans laquelle la longueur de dispositif de retenue fait au moins environ 400 µm et la longueur de pas est inférieure ou égale à environ 500 µm.

9. Suture à auto-retenue selon la revendication 7, dans laquelle la suture est un monofilament en polypropylène de 2-0 USP.

10. Suture à auto-retenue selon la revendication 1, dans laquelle :
le fil de suture (120, 702, 722, 742, 760, 804) présente un diamètre de suture et un axe longitudinal ;
chaque dispositif de retenue (130, 704, 724, 744, 764, 762, 768, 784, 802) est formé par un angle oblique dans une section de la suture ;
chaque dispositif de retenue présente une longueur de dispositif de retenue supérieure à un diamètre de suture mesuré le long dudit axe ; et
les dispositifs de retenue sont répartis en paires, chaque paire comprenant un premier dispositif de retenue et un second dispositif de retenue sensiblement à la même position le long de l'axe mais sur le côté opposé de la suture ;
dans laquelle, pour chaque paire de dispositifs de retenue, il y a au moins une paire adjacente de dispositifs de retenue ; et
dans laquelle les dispositifs de retenue de chaque paire de dispositifs de retenue sont déplacés par une longueur de pas inférieure à deux diamètres de suture le long de l'axe et sensiblement de 90 degrés autour dudit axe par rapport aux dispositifs de retenue de la paire adjacente de dispositifs de retenue.
